(19) Europäisches Patentamt
European Patent Office
Office européen des brevets

(11) **EP 2 351 850 A1**

(12) **EUROPEAN PATENT APPLICATION**
published in accordance with Art. 153(4) EPC

(43) Date of publication:
**03.08.2011 Bulletin 2011/31**

(21) Application number: **09821909.0**

(22) Date of filing: **29.09.2009**

(51) Int Cl.:
***C12Q 1/68*** (2006.01) ***C12N 15/09*** (2006.01)

(86) International application number:
**PCT/JP2009/066963**

(87) International publication number:
**WO 2010/047211 (29.04.2010 Gazette 2010/17)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **23.10.2008 JP 2008273257**

(71) Applicant: **Sysmex Corporation**
**Kobe-shi, Hyogo 651-0073 (JP)**

(72) Inventors:
- **OKA, Noriko**
  **Kobe-shi**
  **Hyogo 651-0073 (JP)**
- **KAJITA, Masahiro**
  **Kobe-shi**
  **Hyogo 651-0073 (JP)**

(74) Representative: **Paemen, Liesbet R.J. et al**
**De Clercq & Partners**
**Edgard Gevaertdreef 10 a**
**9830 Sint-Martens-Latem (BE)**

(54) **METHOD FOR DETERMINING PRESENCE OR ABSENCE OF ABNORMAL CELL**

(57) A method for determining the presence or absence of an abnormal cell in a sample collected from the uterine cervix of a subject, and a method for predicting the progression of a lesion in the uterine cervix in a subject, each of which comprises measuring the frequency of methylation in the genomic DNA of human papillomavirus contained in the sample and determining the presence or absence of the abnormal cell or predicting the progression of the lesion based on the frequency; and a primer set which can be used in the above-mentioned methods.

**Figure 8**

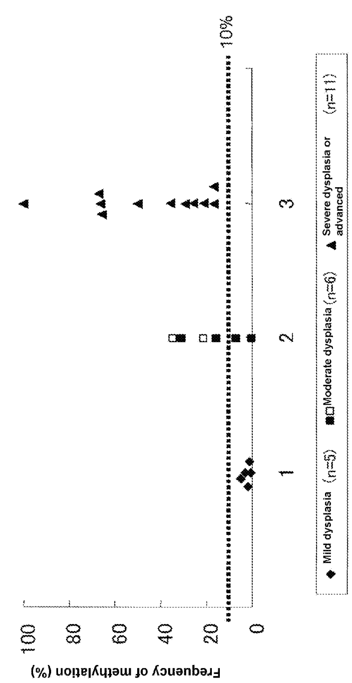


EP 2 351 850 A1

SEQUENCE LISTING

<110> Sysmex Corporation

<120> Method for judging existence of abnormal cells
<130> TM5367PC

<150> JP 2008-273257
<151> 2008-10-23

<160> 43

<170> PatentIn version 3.1

<210> 1
<211> 7904
<212> DNA
<213> Human papillomavirus type 16

<400> 1
actacaataa ttcatgtata aaactaaggg cgtaaccgaa atcggttgaa ccgaaaccgg 60

ttagtataaa agcagacatt ttatgcacca aaagagaact gcaatgtttc aggacccaca 120

ggagcgaccc agaaagttac cacagttatg cacagagctg caaacaacta tacatgatat 180

aatattagaa tgtgtgtact gcaagcaaca gttactgcga cgtgaggtat atgactttgc 240

ttttcgggat ttatgcatag tatatagaga tgggaatcca tatgctgtat gtgataaatg 300

tttaaagttt tattctaaaa ttagtgagta tagacattat tgttatagtt tgtatggaac 360

aacattagaa cagcaataca acaaaccgtt gtgtgatttg ttaattaggt gtattaactg 420

tcaaaagcca ctgtgtcctg aagaaaagca aagacatctg gacaaaaagc aaagattcca 480

taatataagg ggtcggtgga ccggtcgatg tatgtcttgt tgcagatcat caagaacacg 540

tagagaaacc cagctgtaat catgcatgga gatacaccta cattgcatga atatatgtta 600

gatttgcaac cagagacaac tgatctctac tgttatgagc aattaaatga cagctcagag 660

gaggaggatg aaatagatgg tccagctgga caagcagaac cggacagagc ccattacaat 720

attgtaacct tttgttgcaa gtgtgactct acgcttcggt tgtgcgtaca aagcacacac 780

gtagacattc gtactttgga agacctgtta atgggcacac taggaattgt gtgccccatc 840

tgttctcaga aaccataatc taccatggct gatcctgcag gtaccaatgg ggaagagggt 900

acgggatgta atggatggtt ttatgtagag gctgtagtgg aaaaaaaaac aggggatgct 960

atatcagatg acgagaacga aaatgacagt gatacaggtg aagatttggt agattttata 1020

gtaaatgata atgattattt aacacaggca gaaacagaga cagcacatgc gttgtttact 1080

gcacaggaag caaaacaaca tagagatgca gtacaggttc taaaacgaaa gtatttggta 1140

gtccacttag tgatattagt ggatgtgtag acaataatat tagtcctaga ttaaaagcta 1200

tatgtataga aaaacaaagt agagctgcaa aaaggagatt atttgaaagc gaagacagcg 1260

```
ggtatggcaa tactgaagtg gaaactcagc agatgttaca ggtagaaggg cgccatgaga   1320
ctgaaacacc atgtagtcag tatagtggtg gaagtggggg tggttgcagt cagtacagta   1380
gtggaagtgg gggagagggt gttagtgaaa gacacactat atgccaaaca ccacttacaa   1440
atattttaaa tgtactaaaa actagtaatg caaaggcagc aatgttagca aaatttaaag   1500
agttatacgg ggtgagtttt tcagaattag taagaccatt taaaagtaat aaatcaacgt   1560
gttgcgattg gtgtattgct gcatttggac ttacacccag tatagctgac agtataaaaa   1620
cactattaca acaatattgt ttatatttac acattcaaag tttagcatgt tcatggggaa   1680
tggttgtgtt actattagta agatataaat gtggaaaaaa tagagaaaca attgaaaaat   1740
tgctgtctaa actattatgt gtgtctccaa tgtgtatgat gatagagcct ccaaaattgc   1800
gtagtacagc agcagcatta tattggtata aaacaggtat atcaaatatt agtgaagtgt   1860
atggagacac gccagaatgg atacaaagac aaacagtatt acaacatagt tttaatgatt   1920
gtacatttga attatcacag atggtacaat gggcctacga taatgacata gtagacgata   1980
gtgaaattgc atataaatat gcacaattgg cagacactaa tagtaatgca agtgcctttc   2040
taaaaagtaa ttcacaggca aaaattgtaa aggattgtgc aacaatgtgt agacattata   2100
aacgagcaga aaaaaaacaa atgagtatga gtcaatggat aaaatataga tgtgataggg   2160
tagatgatgg aggtgattgg aagcaaattg ttatgttttt aaggtatcaa ggtgtagagt   2220
ttatgtcatt tttaactgca ttaaaaagat ttttgcaagg catacctaaa aaaaattgca   2280
tattactata tggtgcagct aacacaggta aatcattatt tggtatgagt ttaatgaaat   2340
ttctgcaagg gtctgtaata tgttttgtaa attctaaaag ccatttttgg ttacaaccat   2400
tagcagatgc caaaataggt atgttagatg atgctacagt gccctgttgg aactacatag   2460
atgacaattt aagaaatgca ttggatggaa atttagtttc tatggatgta aagcatagac   2520
cattggtaca actaaaatgc cctccattat taattacatc taacattaat gctggtacag   2580
attctaggtg gccttattta cataatagat tggtggtgtt tacatttcct aatgagtttc   2640
catttgacga aaacggaaat ccagtgtatg agcttaatga taagaactgg aaatcctttt   2700
tctcaaggac gtggtccaga ttaagtttgc acgaggacga ggacaaggaa aacgatggag   2760
actctttgcc aacgtttaaa tgtgtgtcag gacaaaatac taacacatta tgaaaatgat   2820
agtacagacc tacgtgacca tatagactat tggaaacaca tgcgcctaga atgtgctatt   2880
tattacaagg ccagagaaat gggatttaaa catattaacc accaagtggt gccaacactg   2940
gctgtatcaa agaataaagc attacaagca attgaactgc aactaacgtt agaaacaata   3000
tataactcac aatatagtaa tgaaaagtgg acattacaag acgttagcct tgaagtgtat   3060
```

```
ttaactgcac caacaggatg tataaaaaaa catggatata cagtggaagt gcagtttgat   3120
ggagacatat gcaatacaat gcattataca aactggacac atatatatat ttgtgaagaa   3180
gcatcagtaa ctgtggtaga gggtcaagtt gactattatg gtttatatta tgttcatgaa   3240
ggaatacgaa catattttgt gcagtttaaa gatgatgcag aaaaatatag taaaaataaa   3300
gtatgggaag ttcatgcggg tggtcaggta atattatgtc ctacatctgt gtttagcagc   3360
aacgaagtat cctctcctga aattattagg cagcacttgg ccaaccaccc cgccgcgacc   3420
cataccaaag ccgtcgcctt gggcaccgaa gaaacacaga cgactatcca gcgaccaaga   3480
tcagagccag acaccggaaa cccctgccac accactaagt tgttgcacag agactcagtg   3540
gacagtgctc caatcctcac tgcatttaac agctcacaca aaggacggat taactgtaat   3600
agtaacacta cacccatagt acatttaaaa ggtgatgcta atactttaaa atgtttaaga   3660
tatagattta aaaagcattg tacattgtat actgcagtgt cgtctacatg gcattggaca   3720
ggacataatg taaaacataa aagtgcaatt gttacactta catatgatag tgaatggcaa   3780
cgtgaccaat tttgtctca agttaaaata ccaaaaacta ttacagtgtc tactggattt   3840
atgtctatat gacaaatctt gatactgcat ccacaacatt actggcgtgc tttttgcttt   3900
gctttgtgtg cttttgtgtg tctgcctatt aatacgtccg ctgcttttgt ctgtgtctac   3960
atacacatca ttaataatat tggtattact attgtggata acagcagcct ctgcgtttag   4020
gtgttttatt gtatatatta tatttgttta tataccatta tttttaatac atacacatgc   4080
acgcttttta attacataat gtatatgtac ataatgtaat tgttacatat aattgttgta   4140
taccataact tactattttt tcttttttat tttcatatat aatttttttt tttgtttgtt   4200
tgtttgtttt ttaataaact gttattactt aacaatgcga cacaaacgtt ctgcaaaacg   4260
cacaaaacgt gcatcggcta cccaacttta taaaacatgc aaacaggcag gtacatgtcc   4320
acctgacatt atacctaagg ttgaaggcaa aactattgct gaacaaatat tacaatatgg   4380
aagtatgggt gtattttttg gtgggttagg aattggaaca gggtcgggta caggcggacg   4440
cactgggtat attccattgg gaacaaggcc tcccacagct acagatacac ttgctcctgt   4500
aagaccccct ttaacagtag atcctgtggg cccttctgat ccttctatag tttctttagt   4560
ggaagaaact agttttattg atgctggtgc accaacatct gtaccttcca ttcccccaga   4620
tgtatcagga tttagtatta ctacttcaac tgataccaca cctgctatat tagatattaa   4680
taatactgtt actactgtta ctacacataa taatcccact ttcactgacc catctgtatt   4740
gcagcctcca acacctgcag aaactggagg gcattttaca ctttcatcat ccactattag   4800
tacacataat tatgaagaaa ttcctatgga tacatttatt gttagcacaa accctaacac   4860
agtaactagt agcacaccca taccagggtc tcgcccagtg gcacgcctag gattatatag   4920
```

tcgcacaaca caacaggtta aagttgtaga ccctgctttt gtaaccactc ccactaaact 4980
tattacatat gataatcctg catatgaagg tatagatgtg gataatacat tatatttttc 5040
tagtaatgat aatagtatta atatagctcc agatcctgac tttttggata tagttgcttt 5100
acataggcca gcattaacct ctaggcgtac tggcattagg tacagtagaa ttggtaataa 5160
acaaacacta cgtactcgta gtggaaaatc tataggtgct aaggtacatt attattatga 5220
tttaagtact attgatcctg cagaagaaat agaattacaa actataacac cttctacata 5280
tactaccact tcacatgcag cctcacctac ttctattaat aatggattat atgatattta 5340
tgcagatgac tttattacag atacttctac aaccccggta ccatctgtac cctctacatc 5400
tttatcaggt tatattcctg caaatacaac aattcctttt ggtggtgcat acaatattcc 5460
tttagtatca ggtcctgata tacccattaa tataactgac caagctcctt cattaattcc 5520
tatagttcca gggtctccac aatatacaat tattgctgat gcaggtgact tttatttaca 5580
tcctagttat tacatgttac gaaaacgacg taaacgttta ccatattttt tttcagatgt 5640
ctctttggct gcctagtgag gccactgtct acttgcctcc tgtcccagta tctaaggttg 5700
taagcacgga tgaatatgtt gcacgcacaa acatatatta tcatgcagga acatccagac 5760
tacttgcagt tggacatccc tattttccta ttaaaaaacc taacaataac aaaatattag 5820
ttcctaaagt atcaggatta caatacaggg tatttagaat acatttacct gaccccaata 5880
agtttggttt tcctgacacc tcattttata atccagatac acagcggctg gtttgggcct 5940
gtgtaggtgt tgaggtaggt cgtggtcagc cattaggtgt gggcattagt ggccatcctt 6000
tattaaataa attggatgac acagaaaatg ctagtgctta tgcagcaaat gcaggtgtgg 6060
ataatagaga atgtatatct atggattaca aacaaacaca attgtgttta attggttgca 6120
aaccacctat aggggaacac tggggcaaag gatccccatg taccaatgtt gcagtaaatc 6180
caggtgattg tccaccatta gagttaataa acacagttat tcaggatggt gatatggttc 6240
atactggctt tggtgctatg gactttacta cattacaggc taacaaaagt gaagttccac 6300
tggatatttg tacatctatt tgcaaatatc cagattatat taaaatggtg tcagaaccat 6360
atggcgacag cttatttttt tatttacgaa gggaacaaat gtttgttaga catttattta 6420
atagggctgg tactgttggt gaaaatgtac cagacgattt atacattaaa ggctctgggt 6480
ctactgcaaa tttagccagt tcaaattatt ttcctacacc tagtggttct atggttacct 6540
ctgatgccca aatattcaat aaaccttatt ggttacaacg agcacagggc cacaataatg 6600
gcatttgttg ggggtaaccaa ctatttgtta ctgttgttga tactacacgc agtacaaata 6660
tgtcattatg tgctgccata tctacttcag aaactacata taaaaatact aactttaagg 6720

agtacctacg acatggggag gaatatgatt tacagtttat ttttcaactg tgcaaaataa 6780

ccttaactgc agacgttatg acatacatac attctatgaa ttccactatt ttggaggact 6840

ggaattttgg tctacaacct cccccaggag gcacactaga agatacttat aggtttgtaa 6900

cccaggcaat tgcttgtcaa aaacatacac ctccagcacc taaagaagat gatcccctta 6960

aaaaatacac tttttgggaa gtaaatttaa aggaaaagtt ttctgcagac ctagatcagt 7020

ttcctttagg acgcaaattt ttactacaag caggattgaa ggccaaacca aaatttacat 7080

taggaaaacg aaaagctaca cccaccacct catctacctc tacaactgct aaacgcaaaa 7140

aacgtaagct gtaagtattg tatgtatgtt gaattagtgt tgtttgttgt gtatatgttt 7200

gtatgtgctt gtatgtgctt gtaaatatta agttgtatgt gtgtttgtat gtatggtata 7260

ataaacacgt gtgtatgtgt ttttaaatgc ttgtgtaact attgtgtcat gcaacataaa 7320

taaacttatt gtttcaacac ctactaattg tgttgtggtt attcattgta tataaactat 7380

atttgctaca tcctgttttt gttttatata tactatattt tgtagcgcca ggcccatttt 7440

gtagcttcaa ccgaattcgg ttgcatgctt tttggcacaa aatgtgtttt tttaaatagt 7500

tctatgtcag caactatggt ttaaacttgt acgtttcctg cttgccatgc gtgccaaatc 7560

cctgttttcc tgacctgcac tgcttgccaa ccattccatt gtttttaca ctgcactatg 7620

tgcaactact gaatcactat gtacattgtg tcatataaaa taaatcacta tgcgccaacg 7680

ccttacatac cgctgttagg cacatatttt tggcttgttt taactaacct aattgcatat 7740

ttggcataag gtttaaactt ctaaggccaa ctaaatgtca ccctagttca tacatgaact 7800

gtgtaaaggt tagtcataca ttgttcattt gtaaaactgc acatgggtgt gtgcaaaccg 7860

attttgggtt acacatttac aagcaactta tataataata ctaa 7904

<210> 2
<211> 7942
<212> DNA
<213> Human papillomavirus type 52

<400> 2
taaattataa tcttatacta gtaaaaaata gggtgtaacc gaaaacggtc agaccgaaac 60

cggtgtatat atatagaaca cagtgtagct aacgcacggc catgtttgag gatccagcaa 120

cacgaccccg gaccctgcac gaattgtgtg aggtgctgga agaatcggtg catgaaataa 180

ggctgcagtg tgtgcagtgc aaaaaagagc tacaacgaag agaggtatac aagtttctat 240

ttacagattt acgaatagta tatagagaca ataatccata tggcgtgtgt attatgtgcc 300

tacgcttttt atctaagata agtgaatata ggcattatca atattcactg tatgggaaaa 360

cattagaaga gagggtaaaa aaaccattaa gtgaaataac tattagatgt ataatttgtc 420

aaacgccatt atgtcctgaa gaaaaagaaa gacatgttaa tgcaaacaag cgatttcata 480
atattatggg tcgttggaca gggcgctgtt cagagtgttg gagaccccga cctgtgaccc 540
aagtgtaacg tcatgcgtgg agacaaagca actataaaag attatatatt agatctgcaa 600
cctgaaacaa ctgacctaca ctgctatgag caattaggtg acagctcaga tgaggaggat 660
acagatggtg tggaccggcc agatggacaa gcagaacaag ccacaagcaa ttactacatt 720
gtgacatatt gtcacagttg tgatagcaca ctacggctat gcattcatag cactgcgacg 780
gaccttcgta ctctacagca aatgctgttg ggcacattac aagttgtgtg ccccggctgt 840
gcacggctat aaacaaccct gcaatggagg accctgaagg tacagagggc gaaagggagg 900
gatgtacagg ctggtttgaa gtagaggcaa taatagaaaa acaaacagga gataacattt 960
cagaggacga ggatgaaaat gcatatgata gtggaacaga tctaatagat tttatagatg 1020
attcaaatat aaataatgaa caggcagaac atgaggcagc ccgggcattg tttaatgcac 1080
aggaagggga ggatgattta catgctgtgt ctgcagtaaa acgaaagttt acaagcagtc 1140
cggaaagtgc tgggcaagat ggtgtagaaa aacatggtag tccgcgtgca aaacacattt 1200
gtgtaaatac agagtgtgtt ttaccaaaac gcaaaccatg tcacgtagaa gacagcggct 1260
atggcaatag tgaagtggaa gcgcagcaga tggcagacca ggtagacggg caaaatggcg 1320
actggcaaag taacagtagt caatcaagtg ggtgggggc tagtaattca gatgtaagtt 1380
gtactagtat agaggacaat gaggaaaata gtaatagaac gctaaaaagc atacaaaata 1440
ttatgtgcga aaatagcata aaaacaactg tattatttaa atttaaagaa acatatggtg 1500
ttagctttat ggaattagta agaccattta aaagtaatag aagtagttgt acagattggt 1560
gtattatagg aatgggagta acaccatcag ttgcagaagg attaaaagta ttaatacagc 1620
cctatagcat atatgcccat ttgcaatgtt taacatgtga cagaggcgtg cttatactgc 1680
tgctaattag gtttaaatgt ggaaaaaaca gattaacagt gtccaaacta atgtcacagc 1740
tgttaaatat accagaaaca catatggtaa tagaaccacc aaaattacga agtgctacct 1800
gtgcattata ttggtataga acaggtttgt ctaatattag tgaggtatat ggtaccaccc 1860
cagaatggat agaacaacaa acagtattac agcatagctt tgacaatagc atattcgatt 1920
ttggagaaat ggtgcaatgg gcatatgatc atgatataac agatgatagt gacatagcat 1980
ataaatatgc acagttagca gatgtaaata gcaatgctgc agcattccta aaaagcaatt 2040
cgcaagcaaa aatagtaaag gactgtgcaa ccatgtgtag acattataaa cgggcagaaa 2100
gaaaacatat gaatattgga caatggatac agtatagatg tgatagaata gatgatggtg 2160
gagattggag gcctatagta agatttttaa gatatcaaga catagaattt acagcctttt 2220
tagacgcatt taaaaaattt ttaaaaggta tacctaaaaa aaattgttta gtattatatg 2280

gacctgcaaa cacaggaaaa tcatattttg gaatgagttt aattaggttc ttaagtggat 2340
gtgtaatatc ctatgtaaac tcaaaaagcc atttttggct acaaccatta acagatgcaa 2400
aagtgggtat gatagatgat gtaacaccta tatgttggac atatatagat gattatatga 2460
gaaatgcact ggatggaaat gatatatcag tagatgtaaa gcatagagcc ttagtacaaa 2520
taaaatgccc accattaatt ttaacaacaa atacaaatgc aggaacagat cctaggtggc 2580
catatttaca tagtagattg gttgtgtttc atttcaaaaa cccatttcca tttgatgaaa 2640
atggcaatcc tatatatgaa attaacaacg aaaattggaa atcctttttc tcaaggacgt 2700
ggtgcaaatt agatttaata caggaagagg acaaggaaaa cgatggagtc gataccggca 2760
cgtttaaatg cagtgcagga aaaaatacta gatctatacg aagctgatag taatgaccta 2820
aacgcacaaa ttgaacattg gaaattgact cgaatggaat gtgttttgtt ttacaaagca 2880
aaggaactgg gaataactca tataggccac caggtggtgc caccaatggc agtgtctaag 2940
gcaaaggcct gccaagctat tgaactacaa ttggcattgg aggcattaaa caaaacacaa 3000
tatagcacag atggatggac attacaacaa acaagtctag aaatgtggcg tgcagaacca 3060
caaaaatact ttaaaaaaca tgggtataca ataacagtgc aatacgataa tgataaaaac 3120
aatactatgg attatacaaa ctggaaggaa atttatttac ttggtgagtg tgaatgtaca 3180
attgtagaag gacaagtaga ttactatggg ttatattatt ggtgtgatgg agaaaaaata 3240
tattttgtaa aatttagtaa cgatgcaaag caatattgtg taacaggagt atgggaagta 3300
catgtgggtg gtcaggtaat tgtttgtcct gcatctgtat ctagtaacga agtatccact 3360
actgaaactg ctgtccacct atgcaccgaa acctccaaga cctccgcagt gtccgtgggt 3420
gccaaagaca cacacctaca accaccacag aaacgacgac gaccagacgt cacagactcc 3480
agaaacacca agtaccccaa caacctttg cggggacaac aatccgtgga cagtactaca 3540
cggggactcg tcactgcaac tgagtgcaca aacaaaggac gggttgcaca tacaacttgt 3600
actgcaccta taatacacct aaaaggtgat cctaatagtt taaaatgttt aagatatagg 3660
gtaaaaacac ataaaagttt gtatgttcaa atttcatcta cctggcattg gaccagtaat 3720
gaatgtacaa ataataaact aggtattgta acaataacgt acagtgatga aacacaacgt 3780
caacaatttt taaaaactgt taaaatacca aatactgtgc aagttataca aggtgtcatg 3840
tcattgtgat atttgtacat atgtatatat gtatatgtgt atggtaaaca cccaacacaa 3900
gccaatattg ctgctattgt gtatatataa caatgttagg attatttgta ttttgtttta 3960
ttttgcttat ggtgttttgt gcagtgctta ggccgctctt gctatctata tcggtgtatg 4020
cgcaggtgtt ggtgctggtg cttttgctat gggtatctat tgggtcacca tttaaagtgt 4080

tttttttgta cctactgttt ttatattttc caatgttttg tattcactgt catgcacagt 4140
atttggcaca actgcaataa ctgtacatgt agattggcta catgcatata tgcaaaatat 4200
actttttcac ttttgtagtt tgtctaataa atacttttat atttttaat agcttgtcgc 4260
aatgagatac agacggtcta cacggcacaa acgtgcttct gcaacacagc tatatcaaac 4320
atgcaaagcc tctggcacct gcccccccga tgttattcct aaagtggaag gcacaactat 4380
tgcagatcaa cttttaaaat atggcagcct aggggtgttt tttggaggtt tgggtatagg 4440
tacaggtgca ggctctggtg gtagggcagg ctatgtgcca ttgtccactc gtcctcccac 4500
tagtagtatt accacgtcca ccattcgtcc ccctgtaact gtagaaccca ttggtccctt 4560
agaaccatct atagtttcta tgatagaaga aacaacattt attgagtctg gcgcacctgc 4620
tccatctatt ccatcagcaa cagggtttga tgttacaaca tctgcaaata atactcctgc 4680
aataattaat gtaacatcta taggtgaatc atctgtacaa tcagtttcta cacatttaaa 4740
tcctacattc actgaaccat ctataataca gcccccggca cctgcagaag catctggtca 4800
tgtattgttt tctagtccaa ctattagtac acacacctat gaagaaatcc ctatggatac 4860
atttgttacc tctactgaca gcagcagtgt aacaagtagt acacctattc cagggtctcg 4920
ccctacgaca cgccttggtt tatatagccg tgccacacaa caggttaagg tagtcgaccc 4980
tgcttttatg tcatcaccac agaaattagt aacatataac aatcctgttt ttgagggcgt 5040
tgatacagat gaaactataa tttttgatcg ttcacaactt ttacctgcac cggatcctga 5100
ttttttagac attatagctt tgcataggcc tgcattaacc tctcgaagag gtactgttag 5160
gtttagcagg cttggtaata aggccaccct acgtacacgt agtggaaaac aaattggggc 5220
acgggtacat tattatcatg atattagtcc tatccagcct gctgaagttc aggaagacat 5280
agaattgcaa cctttattac cacagtctgt gtccccttac actattaatg atggtttgta 5340
tgatgtgtat gcagattctt tgcagcaacc cacgtttcac ttaccttcca cactttctac 5400
ccataataat actttcactg tacctattaa tagtggtatt gactttgtat atcaacccac 5460
tatgtccatt gagtcaggtc ctgacattcc attaccttcg ttacccacac atactccttt 5520
tgttcctata gcccctacag ctccatctac atctattatt gttgatggta cagattttat 5580
tttacatcct agttattttt tactacgtcg caggcgtaaa cgttttccat atttttttac 5640
agatgtccgt gtggcggcct agtgaggcca ctgtgtacct gcctcctgta cctgtctcta 5700
aggttgtaag cactgatgag tatgtgtctc gcacaagcat ctattattat gcaggcagtt 5760
ctcgattact aacagtagga catccctatt tttctattaa aaacaccagt agtggtaatg 5820
gtaaaaaagt tttagttccc aaggtgtctg gcctgcaata cagggtattt agaattaaat 5880
tgccggaccc taataaattt ggttttccag atacatcttt ttataaccca gaaacccaaa 5940

```
ggttggtgtg ggcctgtaca ggcttggaaa ttggtagggg acagccttta ggtgtgggta   6000
ttagtgggca tcctttatta aacaagtttg atgatactga aaccagtaac aaatatgctg   6060
gtaaacctgg tatagataat agggaatgtt tatctatgga ttataagcag actcagttat   6120
gcattttagg atgcaaacct cctataggtg aacattgggg taagggaacc ccttgtaata   6180
ataattcagg aaatcctggg gattgtcctc ccctacagct cattaacagt gtaatacagg   6240
atggggacat ggtagataca ggatttggtt gcatggattt taataccttg caagctagta   6300
aaagtgatgt gcccattgat atatgtagca gtgtatgtaa gtatccagat tatttgcaaa   6360
tggctagcga gccatatggt gacagtttgt tcttttttct tagacgtgag caaatgtttg   6420
ttagacactt ttttaatagg gccggtacct taggtgaccc tgtgccaggt gatttatata   6480
tacaagggtc taactctggc aatactgcca ctgtacaaag cagtgctttt tttcctactc   6540
ctagtggttc tatggtaacc tcagaatccc aattatttaa taaaccgtac tggttacaac   6600
gtgcgcaggg ccacaataat ggcatatgtt ggggcaatca gttgtttgtc acagttgtgg   6660
ataccactcg tagcactaac atgactttat gtgctgaggt taaaaaggaa agcacatata   6720
aaaatgaaaa ttttaaggaa taccttcgtc atggcgagga atttgattta caatttattt   6780
ttcaattgtg caaaattaca ttaacagctg atgttatgac atacattcat aagatggatg   6840
ccactatttt agaggactgg caatttggcc ttaccccacc accgtctgca tctttggagg   6900
acacatacag atttgtcact tctactgcta taacttgtca aaaaaacaca ccacctaaag   6960
gaaaggaaga tcctttaaag gactatatgt tttgggaggt ggatttaaaa gaaaagtttt   7020
ctgcagattt agatcagttt cctttaggta ggaagttttt gttacaggca gggctacagg   7080
ctaggcccaa actaaaacgc cctgcatcat cggccccacg tacctccaca aagaagaaaa   7140
aggttaaaag gtaaccattg tctgttgggt aattgtctgt gtcatgtatg tgttgtgtat   7200
gtcaaacaca ggttaaaagg taaccattgt ttgttatgta attgttttgt gtgtgtactg   7260
tgttgtttgc atgttatgta tgtgtgtgca tgtttgttgt atttgtcagt tcctgtatgt   7320
atgttttgtg tatgtattaa taaagtactg tatttactaa actatttata gtagtcttat   7380
gttatgttat ggttgcaccc acatgagtaa caatacagtt gctcctaatc tattgcatct   7440
cctgccctac cctgtgtccc ctgccctacc ctgtgtccta ctttgttaca ctactaatta   7500
gccttatact ctccattttg taccattttg tactatccac cattttaaat cctaaccgaa   7560
ttcggttggt cttggcacaa ctttggttgt ccttggcaca gtaacaacta tttttatata   7620
agtttcagca aactgcttaa tcctttggtt tcctgcagtc cactggtcta cacttgttgt   7680
cccgcctaaa ctgacttctt gctgactcac aggtcctgca gtgcagctaa acaatacatt   7740
```

gcctaacatt gcatgtttta aactgctttt aggcacatat tttatttaaa ctttcaatgc 7800

actaattaca gtgttggctt acacaagtac atcctacgcc aaatatgtct tgtaaaacat 7860

gattaaatac tgttactcac caggtgtgca ctacacgacc ggttacggtt accgtaccca 7920

caaccacttt tttttataat ta 7942

<210> 3
<211> 7824
<212> DNA
<213> Human papillomavirus type 58

<400> 3
ctaaactata atgccaaatc ttgtaaaaac tagggtgtaa ccgaaaacgg tctgaccgaa 60

accggtgcat atataaagca gacatttttt ggtaggctac tgcaggacta tgttccagga 120

cgcagaggag aaaccacgga cattgcatga tttgtgtcag gcgttggaga catctgtgca 180

tgaaatcgaa ttgaaatgcg ttgaatgcaa aaagactttg cagcgatctg aggtatatga 240

ctttgtattt gcagatttaa gaatagtgta tagagatgga aatccatttg cagtatgtaa 300

agtgtgctta cgattgctat ctaaaataag tgagtataga cattataatt attcgctata 360

tggagacaca ttagaacaaa cactaaaaaa gtgtttaaat gaaatattaa ttagatgtat 420

tatttgtcaa agaccattgt gtccacaaga aaaaaaaagg catgtggatt taaacaaaag 480

gtttcataat atttcgggtc gttggacagg gcgctgtgca gtgtgttgga gaccccgacg 540

tagacaaaca caagtgtaac ctgtaacaac gccatgagag gaaacaaccc aacgctaaga 600

gaatatattt tagatttaca tcctgaacca actgacctat tctgctatga gcaattatgt 660

gacagctcag acgaggatga aataggcttg gacgggccag atggacaagc acaaccggcc 720

acagctaatt actacattgt aacttgttgt tacacttgtg gcaccacggt tcgtttgtgt 780

atcaacagta caacaaccga cgtacgaacc ctacagcagc tgcttatggg cacatgtacc 840

attgtgtgcc ctagctgtgc acagcaataa acaccatctg caatggatga ccctgaaggt 900

acaaacgggg taggggcggg ctgtactggc tggtttgagg tagaagcggt aatagaacga 960

agaacaggag ataatatttc agatgatgag gacgaaacag cagacgatag tggtacagat 1020

ttaatagagt ttatagatga ttcagtacaa agtactacac aggcagaagc agaggcagcc 1080

cgagcgttgt ttaatgtaca ggaaggggtg gacgatataa atgctgtgtg tgcactaaaa 1140

cgaaagtttg cagcatgctc agaaagtgct gtagaggact gtgtggaccg ggctgcaaat 1200

gtgtgtgtat cgtggaaata taaaaataaa gaatgcacac acagaaaacg aaaaattatt 1260

gagctagaag acagcggata tggcaatact gaagtggaaa ctgagcagat ggcacaccag 1320

gtagaaagcc aaaatggcga cgcagactta aatgactcgg agtctagtgg ggtgggggct 1380

agttcagatg taagcagtga aacggatgta gacagttgta atactgttcc attacaaaat 1440
attagtaata ttctacataa cagtaatact aaagcaacgc tattatataa attcaaagaa 1500
gcttatggag taagttttat ggaattagtt agaccattta aaagtgataa aacaagctgt 1560
acagattggt gtataacagg gtatggaata agtccctccg tagcagaaag tttaaaagta 1620
ctaattaaac agcacagtat atatacacac ctacaatgtt taacgtgtga cagaggaatt 1680
atattattat tgttaattag atttaaatgt agcaaaaata gattaactgt ggcaaaatta 1740
atgagtaatt tactatcaat tcctgaaaca tgtatgatta tcgagccacc aaaattacga 1800
agtcaagcat gtgccttata ttggtttaga acagcaatgt caaatataag tgatgtgcaa 1860
gggacaacac cagaatggat agatagatta acagtgttac agcatagctt taatgatgat 1920
atatttgatt taagtgaaat gatacaatgg gcatatgata atgacattac agatgatagt 1980
gacattgcat ataaatatgc acagttagca gatgttaata gtaatgcagc agcattttta 2040
agaagcaatg cacaagcaaa aatagtaaaa gactgtggcg ttatgtgcag acattataaa 2100
agagcagaaa agcgtggtat gacaatggga caatggatac aaagtaggtg tgaaaaaaca 2160
aatgatggag gtaattggag accaatagta caatttttaa gatatcaaaa tattgaattt 2220
acagcatttt tagttgcatt taaacagttt ttacaaggtg taccaaaaaa aagttgtatg 2280
ttactgtgtg gcccagcaaa tacagggaaa tcatattttg gaatgagttt aatacatttt 2340
ttaaaaggat gcattatttc atatgtaaat tccaaaagtc atttttggtt gcagccatta 2400
tcagatgcta aactaggtat gatagatgat gtaacagcca taagctggac atatatagat 2460
gattatatga gaaatgcatt agatggtaac gacatttcaa tagatgtaaa acatagggca 2520
ttagtacaat taaaatgtcc accattaata attacctcaa atacaaatgc aggcaaagat 2580
tcacgatggc catatttgca cagtagacta acagtatttg aatttaacaa tccatttcca 2640
tttgatgcaa atggtaatcc agtgtataaa ataaatgatg aaaattggaa atcctttttc 2700
tcaaggacgt ggtgcaaatt aggcttaata gaggaagagg acaaggaaaa cgatggagga 2760
aatatcagca cgtttaagtg cagtgcagga caaaatccta gacatatacg aagctgataa 2820
aaatgattta acatcacaaa ttgaacattg gaaactaata cgcatggagt gtgctataat 2880
gtatacagcc agacaaatgg gaatatcaca tttgtgccac caggtggtgc cgtcattggt 2940
agcatcaaag actaaagcgt ttcaagtaat tgaactgcaa atggcattag agacattaaa 3000
tgcatcacca tataaaacag atgaatggac attgcaacaa acaagcttag aagtgtggtt 3060
atcagagcca caaaaatgct ttaaaaaaaa aggcataaca gtaactgtac aatatgacaa 3120
tgataaagca aacacaatgg attatacaaa ttggagtgaa atatatatta ttgaggaaac 3180
aacatgtact ttggtagcag gagaagttga ctatgtgggg ttgtattata tacatggcaa 3240

```
tgaaaagacg tattttaaat attttaaaga ggatgcaaaa aagtactcta aaacacaatt   3300
atgggaggta catgtgggta gtcgggtaat tgtatgtcct acatctatac ctagtgatca   3360
aatatccact actgaaactg ctgacccaaa gaccaccgag gccaccaaca acgaaagtac   3420
acaggggaca aagcgacgac gactcgattt accagactcc agagacaaca cccagtactc   3480
cacaaagtat acagactgcg ccgtggacag tagaccacga ggaggaggac tacacagtac   3540
aactaactgt acatacaaag ggcggaacgt gtgtagttct aaagtttcac ctatcgtgca   3600
tttaaaaggt gacccaaata gtttaaaatg tttaagatat agattaaaac catttaaaga   3660
cttatactgt aatatgtcat ccacatggca ttggaccagt gatgacaaag gtgacaaagt   3720
aggaattgtt actgtaacat acacaacgga aacacaacga caactgtttt taaacactgt   3780
taaaatacca cccactgtgc aaataagtac tggtgttatg tcattgtaat tgtattgtac   3840
aattactgta tgtaaaccac aagccaatat gtgctgctaa gtgtatatac aatgatatta   3900
cctatttttg ttgtttgttt tatactgttt ttatgcttgt gcattttttt gcggccattg   3960
gtgctatcta tttctatata tgcttggttg ctggtgttgg tgttgctgct ttgggtgtct   4020
gtggggtcgg ctctacgaat ttttttctgt tacttaatat ttttatatat accaatgatg   4080
tgtattaatt ttcatgcaca atacttaacc caacaagact aactgtatac tggttctgca   4140
catggtggta tggtattgta aatatttact gttgtgtgtg ttgtttttat tatttttata   4200
catttactaa taaatacttt tatattttta gcactgtctt attatgagac acaaacggtc   4260
tacaaggcgc aagcgtgcat ctgctacaca actttaccaa acatgcaagg cctcaggcac   4320
ctgcccacct gatgttatac ccaaagttga aggcactact atagcagatc aaatattacg   4380
atatggtagc ttaggggtgt tttttggagg tttaggcatt ggtacagggt cgggtacagg   4440
tggcaggact ggatatgtgc cccttggtag taccccaccg tctgaggcta tacctttaca   4500
gcccatacgt cccccagtta ccgttgatac tgtggggcct ttggattctt ctattgtatc   4560
tttaatagag gaatctagtt ttatagacgc cggtgcacca gccccatcaa ttcccactcc   4620
atctggtttt gatattacca cctctgcaga tactacacct gcaatactta atgtttcctc   4680
tattggagaa tcatctatac aaactgtttc tacacattta aatccctcct ttactgagcc   4740
atccgtactc cgccctcctg cacctgcaga ggcctctgga catttaatat tttcctctcc   4800
tactgttagc acacatagtt atgaaaacat accaatggat acctttgtta tttctactga   4860
cagtggcaat gtcacgtcta gcacacccat tccagggtct cgccctgtgg cacgccttgg   4920
tttatacagt cgcaacaccc aacaagttaa ggttgttgac cctgcttttt taacatctcc   4980
tcatagactt gtaacatatg ataatccagc atttgaaggc tttaaccctg aggacacatt   5040
```

gcagtttcaa catagtgaca tatcgcctgc tcctgatcct gattttctag atattgttgc 5100
attacacaga cctgcattaa cctctcgcag ggtactgta cgttatagta gggttgggca 5160
aaaggctaca cttcgtactc gcagtggaaa gcaaataggg gctaaagtac attactacca 5220
agacttaagt cccatacagc ctgtccagga acaggtacaa cagcagcaac aatttgaatt 5280
acaatcttta aatacttctg tttctcccta tagtattaat gatggacttt atgatattta 5340
tgctgacgat gctgatacta tacatgattt tcagagtcct ctgcactcac atacgtcctt 5400
tgccaccaca cgtaccagta atgtgtccat accattaaat actggatttg acactcctct 5460
tgtgtcattg gaacctggtc cagacattgc atcttctgta acatctatgt ctagtccatt 5520
tattcctata tctccactaa ctccttttaa taccataatt gtggatggtg ctgattttat 5580
gttgcaccct agctatttta ttttgcgtcg cagacgtaaa cgttttccat atttttttgc 5640
agatgtccgt gtggcggcct agtgaggcca ctgtgtacct gcctcctgtg cctgtgtcta 5700
aggttgtaag cactgatgaa tatgtgtcac gcacaagcat ttattattat gctggcagtt 5760
ccagactttt ggctgttggc aatccatatt tttccatcaa aagtcccaat aacaataaaa 5820
aagtattagt tcccaaggta tcaggcttac agtatagggt ctttagggtg cgtttacctg 5880
atcccaataa atttggtttt cctgatacat ctttttataa ccctgataca caacgtttgg 5940
tctgggcatg tgtaggcctt gaaataggta ggggacagcc attgggtgtt ggcgtaagtg 6000
gtcatcctta tttaaataaa tttgatgaca ctgaaaccag taacagatat cccgcacagc 6060
cagggtctga taacagggaa tgcttatcta tggattataa acaaacacaa ttatgtttaa 6120
ttggctgtaa acctcccact ggtgagcatt ggggtaaagg tgttgcctgt aacaataatg 6180
cagctgctac tgattgtcct ccattggaac tttttaattc tattattgag gatggtgaca 6240
tggtagatac agggtttgga tgcatggact ttggtacatt gcaggctaat aaaagtgatg 6300
tgcctattga tatttgtaac agtacatgca aatatccaga ttatttaaaa atggccagtg 6360
aaccttatgg ggatagtttg ttcttttttc ttagacgtga gcagatgttt gttagacact 6420
tttttaatag ggctggaaaa cttggcgagg ctgtcccgga tgacctttat attaaagggt 6480
ccggtaatac tgcagttatc caaagtagtg cattttttcc aactcctagt ggctctatag 6540
ttacctcaga atcacaatta tttaataagc cttattggct acagcgtgca caaggtcata 6600
acaatggcat ttgctggggc aatcagttat ttgttaccgt ggttgatacc actcgtagca 6660
ctaatatgac attatgcact gaagtaacta aggaaggtac atataaaaat gataatttta 6720
aggaatatgt acgtcatgtt gaagaatatg acttacagtt tgtttttcag ctttgcaaaa 6780
ttacactaac tgcagagata atgacatata tacatactat ggattccaat attttggagg 6840
actggcaatt tggtttaaca cctcctccgt ctgccagttt acaggacaca tatagatttg 6900

```
ttacctccca ggctattact tgccaaaaaa cagcaccccc taaagaaaag gaagatccat   6960
taaataaata tactttttgg gaggttaact taaaggaaaa gttttctgca gatctagatc   7020
agtttccttt gggacgaaag tttttattac aatcaggcct taaagcaaag cccagactaa   7080
aacgttcggc ccctactacc cgtgcaccat ccaccaaacg caaaaaggtt aaaaaataat   7140
tgttgtggta cttacactat tttattatac atgtttgttt gttttatgta tgtgttgtct   7200
gtttgtttat gtttgtgtat atgttgtatg tgttatgtgt catgtttgtg tacatgttct   7260
atgtccttgt cagtttcctg tttctgtata tatgtaataa actattgtgt gtattgtaaa   7320
ctatttgtat tgtttgggtg tatctatgag taaggtgctg tccctaaatt gccctaccct   7380
gccctgccta ttatgcatac ctatgtaata gtatttgtat gatatgtatt ttatagtttt   7440
taacagtact gcctccattt tactttacct ccattttgtg catgtaaccg atttcggttg   7500
ctggcacaaa cgtgtttttt ttaaactaca atttaaacaa tacagttaat cctttccctt   7560
cctgcactgc ttttgcctat acttgcatat gtgactcata tatacatgca gtgcagttgc   7620
aaaatgttta attatactca tagtttaaac atgcttatag gcacatattt taacttactt   7680
tcaatgctta agtgcagttt tggcttgcac aatagtttgt tatgccaaac tatgtcttgt   7740
aaaagtgact cactaacatt tattgccagg tgtggactaa ccgttttggg tcacattgtt   7800
catgtttcaa cattttatat aata                                          7824
```

<210> 4
<211> 35
<212> DNA
<213> Artificial

<220>
<223> PCR Primer

<400> 4
ttaatattta ttaattgtgt tgtggttatt tattg 35

<210> 5
<211> 35
<212> DNA
<213> Artificial

<220>
<223> PCR Primer

<400> 5
taaccttaaa aatttaaacc ttataccaaa tatac 35

<210> 6
<211> 30
<212> DNA

<213>  Artificial

<220>
<223>  PCR Primer

<400>  6
agtaggattg aaggttaaat taaaatttat    30

<210>  7
<211>  30
<212>  DNA
<213>  Artificial

<220>
<223>  PCR Primer

<400>  7
aacacatttt ataccaaaaa acatacaacc    30

<210>  8
<211>  27
<212>  DNA
<213>  Artificial

<220>
<223>  PCR Primer

<400>  8
aatagggttg gtattgttgg tgaaaat    27

<210>  9
<211>  30
<212>  DNA
<213>  Artificial

<220>
<223>  PCR Primer

<400>  9
ttccaatcct ccaaaataat aaaattcata    30

<210>  10
<211>  31
<212>  DNA
<213>  Artificial

<220>
<223>  PCR Primer

<400>  10
gtattatttt gtattattta ttattttaaa t    31

<210>  11
<211>  33
<212>  DNA
<213>  Artificial

<220>
<223>  PCR Primer

<400>  11
ccctattttt tactaatata aaattataat tta                                33

<210>  12
<211>  30
<212>  DNA
<213>  Artificial

<220>
<223>  PCR Primer

<400>  12
tttatttata tttattattg ttgatggtat                                    30

<210>  13
<211>  25
<212>  DNA
<213>  Artificial

<220>
<223>  PCR Primer

<400>  13
taaaaaacca aatttattaa aatcc                                         25

<210>  14
<211>  23
<212>  DNA
<213>  Artificial

<220>
<223>  PCR Primer

<400>  14
attttagagg attggtaatt tgg                                           23

<210>  15
<211>  26
<212>  DNA
<213>  Artificial

<220>
<223>  PCR Primer

<400>  15
aacctttttc ttctttataa aaatac                                        26

<210>  16
<211>  26
<212>  DNA
<213>  Artificial

<220>
<223> PCR Primer

<400> 16
ttttattttt attttgtgta tgtaat 26

<210> 17
<211> 25
<212> DNA
<213> Artificial

<220>
<223> PCR Primer

<400> 17
taatcctaca ataacctacc aaaaa 25

<210> 18
<211> 24
<212> DNA
<213> Artificial

<220>
<223> PCR Primer

<400> 18
atggtgttga ttttatgttg tatt 24

<210> 19
<211> 25
<212> DNA
<213> Artificial

<220>
<223> PCR Primer

<400> 19
aactatcccc tacctatttc aaaac 25

<210> 20
<211> 24
<212> DNA
<213> Artificial

<220>
<223> PCR Primer

<400> 20
ttaatatttt ggaggattgg taat 24

<210> 21
<211> 30
<212> DNA
<213> Artificial

<220>

<223> PCR Primer

<400> 21
atataataaa ataatataaa taccacaaca 30

<210> 22
<211> 29
<212> DNA
<213> Artificial

<220>
<223> PCR Primer

<400> 22
aattaggttt taaagtaaag tttagatta 29

<210> 23
<211> 29
<212> DNA
<213> Artificial

<220>
<223> PCR Primer

<400> 23
ttatttaaat tataatttaa aaaaaacac 29

<210> 24
<211> 36
<212> DNA
<213> Artificial Sequence

<220>
<223> PCR Primer

<400> 24
ttgttgatgt aggtgatttt tatttatatt ttagtt 36

<210> 25
<211> 29
<212> DNA
<213> Artificial Sequence

<220>
<223> PCR Primer

<400> 25
ccactaatac ccacacctaa taactaacc 29

<210> 26
<211> 22
<212> DNA
<213> Artificial Sequence

<220>
<223> PCR Primer

<400> 26
tttaggtgat tttgtgttag gt 22

<210> 27
<211> 25
<212> DNA
<213> Artificial Sequence

<220>
<223> PCR Primer

<400> 27
tcctctaaaa taataacatc catct 25

<210> 28
<211> 24
<212> DNA
<213> Artificial Sequence

<220>
<223> PCR Primer

<400> 28
ttggttgtat ggattttaat attt 24

<210> 29
<211> 21
<212> DNA
<213> Artificial Sequence

<220>
<223> PCR Primer

<400> 29
aacaaacaac taattacccc a 21

<210> 30
<211> 21
<212> DNA
<213> Artificial Sequence

<220>
<223> PCR Primer

<400> 30
tggttagtga attttatggg g 21

<210> 31
<211> 30
<212> DNA
<213> Artificial Sequence

<220>
<223> PCR Primer

<400> 31
ttacaaaact aaaaaacaaa ctataaatca 30

<210> 32
<211> 31
<212> DNA
<213> Artificial Sequence

<220>
<223> PCR Primer

<400> 32
gatgtaggtg atttttattt atattttagt t 31

<210> 33
<211> 27
<212> DNA
<213> Artificial Sequence

<220>
<223> PCR Primer

<400> 33
atccaactac aaataatcta aatattc 27

<210> 34
<211> 30
<212> DNA
<213> Artificial Sequence

<220>
<223> PCR Primer

<400> 34
gattatgtat tttgggaggt taatttaaaa 30

<210> 35
<211> 30
<212> DNA
<213> Artificial Sequence

<220>
<223> PCR Primer
<400> 35
cataacatac atacacacat aacatactat 30

<210> 36
<211> 28
<212> DNA
<213> Artificial Sequence

<220>
<223> PCR Primer

<400> 36
ggtgattgtt ttttattaga attaaaaa 28

<210> 37
<211> 26
<212> DNA
<213> Artificial Sequence

<220>
<223> PCR Primer

<400> 37
aataccatta ttatatccct aaacac 26

<210> 38
<211> 33
<212> DNA
<213> Artificial Sequence

<220>
<223> PCR Primer

<400> 38
gttatttgat ttaaataaat ttggtttatt tga 33

<210> 39
<211> 27
<212> DNA
<213> Artificial Sequence

<220>
<223> PCR Primer

<400> 39
tccataacac catatccaat atctacc 27

<210> 40
<211> 25
<212> DNA
<213> Artificial Sequence

<220>
<223> PCR Primer

<400> 40
tggtagtttt agattttttg ttgtt 25

<210> 41
<211> 21
<212> DNA
<213> Artificial Sequence

<220>
<223> PCR Primer

<400> 41
ctttacccca atattcccct a 21

```
<210>  42
<211>  25
<212>  DNA
<213>  Artificial Sequence

<220>
<223>  PCR Primer

<400>  42
tttttagtgg ttttatggta atttt                                             25


<210>  43
<211>  29
<212>  DNA
<213>  Artificial Sequence

<220>
<223>  PCR Primer

<400>  43
aaatttttaa caaattacaa cctataata                                         29
```

## Description

### TECHNICAL FIELD

The present invention relates to methods for determining whether or not abnormal cells originated from severe dysplasia or lesion in more advanced stages of uterine cervix are contained in a sample obtained from uterine cervix of a subject and for predicting whether or not uterine cervical tissue progresses to severe dysplasia or lesion in more advanced stages, by detecting methylation of genomic DNA of human papilloma virus (hereinafter also referred to as "HPV") in the sample, and to a primer set used for the methods.

### BACKGROUND ART

It has been known that the infection with HPV accounts for a significant part of risk factors for cervical cancer. In most cases, the infection with HPV is transient in which HPV spontaneously disappears from the infected cells in a certain period after the infection. However, HPV does not disappear from 5 to 10% of the patients infected with HPV, producing a persistent infection and resulting in the development of cervical cancer.

Cervical cancer is developed in the uterine cervical surface epithelium, and is mostly squamous cell cancer. In the conventional examinations for cervical cancer, cells taken from uterine cervix are subjected to the screening by cytological diagnosis. When the cells are diagnosed to be abnormal, then detailed examinations such as histological diagnosis are carried out.

In histological diagnosis of uterine cervix, the cells are classified into three stages, i.e. mild, moderate and severe dysplasias, as the premalignant stages, according to the extent of the emergence of atypical cells in epithelium. When lesion is exacerbated beyond severe dysplasia, it comes to the stage in which cancer cells appear in epithelium. The lesion proceeds to "intraepithelial cancer" in which cancer cells are localized in epithelium and then to "microinvasive squamous cancer" and "invasive squamous cancer" in which cancer cells invade from epithelium to subcutaneous tissue.

Most of mild and moderate dysplasias disappear. In order to avoid overtreatment, most of such lesions are followed-up without any treatment. However, as antecedent lesions of severe dysplasia are highly possible to progress to invasive cancer without any treatment, the patients diagnosed as severe dysplasia may frequently undergo treatment such as surgical operations.

The results of pathological diagnosis by histological diagnosis may vary depending on skill of examiners. Thus, the reproducibility of pathological diagnosis is low; due to this, in some cases, patients who are diagnosed as moderate dysplasia in pathological diagnosis may receive treatment such as surgical operations in fear of delay in the start of treatment for severe dysplasia and invasive cancer. In such case, although delay in the start of treatment may be prevented, there is a possibility for overtreatment.

Therefore, it is important to determine whether or not lesion of a subject is severe dysplasia or in more advanced stages, in order to determine treatment strategies for the subject.

Even when a patient is diagnosed as mild or moderate dysplasia by histological diagnosis of uterine cervix, he/she may later progress to severe dysplasia in some cases. Of course, it is important in view of early treatment to predict beforehand whether lesion will progress to severe dysplasia. However, it was difficult to make such prediction with conventional diagnosis methods.

Histological diagnosis of uterine cervix is carried out by collecting a small amount of uterine cervical tissue with biopsy from a subject who has been determined to be in need by a screening cytological diagnosis, and microscopically observing the tissue stained with hematoxylin-eosin (HE) staining.

Histological diagnosis requires the decision by examiners based on the observations, and cervical cancer may be overlooked depending on for instance the way of preparation of samples to be examined. In addition, because it requires a great deal of skill of examiners, it is difficult to promptly and accurately examine large number of samples.

HPV has a circular double-stranded DNA as a genome and is classified into more than 100 subtypes. Among these subtypes, those highly possible to cause cervical cancer are classified as high-risk HPV. Specific high risk-HPV includes HPV-16, HPV-18, HPV-31, HPV-33, HPV-35, HPV-39, HPV-45, HPV-51, HPV-52, HPV-56, HPV-58, HPV-59, HPV-68, HPV-73 and HPV-82. Among these, HPV-16, HPV-18, HPV-52 and HPV-58 are known as high-risk species which are liable to be detected from patients who have developed cervical cancer. Genomic DNA of HPV contains the regions conserved among subtypes such as L1 region, L2 region and LCR which encode capsid proteins and E1, E2, E4, E5, E6 and E7 regions which encode non-structural proteins.

Recently, T. Turan et al. reported that methylation of 5'-(CG)-3' (CpG) in genomic DNA of HPV such as in L1 region, LCR may be used as an index for detecting cervical cancer (Non Patent Literature 1). T, Turan et al. disclose that methylation in L1 region is detected only in cancer samples with the exception of some antecedent lesions, so that the detection of methylation in L1 region may be an important molecular marker for cancer diagnosis (see Abstract, for example).

WO 2008/071998 (Patent Literature 1) discloses that overmethylation in HPV genome in the samples from patients indicates the progression of diseases caused by HPV infection. Patent Literature 1 specifically discloses the detection of methylation in L2 and E2 regions of HPV genomic DNA.

Patent Literature 1: WO 2008/071998
Non Patent Literature 1: T. Turan et al., "Methylation of human papillomavirus-18 L1 gene: A biomarker of neoplastic progression?" Virology 349 (2006) p.175-183

## SUMMARY OF INVENTION

### Technical Problem

The present invention aims to provide a method which allows more accurate and easier determination on whether or not uterine cervical lesion of a subject is severe dysplasia or lesion in more advanced stages.
The present invention also aims to provide a method which allows more accurate and easier prediction on whether or not uterine cervical lesion of a subject will progress to severe dysplasia or lesion in more advanced stages.
The present invention further aims to provide a primer set which is used for the above methods of detection and determination.

### Solution to Problem

The present inventors have measured the frequency of methylation in L1 region of HPV genomic DNA in the samples obtained from uterine cervix of the patients who have been diagnosed as having lesions in various stages by histological diagnosis. As a result, they have found that the frequency of metylation in L1 region can be an index for determination on whether the lesion is severe dysplasia or lesion in more advanced stages, or is in less advanced stages than severe dysplasia. Further, they have found that the frequency of methylation in L1 region may be an index for determination on whether the lesion which has been diagnosed as in less advanced stages than severe dysplasia will progress to severe dysplasia or lesion in more advanced stages, and completed the present invention.
Thus, the present invention provides:

(1) a method of determining the presence or absence of abnormal cells originated from severe dysplasia or lesion in more advanced stages of uterine cervix in a sample obtained from uterine cervix of a subject, comprising the steps of:

measuring a frequency of methylation of 5'-(CG)-3' (CpG) in L1 region of HPV genomic DNA in the sample; and
determining whether or not the abnormal cells are contained in the sample based on the measured frequency of methylation;

(2) the method according to (1), wherein, in the step of determining, the measured frequency of methylation is compared to a predetermined threshold and the sample is determined to contain the abnormal cells when the frequency of methylation is higher than the threshold;
(3) a method of predicting whether or not uterine cervix tissue of a subject progresses to severe dysplasia or lesion in more advanced stages, comprising the steps of:

measuring a frequency of methylation of 5'-(CG)-3' (CpG) in L1 region of HPV genomic DNA in a sample obtained from uterine cervix of the subject; and
predicting whether or not the tissue progresses to severe dysplasia or lesion in more advanced stages based on the measured frequency of methylation;

(4) the method according to (3), wherein, in the step of predicting, the measured frequency of methylation is compared to a predetermined threshold and the tissue is predicted to progress to severe dysplasia or lesion in more advanced stages when the frequency of methylation is higher than the threshold;
(5) the method according to any one of (1) to (4), wherein the frequency of methylation is obtained by dividing the number of methylated CpG(s) present in said L1 region which is subjected to the measurement of the frequency of methylation by the number of all CpGs present in said L1 region;
(6) the method according to any one of (1) to (5), wherein said L1 region which is subjected to the measurement of the frequency of methylation is a region which comprises at least one CpG existing within 80% from 5'-terminal among all CpGs in the L1 region;

(7) the method according to any one of (1) to (6), wherein HPV is at least one selected from HPV-16, HPV-18, HPV31, HPV33, HPV35, HPV-52 and HPV-58;
(8) the method according to (7), wherein HPV is HPV-16 and wherein said L1 region which is subjected to the measurement of the frequency of methylation is a region which comprises at least one CpG among the $1^{st}$ to $15^{th}$ CpGs from 5'-terminal of L1 region of HPV-16 and does not comprise the $16^{th}$ to $19^{th}$ CpGs;
(9) the method according to (7), wherein HPV is HPV-31 and wherein said L1 region which is subjected to the measurement of the frequency of methylation is a region which comprises at least one CpG among the $1^{st}$ to $17^{th}$ CpGs from 5'-terminal of L1 region of HPV-31 and does not comprise the $18^{th}$ to $22^{nd}$ CpGs;
(10) the method according to (7), wherein HPV is HPV-52 and wherein said L1 region which is subjected to the measurement of the frequency of methylation is a region which comprises at least one CpG among the $1^{st}$ to $17^{th}$ CpGs from 5'-terminal of L1 region of HPV-52 and does not comprise the $18^{th}$ to $22^{nd}$ CpGs;
(11) the method according to (7), wherein HPV is HPV-58 and wherein said L1 region which is subjected to the measurement of the frequency of methylation is a region which comprises at least one CpG among the $1^{st}$ to $19^{th}$ CpGs from 5'-terminal of L1 region of HPV-58 and does not comprise the $20^{th}$ to $25^{th}$ CpGs;
(12) the method according to any one of (1) to (11), wherein severe dysplasia or lesion in more advanced stages includes severe dysplasia, intraepithelial cancer, microinvasive squamous cancer and invasive squamous cancer;
(13) a primer set for determining the presence or absence of abnormal cells originated from severe dysplasia or lesion in more advanced stages of uterine cervix, or for predicting the progress to severe dysplasia or lesion in more advanced stages, which is used in a nucleic acid amplification method for amplification of a region comprising at least one CpG existing within 80% from 5'-terminal among all CpGs in L1 region of HPV genomic DNA, said region having been treated with bisulfite;
(14) the primer set according to (13), wherein HPV is at least one selected from HPV-16, HPV-18, HPV-31, HPV33, HPV35, HPV-52 and HPV-58;
(15) the primer set according to (14), wherein HPV is HPV-16 and wherein the region amplified in the amplification method is a region which comprises at least one CpG among the $1^{st}$ to $15^{th}$ CpGs from 5'-terminal of L1 region of HPV-16 and does not comprise the $16^{th}$ to $19^{th}$ CpGs, said region having been treated with bisulfite;
(16) the primer set according to (14), wherein HPV is HPV-18 and wherein the region amplified in the amplification method is a region which comprises at least one CpG among the $1^{st}$ to $25^{th}$ CpGs from 5'-terminal of L1 region of HPV-18 and does not comprise the $26^{th}$ to $32^{nd}$ CpGs, said region having been treated with bisulfite;
(17) the primer set according to (14), wherein HPV is HPV-31 and wherein the region amplified in the amplification method is a region which comprises at least one CpG among the $1^{st}$ to $17^{th}$ CpGs from 5'-terminal of L1 region of HPV-31 and does not comprise the $18^{th}$ to $22^{nd}$ CpGs, said region having been treated with bisulfite;
(18) the primer set according to (14), wherein HPV is HPV-33 and wherein the region amplified in the amplification method is a region which comprises at least one CpG among the $1^{st}$ to $16^{th}$ CpGs from 5'-terminal of L1 region of HPV-33 and does not comprise the $17^{th}$ to $21^{st}$ CpGs, said region having been treated with bisulfite;
(19) the primer set according to (14), wherein HPV is HPV-35 and wherein the region amplified in the amplification method is a region which comprises at least one CpG among the $1^{st}$ to $13^{th}$ CpGs from 5'-terminal of L1 region of HPV-35 and does not comprise the $14^{th}$ to $17^{th}$ CpGs, said region having been treated with bisulfite;
(20) the primer set according to (14), wherein HPV is HPV-52 and wherein the region amplified in the amplification method is a region which comprises at least one CpG among the $1^{st}$ to $17^{th}$ CpGs from 5'-terminal of L1 region of HPV-52 and does not comprise the $18^{th}$ to $22^{nd}$ CpGs; and
(21) the primer set according to (14), wherein HPV is HPV-58 and wherein the region amplified in the amplification method is a region which comprises at least one CpG among the $1^{st}$ to $19^{th}$ CpGs from 5'-terminal of L1 region of HPV-58 and does not comprise the $20^{th}$ to $25^{th}$ CpGs.

Advantageous Effect of Invention

According to the present methods in which methylation is simply measured in a specific region of HPV genomic DNA in samples obtained from uterine cervix of subjects, it is possible to carry out the determination on whether or not the samples contain abnormal cells originated from clinically important severe dysplasia or lesion in more advanced stages, or the prediction on whether or not uterine cervical tissue progresses to severe dysplasia or lesion in more advanced stages.
The present methods allow accurate determination or prediction independent of skills of examiners, because the frequency of methylation of HPV genomic DNA in samples is measured.

## BRIEF DESCRIPTION OF DRAWINGS

Fig.1 shows the results of detection of methylated CpGs in L1 region or LCR of HPV-16 genomic DNA in samples obtained from subjects.

Fig. 2 shows the results of detection of methylated CpGs in L1 region or LCR of HPV-52 genomic DNA in samples obtained from subjects.

Fig. 3 shows the results of detection of methylated CpGs in L1 region or LCR of HPV-58 genomic DNA in samples obtained from subjects.

Fig. 4 shows total genomic sequence of HPV-16 and the positions of CpGs comprised in L1 region and LCR therein.

Fig. 5 shows total genomic sequence of HPV-52 and the positions of CpGs comprised in L1 region and LCR therein.

Fig. 6 shows total genomic sequence of HPV-58 and the positions of CpGs comprised in L1 region and LCR therein.

Fig.7 illustrates the way of calculation of the frequency of methylation.

Fig. 8 is a scatter diagram showing the frequency of methylation in L1 regions of HPV-16, HPV-58 and HPV-52 in relation to the severity of lesion diagnosed by histological diagnosis for operation samples.

Fig. 9 is a scatter diagram of the frequency of methylation of CpGs existing within 80% from 5'-terminal among CpGs in L1 region in relation to the severity of lesion diagnosed by histological diagnosis.

Fig. 10 is a scatter diagram of the frequency of methylation of CpGs existing at 3'-terminal side beyond 80% from 5'-terminal among CpGs in L1 region in relation to the severity of lesion diagnosed by histological diagnosis.

Fig. 11 shows results of histological diagnosis and of determination based on the frequency of methylation of biopsy samples and of histological diagnosis of operation samples obtained from the same subjects.

Fig. 12 is a scatter diagram showing the frequency of methylation (%) in L1 region at the first visit of patients with different courses.

Fig. 13 is a scatter diagram showing the frequency of methylation (%) in L1 region at the first visit of patients in relation to the severity of lesion diagnosed by histological diagnosis.

## DESCRIPTION OF EMBODIMENTS

As used herein, "frequency of methylation" may indicate a ratio of the number of methylated CpG(s) in an analytical region for methylation. For example, because the number of total CpGs in an analytical region is fixed, the frequency of methylation may be the number of methylated CpG(s) per se in the region. The frequency of methylation can also be a value obtained by dividing the number of methylated CpG(s) in an analytical region by the number of all CpGs in the region. In the present methods, it is preferable that the frequency of methylation is a value obtained by dividing the number of methylated CpG(s) in an analytical region by the number of all CpGs in the region. In such case, the frequency of methylation can be calculated with the following formula I:

$$\text{(Frequency of methylation) (\%)} = \text{(the number of methylated CpG(s) in an analytical region)} / \text{(the number of all CpGs in an analytical region)} \times 100$$

The "analytical region" is the whole or partial L1 region of HPV genomic DNA. Preferably, the analytical region is a region which comprises at least one CpG existing within 80% from 5'-terminal among all CpGs in L1 region. The region more preferably does not comprise CpG(s) existing within 20% from 3'-terminal.

By measuring the frequency of methylation of CpGs within such region, it is possible to more accurately carry out the determination on whether or not a sample obtained from uterine cervix contains abnormal cells originated from severe dysplasia or lesion in more advanced stages or the prediction on the progress to severe dysplasia or lesion in more advanced stages of uterine cervix.

More specifically, for L1 region of HPV-16, the analytical region is preferably a region which comprises at least one CpG among the $1^{st}$ to $15^{th}$ CpGs from 5'-terminal and does not comprise the $16^{th}$ to $19^{th}$ CpGs, among 19 CpGs in total. For L1 region of HPV-52, the analytical region is preferably a region which comprises at least one CpG among the $1^{st}$ to $17^{th}$ CpGs from 5'-terminal and does not comprise the $18^{th}$ to $22^{nd}$ CpGs, among 22 CpGs in total. For L1 region of HPV-58, the analytical region is preferably a region which comprises at least one CpG among the$1^{st}$ to $19^{th}$ CpGs from 5'-terminal and does not comprise the $20^{th}$ to $25^{th}$ CpGs, among 25 CpGs in total.

As used herein, "methylated CpG(s)" and "methylation of CpG(s)" mean that cytosine in a consecutive 5'-(CG)-3' in HPV genomic DNA is methylated at a 5- or 6-position of cytosine base.

As used herein, "L1 region of HPV genomic DNA" is a region defined as L1 region in the sequences which are accessible through public databases showing sequence information of HPV genomic DNA (e.g. GenBank from National Center for Biotechnology Information (NCBI)). For example, total genomic DNA sequence of HPV-16 (GenBank accession number: NC_001526; SEQ ID NO: 1) comprises L1 region from positions 5559 to 7154, genomic DNA sequence of HPV-52 (GenBank accession number: NC_001592; SEQ ID NO: 2) comprises L1 region from positions 5565 to 7154, genomic DNA sequence of HPV-58 (GenBank accession number: NC_001443: SEQ ID NO: 3) comprises L1 region from positions 5565 to 7139, genomic DNA sequence of HPV-18 (GenBank accession number: NC_001357) comprises L1 region from positions 5430 to 7136, genomic DNA sequence of HPV-31 (GenBank accession number: J04353) comprises L1 region from positions 5552 to 7066, genomic DNA sequence of HPV-33 (GenBank accession number: NC_001528) comprises L1 region from positions 5594 to 7093, and genomic DNA sequence of HPV-35 (GenBank accession number: M74117) comprises L1 region from positions 5574 to 7091.

As used herein, "severe dysplasia and lesion in more advanced stages" are lesions classified as "severe dysplasia", "intraepithelial cancer", "microinvasive squamous cancer" and "invasive squamous cancer" based on the classification according to "General Rules for Clinical and Pathological Study of Uterine Cervical Cancer in Japan 1997" by Japan Society of Obstetrics and Gynecology.

The subjects who are diagnosed as these lesions need to undergo treatment such as surgical operations; thus it is clinically important to determine whether or not it is severe dysplasia or lesion in more advanced stages.

The lesions in less advanced stages than "severe dysplasia" are classified to "normal in epithelium", "mild dysplasia" and "moderate dysplasia". Most of the subjects who are diagnosed as these lesions undergo follow-up without any treatment.

As used herein, "abnormal cells" denote atypical cells and cancer cells. Atypical cells denote the cells which are not cancer cells but are recognized with nuclear abnormalities such as nuclear enlargement, increase in chromatin, irregular nucleus and the like.

In the present invention, a sample obtained from uterine cervix of a subject is not specifically limited so long as it contains DNA comprised in swabs collected by smearing uterine cervix or tissues collected from uterine cervix. Preferably, it is a processed sample obtained by treating a swab, tissue or a paraffin block of tissue with an appropriate treatment solution. The treatment solution is preferably a buffer containing a surfactant. The processed sample is more preferably obtained by suspending a swab, tissue or a paraffin block of tissue in the treatment solution and homogenizing the suspension.

Tissue may be collected from uterine cervix by well-known methods in the art such as an excision by surgical operations (conization, total hysterectomy etc.), biopsy carried out under the observation with colposcopy.

DNA contained in the sample may preferably be purified, although it is not necessary. The purification of DNA in the sample may be carried out by well-known methods in the art such as ethanol precipitation, phenol/chloroform extraction, use of commercially available nucleic acid purification kits.

The detection of methylation of CpG(s) which is(are) expected to be methylated in L1 region of HPV genomic DNA in the sample may be carried out by methods well-known in the art. Such methods include Bisulfite Sequencing based on the procedures of treating DNA with bisulfite to transform unmethylated cytosine(s) to uracil(s), amplifying DNA in a target region by PCR and sequencing the amplified DNA region (see, for example, T. Turan et al., "Methylation of human papillomavirus-18 L1 gene: A biomarker of neoplastic progression?" Virology 349 (2006) p.175-183), Methylation-Specific PCR (James G.HERMAN et al., Methylation-specific PCR:A novel PCR assay for methylation status of CpG islands, Proc. Natl. Acad. Sci. USA, Vol.93, pp.9821-9826, September 1996), and a method described in WO 2006/132022 based on the oxidization of methylated cytosine with a guide probe.

According to Bisulfite Sequencing, DNA in a sample is reacted with bisulfite such as sodium bisulfite or potassium bisulfite to transform unmethylated cytosine(s) in DNA to uracil(s). Methylated cytosine(s) is(are) not transformed to uracil(s).

The concentration of bisulfite in the transformation of unmethylated cytosine is not specifically limited so long as unmethylated cytosine(s) in DNA in a sample can be sufficiently transformed. More specifically, the concentration of bisulfite is 1 M or more, preferably 1 M to 15M and more preferably 3M to 10M. When the final concentration of sodium bisulfite added in a sample is 4M, unmethylated cytosine(s) can be transformed to uracil(s) with the incubation at 50˚C to 80˚C for 10 to 90 minutes. When bisulfite is used at lower concentration, the incubation time and temperature may be appropriately changed to such extent that unmethylated cytosine(s) are sufficiently transformed.

Next, DNA which has been reacted with bisulfite is amplified with the primer set of the present invention (see below) by a nucleic acid amplification method. The nucleic acid amplification method is not specifically limited and is a well-known nucleic acid amplification method such as PCR or LAMP. The conditions for nucleic acid amplification method may be appropriately selected by a skilled person in the art according to the method to be used, base sequence of the DNA region to be amplified, base sequence of primers and the like.

The primer set of the present invention can amplify a region which comprises at least one CpG existing within 80% from 5'-terminal among all CpGs in L1 region of HPV genomic DNA and has been treated with bisulfite. Preferably, it can amplify the region which does not comprise CpG(s) existing within 20% from 3'-terminal and has been treated with bisulfite.

The base sequences of primers comprised in the present primer set may be such that they can hybridize with a partial base sequence of DNA comprising the analytical region and having been treated with bisulfite and they can initiate amplification of DNA corresponding to the above region in the nucleic acid amplification method.

When HPV to be analyzed is HPV-16, the primer set preferably amplifies, in a nucleic acid amplification method, a region which comprises at least one CpG among the 1st to 15th CpGs from 5'-terminal of L1 region of HPV-16 and does not comprise the 16th to 19th CpGs and has been treated with bisulfite. Specific primer sets are shown below.

The primer set consisting of the primers having the sequences SEQ ID NOs: 8 and 9 amplifies, in a nucleic acid amplification method, a region which comprises the 11th to 15th CpGs from 5'-terminal of L1 region of HPV-16 and has been treated with bisulfite:

SEQ ID NO: 8: AATAGGGTTGGTATTGTTGGTGAAAAT
SEQ ID NO: 9: TTCCAATCCTCCAAAATAATAAAATTCATA

The primer set consisting of the primers having the sequences SEQ ID NOs: 24 and 25 amplifies, in a nucleic acid amplification method, a region which comprises the 1st to 8th CpGs from 5'-terminal of L1 region of HPV-16 and has been treated with bisulfite:

SEQ ID NO: 24: TTGTTGATGTAGGTGATTTTTATTTATATTTTAGTT
SEQ ID NO: 25: CCACTAATACCCACACCTAATAACTAACC

The primer set consisting of the primers having the sequences SEQ ID NOs: 32 and 33 amplifies, in a nucleic acid amplification method, a region which comprises the 1st to 6th CpGs from 5'-terminal of L1 region of HPV-16 and has been treated with bisulfite:

SEQ ID NO: 32: GATGTAGGTGATTTTTATTTATATTTTAGTT
SEQ ID NO: 33: ATCCAACTACAAATAATCTAAATATTC

When HPV to be analyzed is HPV-18, the primer set preferably amplifies, in a nucleic acid amplification method, a region which comprises at least one CpG among the 1st to 25th CpGs from 5'-terminal of L1 region of HPV-18 and does not comprise the 26th to 32nd CpGs and has been treated with bisulfite. Specific primer set is shown below.

The primer set consisting of the primers having the sequences SEQ ID NOs: 38 and 39 amplifies, in a nucleic acid amplification method, a region which comprises the 9th to 16th CpGs from 5'-terminal of L1 region of HPV-18 and has been treated with the bisulfite:

SEQ ID NO: 38: GTTATTTGATTTAAATAAATTTGGTTTATTTGA
SEQ ID NO: 39: TCCATAACACCATATCCAATATCTACC

When HPV to be analyzed is HPV-31, the primer set preferably amplifies, in a nucleic acid amplification method, a region which comprises at least one CpG among the 1st to 17th CpGs from 5'-terminal of L1 region of HPV-31 and does not comprise the 18th to 22nd CpGs and has been treated with bisulfite. Specific primer set is shown below.

The primer set consisting of the primers having the sequences SEQ ID NOs: 36 and 37 amplifies, in a nucleic acid amplification method, a region which comprises the 11th to 17th CpGs from 5'-terminal of L1 region of HPV-31 and has been treated with bisulfite:

SEQ ID NO: 36: GGTGATTGTTTTTTATTAGAATTAAAAA
SEQ ID NO: 37: AATACCATTATTATATCCCTAAACAC

When HPV to be analyzed is HPV-33, the primer set preferably amplifies, in a nucleic acid amplification method, a region which comprises at least one CpG among the 1st to 16th CpGs from 5'-terminal of L1 region of HPV-33 and does not comprise the 17th to 21st CpGs and has been treated with bisulfite. Specific primer set is shown below.

The primer set consisting of the primers having the sequences SEQ ID NOs: 40 and 41 amplifies, in a nucleic acid amplification method, a region which comprises the 4th to 9th CpGs from 5'-terminal of L1 region of HPV-33 and has been treated with bisulfite:

SEQ ID NO: 40: TGGTAGTTTTAGATTTTTTGTTGTT
SEQ ID NO: 41: CTTTACCCCAATATTCCCCTA

When HPV to be analyzed is HPV-35, the primer set preferably amplifies, in a nucleic acid amplification method, a region which comprises at least one CpG among the 1st to 13th CpGs from 5'-terminal of L1 region of HPV-35 and does not comprise the 14th to 17th CpGs and has been treated with bisulfite. Specific primer set is shown below.

The primer set consisting of the primers having the sequences SEQ ID NOs: 42 and 43 amplifies, in a nucleic acid amplification method, a region which comprises the 8th to 13th CpGs from 5'-terminal of L1 region of HPV-35 and has been treated with bisulfite:

SEQ ID NO: 42: TTTTTAGTGGTTTTATGGTAATTTT
SEQ ID NO: 43: AAATTTTTAACAAATTACAACCTATAATA

When HPV to be analyzed is HPV-52, the primer set preferably amplifies, in a nucleic acid amplification method, a region which comprises at least one CpG among the 1st to 17th CpGs from 5'-terminal of L1 region of HPV-52 and does not comprise the 18th to 22nd CpGs and has been treated with bisulfite. Specific primer sets are shown below.
The primer set consisting of the primers having the sequences SEQ ID NOs: 12 and 13 amplifies, in a nucleic acid amplification method, a region which comprises the 1st to 9th CpGs from 5'-terminal of L1 region of HPV-52 and has been treated with bisulfite:

SEQ ID NO: 12: TTTATTTATATTTATTATTGTTGATGGTAT
SEQ ID NO: 13: TAAAAAACCAAATTTATTAAAATCC

The primer set consisting of the primers having the sequences SEQ ID NOs: 26 and 27 amplifies, in a nucleic acid amplification method, a region which comprises the 13th to 18th CpGs from 5'-terminal of L1 region of HPV-52 and has been treated with bisulfite:

SEQ ID NO: 26: TTTAGGTGATTTTGTGTTAGGT
SEQ ID NO: 27: TCCTCTAAAATAATAACATCCATCT

The primer set consisting of the primers having the sequences SEQ ID NOs: 28 and 29 amplifies, in a nucleic acid amplification method, a region which comprises the 10th to 15th CpGs from 5'-terminal of L1 region of HPV-52 and has been treated with bisulfite:

SEQ ID NO: 28: TTGGTTGTATGGATTTTAATATTT
SEQ ID NO: 29: AACAAACAACTAATTACCCCA

When HPV to be analyzed is HPV-58, the primer set preferably amplifies, in a nucleic acid amplification method, a region which comprises at least one CpG among the 1st to 19th CpGs from 5'-terminal of L1 region of HPV-58 and does not comprise the 20th to 25th CpGs and has been treated with bisulfite. Specific primer sets are shown below.
The primer set consisting of the primers having the sequences SEQ ID NOs: 18 and 19 amplifies, in a nucleic acid amplification method, a region which comprises the 1st to 9th CpGs from 5'-terminal of L1 region of HPV-58 and has been treated with bisulfite:

SEQ ID NO: 18: ATGGTGTTGATTTTATGTTGTATT
SEQ ID NO: 19: AACTATCCCCTACCTATTTCAAAAC

The primer set consisting of the primers having the sequences SEQ ID NOs: 30 and 31 amplifies, in a nucleic acid amplification method, a region which comprises the 12th to 19th CpGs from 5'-terminal of L1 region of HPV-58 and has been treated with bisulfite:

SEQ ID NO: 30: TGGTTAGTGAATTTTATGGGG
SEQ ID NO: 31: TTACAAAACTAAAAAACAAACTATAAATCA

Then, the DNA region amplified by a nucleic acid amplification method is sequenced. When the base at the position which is expected to be cytosine according to the sequence information obtained from the public database described above is transformed to uracil (thymine), this base can be determined to be unmethylated cytosine.
When the base at the position which is expected to be cytosine is found to be cytosine in the sequencing, it can be determined to be methylated cytosine.
The sequencing can be carried out by well-known methods in the art, for example, by using a DNA sequencer.
By detecting the number of methylated CpG(s) in L1 region of HPV genomic DNA which has been analyzed according to the above method, the frequency of methylation can be measured. The frequency of methylation can also be measured by dividing the number of methylated CpG(s) by the number of all CpGs in the region.
Based on the frequency of methylation measured as above, the determination on whether or not a sample from a subject contains abnormal cells originated from severe dysplasia or lesion in more advanced stages of uterine cervix or the prediction on whether or not uterine cervical tissue of a subject progresses to severe dysplasia or lesion in more advanced stages is carried out. Such detection and prediction can be carried out by, for example, comparing the frequency of methylation measured and a predetermined threshold.

More specifically, when the frequency of methylation measured for a sample from a subject is higher than the predetermined threshold, the sample can be judged to contain abnormal cells. This is synonymous with judging that the sample does not contain abnormal cells when the frequency of methylation is at or lower than the threshold.

Similarly, when he frequency of methylation measured for a sample from a subject is higher than the predetermined threshold, it can be predicted that uterine cervical lesion of the subject may progress to severe dysplasia or lesion in more advanced stages, even when lesion of the subject has been diagnosed as in less advanced stages than "severe dysplasia" according to the conventional diagnosis methods. This is synonymous with that predicting the lesion may not progress to severe dysplasia or lesion in more advanced stages when the frequency of methylation is at or lower than the threshold.

When the frequency of methylation is at or lower than the threshold, it is also possible to predict that uterine cervical lesion of the subject may disappear.

The threshold to be used for the determination of presence of absence of abnormal cells described above can be decided based on the known severity of lesions from patients and the frequency of methylation in L1 region of HPV genomic DNA in the samples from these patients.

The threshold may be decided as specifically described below. For determination of a threshold, a sample is used which is obtained from uterine cervix of a subject whose severity of lesion has been known according to the method other than the present method such as histological diagnosis. The frequency of methylation in L1 region of HPV genomic DNA for this sample is measured. The result of the measurement is then compared to the severity of lesion determined by the method other than the present method. Then, the threshold can be the value of the frequency of methylation that can most clearly differentiate the frequency of methylation for a sample of a subject who has been determined to have severe dysplasia or lesion in more advanced stages according to the method other than the present method and the frequency of methylation for a sample of a subject who has been determined to have lesion in less advanced stages than severe dysplasia.

For example, the analytical region is set to a region which contains 10 CpGs in total in L1 region. When, for this region, more than 2 methylated CpGs are detected for a sample of a subject who has been determined to have severe dysplasia or lesion in more advanced stages according to histological diagnosis and 1 or less methylated CpG is detected for a sample from a subject who has been determined to have lesion in less advanced stages than severe dysplasia according to histological diagnosis, a threshold can be 2. Thus, when the number of methylated CpGs in a sample from a subject is measured as 2 or more, it can be determined that the sample contains abnormal cells originated from severe dysplasia or lesion in more advanced stages.

When the frequency of methylation is calculated according to the above formula I, a threshold can be 20% in the above example. Thus, when the ratio of methylated CpGs relative to the total CpGs in a sample from a subject is measured as 20% or more, it can be determined that the sample contains abnormal cells originated from severe dysplasia or lesion in more advanced stages.

The threshold to be used for the determination of presence or absence of abnormal cells can also be used for the prediction on progression of lesion. The threshold can also be determined based on the information on progression of lesion of a patient whose progression is known and the frequency of methylation in L1 region of HPV genomic DNA in a sample from the patient collected during a follow-up of progression.

The threshold in this case may be decided as specifically described below. For determination of a threshold, a sample is used which is obtained from a subject, during a follow-up of progression, whose progression information is known whether lesion has been disappeared or progressed to severe dysplasia or lesion in more advanced stages. The frequency of methylation in L1 region of HPV genomic DNA for this sample is measured. The result of the measurement is compared to the progression information. Then, the threshold can be the value of the frequency of methylation which can most clearly differentiate the frequency of methylation for a sample of a subject whose progression information corresponds to the progression to severe dysplasia or lesion in more advanced stages and the frequency of methylation for a sample of a subject whose progression information corresponds to the disappearance of lesion.

## Examples

The present invention is illustrated in more detail by the following Examples which do not intend to limit the present invention.

Example 1

1. Samples

A paraffin block of uterine cervical tissue which was surgically obtained from a subject was sectioned and classified to "mild dysplasia (CIN1)", "moderate dysplasia (CIN2) or "severe dysplasia (CIN3 or more)" by histological diagnosis (hematoxylin-eosin staining).
To three paraffin block sections with 10 m thick from the same subject was added 1 ml xylene and mixed. The mixture was centrifuged and then the supernatant was discarded. The precipitate was added with 1 mL of 100% ethanol to wash. This washing with ethanol was repeated once more. The washed precipitate was incubated at 37˚C for 10 minutes to evaporate ethanol. Thus obtained precipitate is hereinafter referred to as an "operation sample".
A paraffin block of a sample obtained from a subject by scraping a part of uterine cervical tissue under observation with colposcopy was sectioned and classified for the severity of lesion by histological diagnosis as described above.
To three paraffin block sections with 10 m thick from the same subject was added 1 ml xylene and mixed. The mixture was centrifuged and then the supernatant was discarded. The precipitate was added with 1 mL of 100% ethanol to wash. This washing with ethanol was repeated once more. The washed precipitate was incubated at 37˚C for 10 minutes to evaporate ethanol. Thus obtained precipitate is hereinafter referred to as a "biopsy sample".

2. Bisulfite sequencing

2-1. Bisulfite treatment

To the above operation sample or biopsy sample was added 500 l of the solution containing 1% (w/v) SDS and 0.1M NaOH. The obtained mixture was incubated at 100˚C for 20 minutes. The incubated mixture was centrifuged at 4˚C and the supernatant was collected.
To the obtained supernatant was added 500 l of 10M bisulfite solution and mixed. The obtained mixture was incubated at 80˚C for 40 minutes to carry out a bisulfite treatment. Nucleic acid contained in the bisulfite treated solution was purified with a nucleic acid purification kit (QIAquick PCR purification kit, QIAGEN). Sodium hydroxide was added to the obtained nucleic acid to the final concentration of 0.3M. The obtained mixture was incubated at room temperature for 5 minutes. Thus obtained product was purified on a spin column for nucleic acid purification (MicroSpin S-300 HR Columns, GE Healthcare) to obtain 50 l of a bisulfite treated template DNA sample.

2-2. PCR reaction

PCR was carried out on nucleic acid in the bisulfite treated template DNA sample with primer sets shown in the following Table 1.
These primer sets have sequences suitable for amplifying partial regions of HPV genomic DNAs shown as "Amplified region" in Table 1.
[Table 1]

| Target HPV | SEQ ID NO: | Name | Amplified region | Amplified size | Sequence |
|---|---|---|---|---|---|
| HPV-16 | 4 | 2F(16) | LCR | 436bp | TTAATATTTATTAATTGTGTTGTGGTTATTTATTG |
| | 5 | 2R(16) | | | TAACCTTAAAAATTTAAACCTTATACCAAATATAC |
| | 24 | 5F(16) | L1 Anterior * | 439bp | TTGTTGATGTAGGTGATTTTTATTTATATTTTAGTT |
| | 25 | 5R(16) | | | CCACTAATACCCACACCTAATAACTAACC |
| | 6 | 7F(16) | L1-LCR | 440bp | AGTAGGATTGAAGGTTAAATTAAAATTTAT |
| | 7 | 7R(16) | | | AACACATTTTATACCAAAAAACATACAACC |
| | 8 | 6F(16) | L1 | 424bp | AATAGGGTTGGTATTGTTGGTGAAAAT |
| | 9 | 6R(16) | | | TTCCAATCCTCCAAAATAATAAAATTCATA |
| HPV-52 | 10 | 1 F-2 (52) | LCR | 455bp | GTATTATTTTGTATTATTTATTATTTTAAAT |
| | 11 | 1R-2 (52) | | | CCCTATTTTTTACTAATATAAAATTATAATTTA |
| | 26 | 5F(52) | L1 Middle* | 408bp | TTTAGGTGATTTTGTGTTAGGT |
| | 27 | 5R(52) | | | TCCTCTAAAATAATAACATCCATCT |
| | 28 | 4F(52) | L1 Middle* | 385bp | TTGGTTGTATGGATTTTAATATTT |
| | 29 | 4R(52) | | | AACAAACAACTAATTACCCCA |
| | 12 | 2F(52) | L1 Anterior | 367bp | TTTATTTATATTTATTATTGTTGATGGTAT |
| | 13 | 2R(52) | | | TAAAAAACCAAATTTATTAAAATCC |
| | 14 | 3F(52) | L1 Posterior* | 299bp | ATTTTAGAGGATTGGTAATTTGG |
| | 15 | 3R(52) | | | AACCTTTTTCTTCTTTATAAAAATAC |

(continued)

| Target HPV | SEQ ID NO: | Name | Amplified region | Amplified size | Sequence |
|---|---|---|---|---|---|
| HPV-58 | 16 | 1F(58) | LCR | 471 bp | TTTTATTTTTATTTTGTGTATGTAAT |
| | 17 | 1R-2 (58) | | | TAATCCTACAATAACCTACCAAAAA |
| | 30 | 5F(58) | L1 Middle* | 427bp | TGGTTAGTGAATTTTATGGGG |
| | 31 | 5R-2 (58) | | | TTACAAAACTAAAAAACAAACTATAAATCA |
| | 18 | 2F(58) | L1 Anterior | 415bp | ATGGTGTTGATTTTATGTTGTATT |
| | 19 | 2R(58) | | | AACTATCCCCTACCTATTTCAAAAC |
| | 20 | 3F(58) | L1 Posterior* | 344bp | TTAATATTTTGGAGGATTGGTAAT |
| | 21 | 3R(58) | | | ATATAATAAAATAATATAAATACCACAACA |
| | 22 | 4F(58) | L1-LCR | 489bp | AATTAGGTTTTAAAGTAAAGTTTAGATTA |
| | 23 | 4R(58) | | | TTATTTAAATTATAATTTAAAAAAAACAC |
| "L1 Anterior" means a region proximal to 5'-terminal of L1 region, "L1 Posterior" mans a region proximal to 3'-terminal of L1 region and "L1 Middle" means a central region of L1 region. | | | | | |

The composition of PCR reaction solution is as shown below. DNA polymerase used was Ex Taq® Polymerase (TaKaRa Bio Inc.).

| | | |
|---|---|---|
| 10 x buffer | | 1.5 l |
| 2.5 mM dNTP | | 1.2 l |
| Forward primer (10 M) | 0.6 l | |
| Reverse primer (10 M) | 0.6 l | |
| DNA polymerase | 0.075 l | |
| Water | | 9.025 l |
| Template DNA sample | 2 l | |
| Total | | 15 l |

The following Table 2 shows the PCR conditions.

[Table 2]

| | HPV-16/HPV-52 | | HPV-58 | |
|---|---|---|---|---|
| | Temp. (˚C) | Time | Temp. (˚C) | Time |
| Hot start | 95 | 4.5 min. | 95 | 4.5 min. |
| Denaturing | 95 | 30 sec. | 95 | 30 sec. |
| Annealing | 52 | 30 sec. | 54 | 30 sec. |
| Extension | 72 | 40 sec. | 72 | 40 sec. |
| Cycles | 40 or 45 | | 40 or 45 | |

2-3. TA cloning and DNA sequencing

The PCR amplified product was incorporated into a vector provided with TA Cloning Kit (Invitrogen). The obtained construct was used for the transformation of *Escherichia coli* TOP 10. The transformed *E. coli* was cultured overnight on a LB agar medium (1% (w/v) trypton, 0.5% (w/v) yeast extract, 1% (w/v) sodium chloride, and 1.5% (w/v) agar) at 37˚C.

Among thus obtained *E. coli* colonies, bacteria harboring the vector containing the PCR product was selected by colony-PCR method and incubated overnight in LB liquid medium at 37˚C.

A glycerol stock of the cultured *E. coli* was prepared and DNA sequencing of the vector contained in bacteria was carried out at TaKaRa Bio Inc.

Cytosine was determined to be methylated when it was expected to be cytosine based on the sequences SEQ ID NOs: 1 to 3 and it was determined to be cytosine according to the above sequencing. Cytosine was determined to be not methylated when it was expected to be cytosine based on the sequences SEQ ID NOs: 1 to 3 but it was determined to be transformed to uracil (thymine).

3. Results

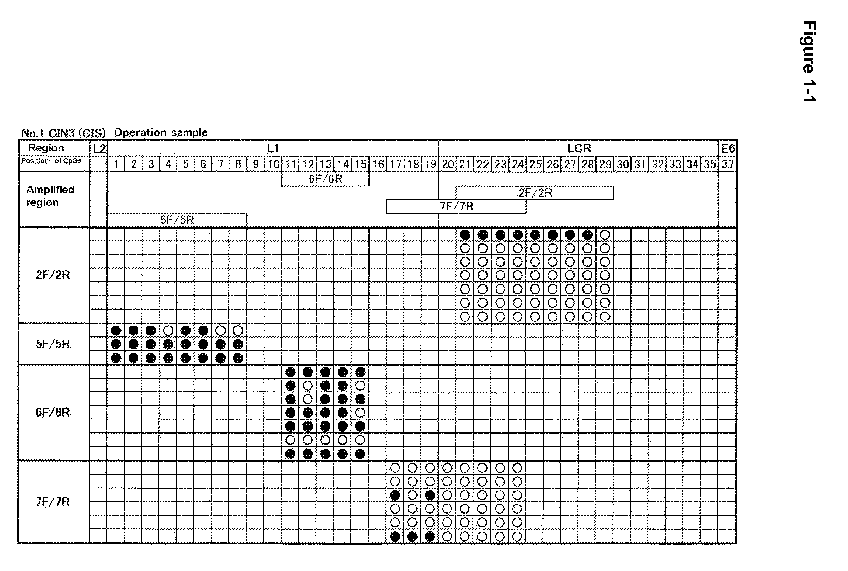

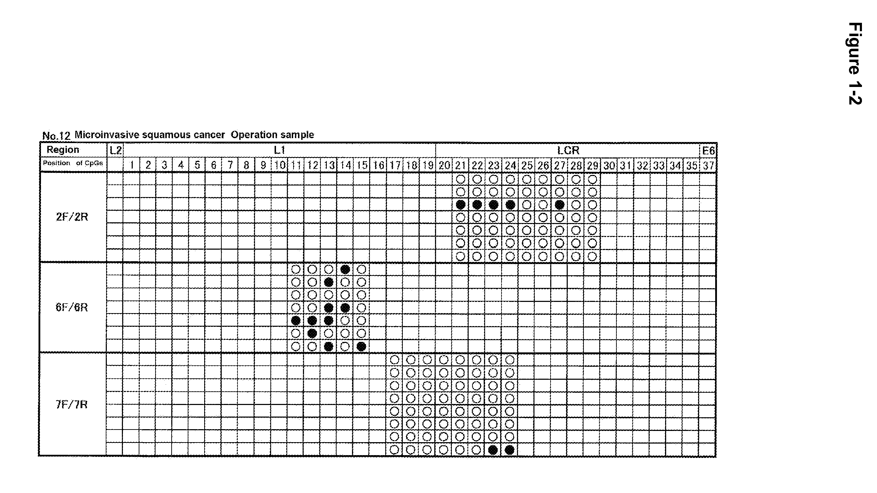

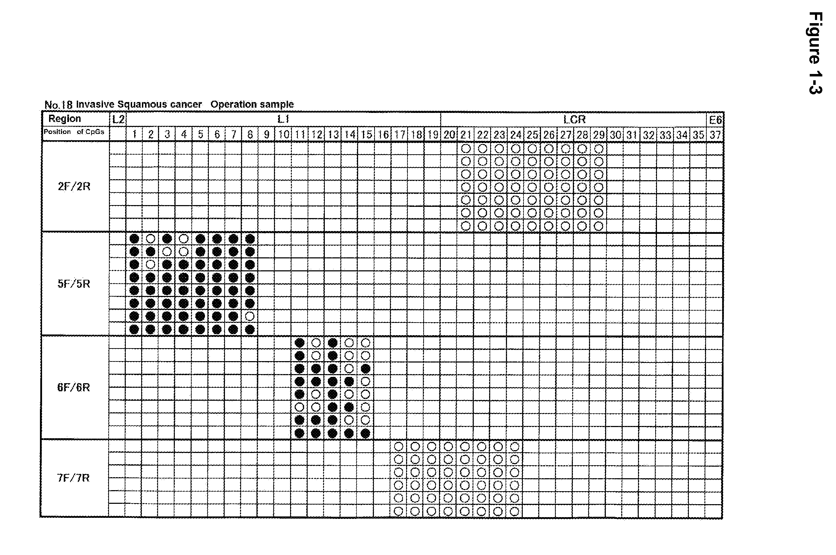

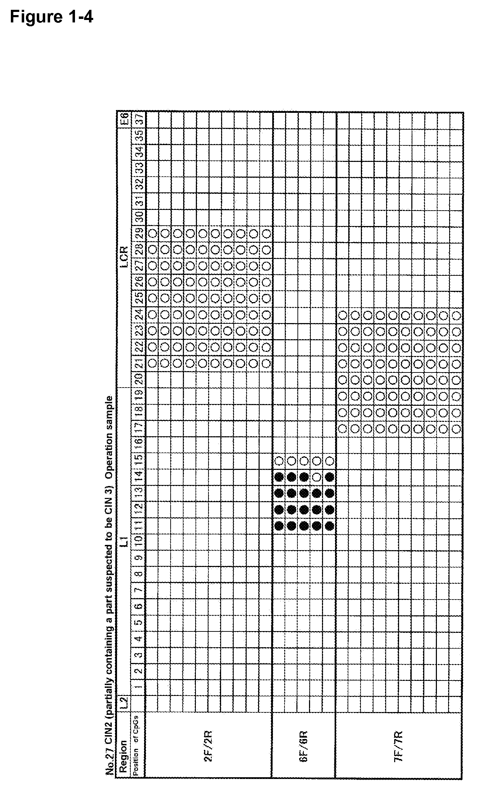

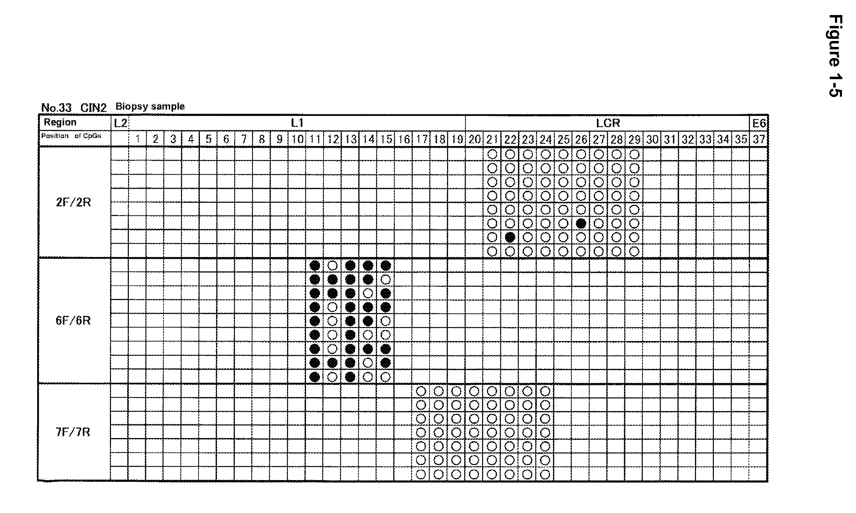

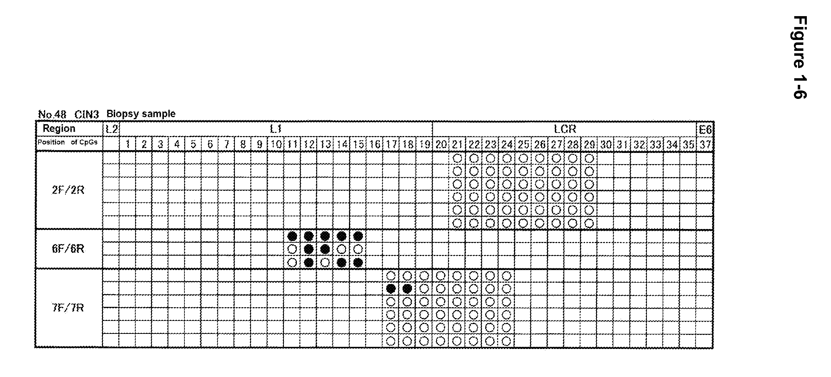

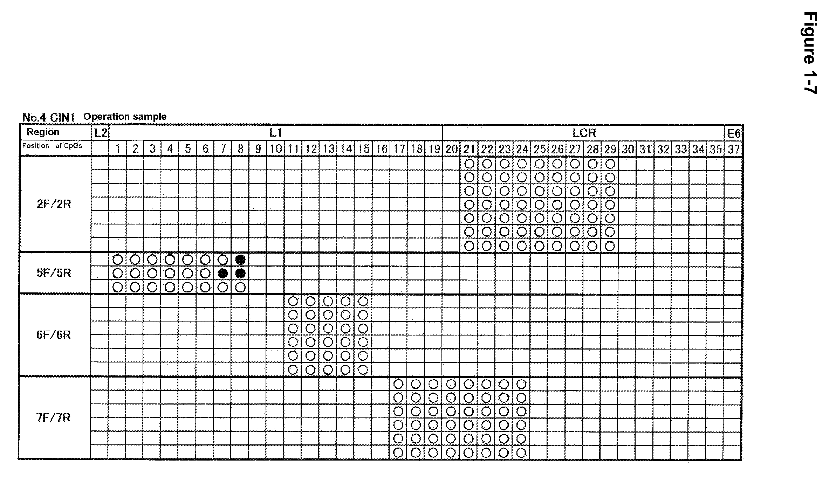

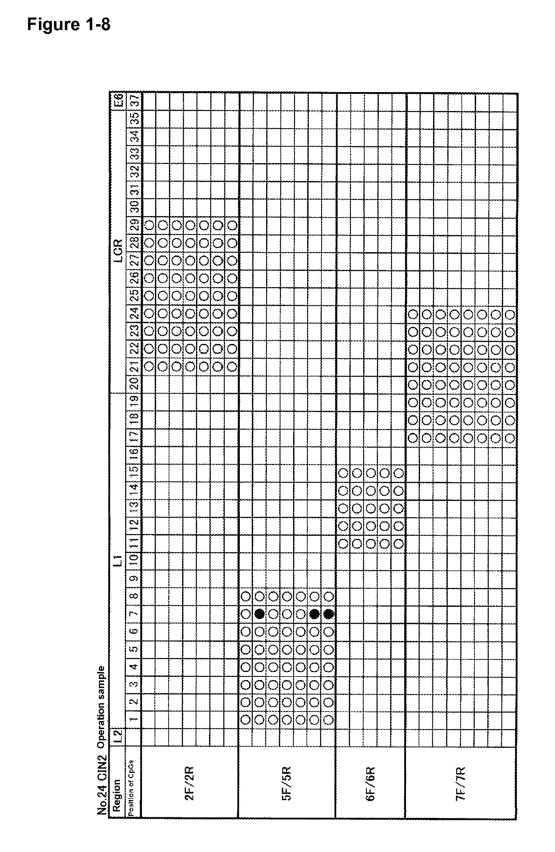

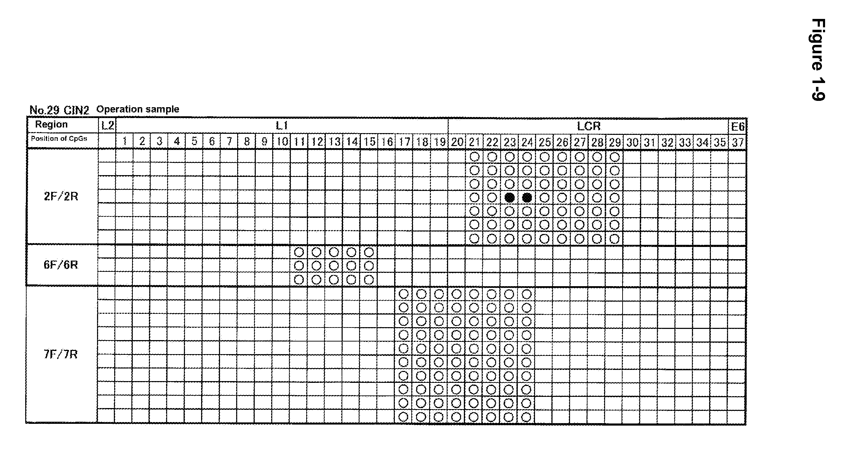

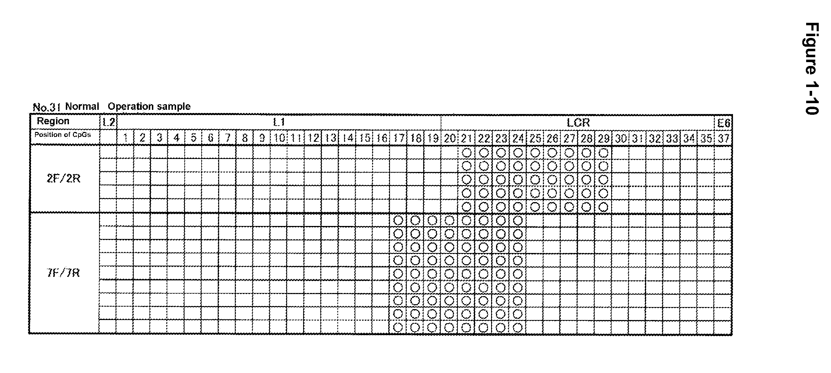

As described above, methylated CpGs were detected in L1 or LCR region of HPV genomic DNA in samples. Some of the results are shown in Figs. 1 to 3.

Fig. 1 shows the results of detection of methylated CpGs in L1 region or LCR of HPV-16 genomic DNA.

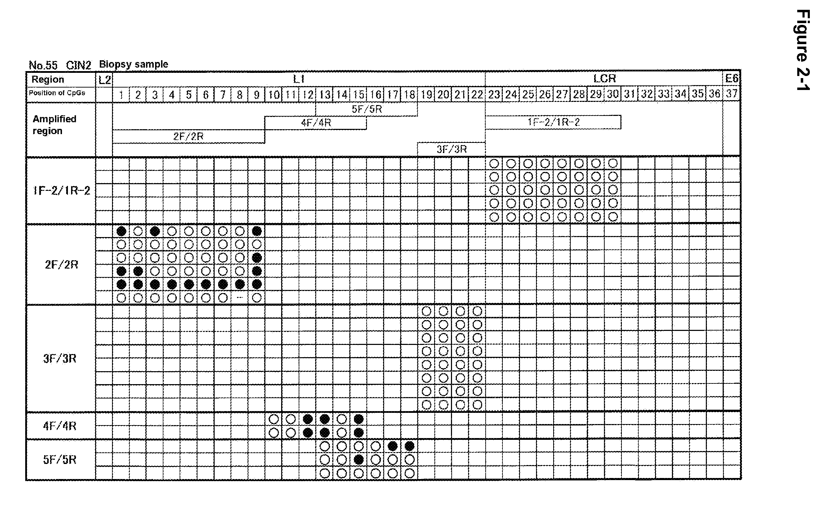

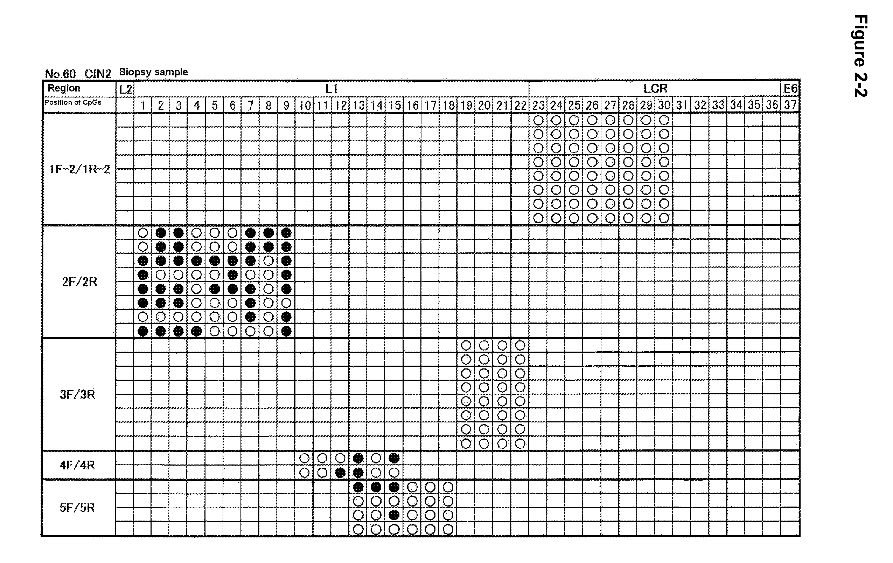

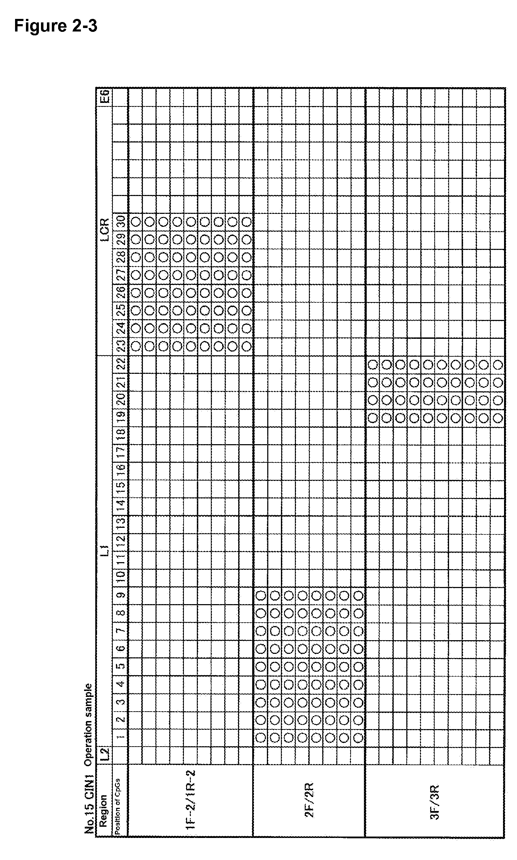

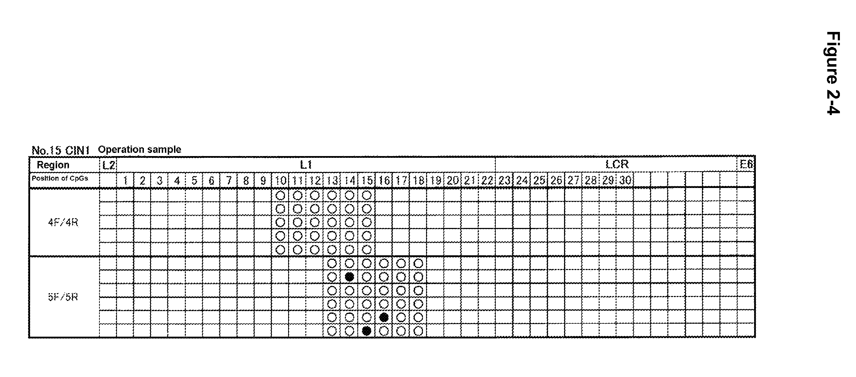

Fig. 2 shows the results of detection of methylated CpGs in L1 region or LCR of HPV-52 genomic DNA.

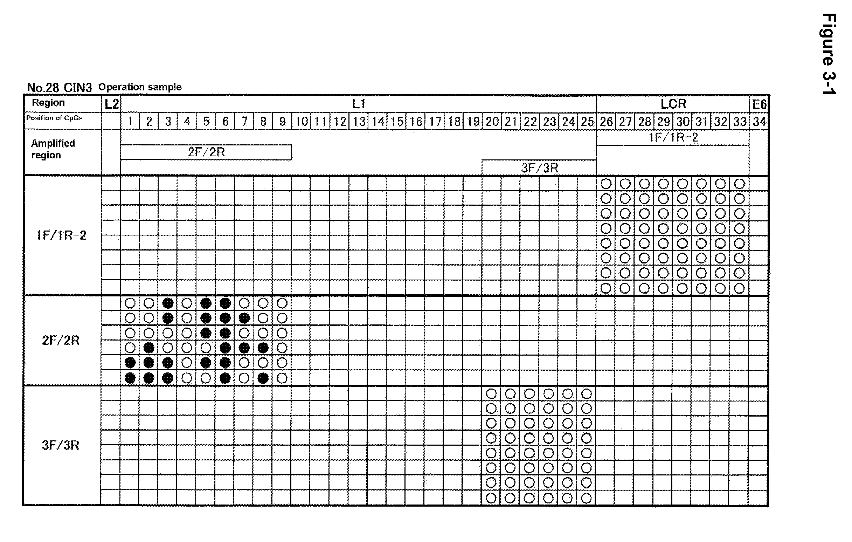

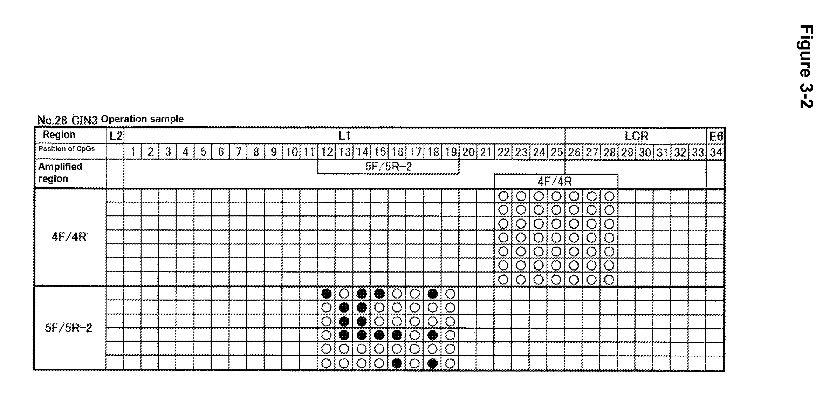

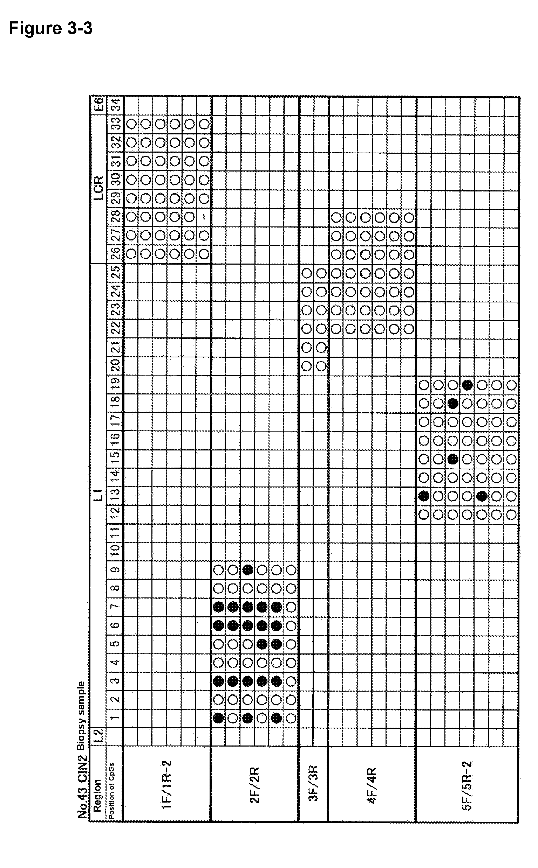

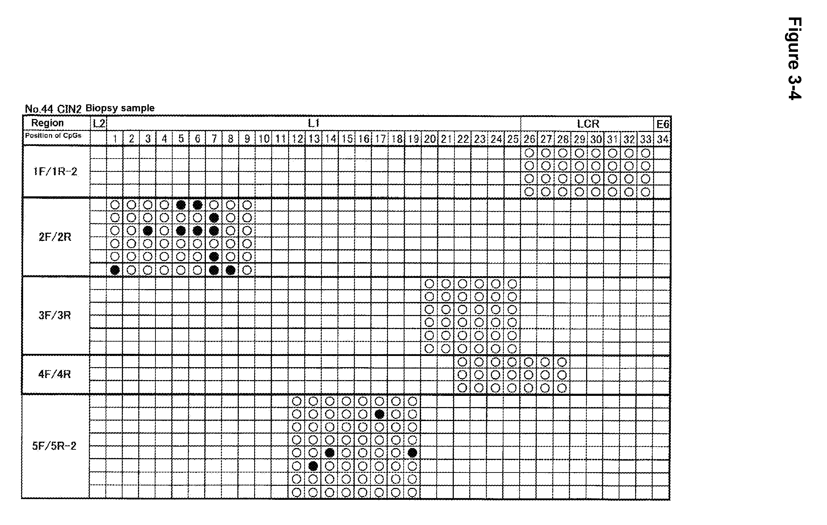

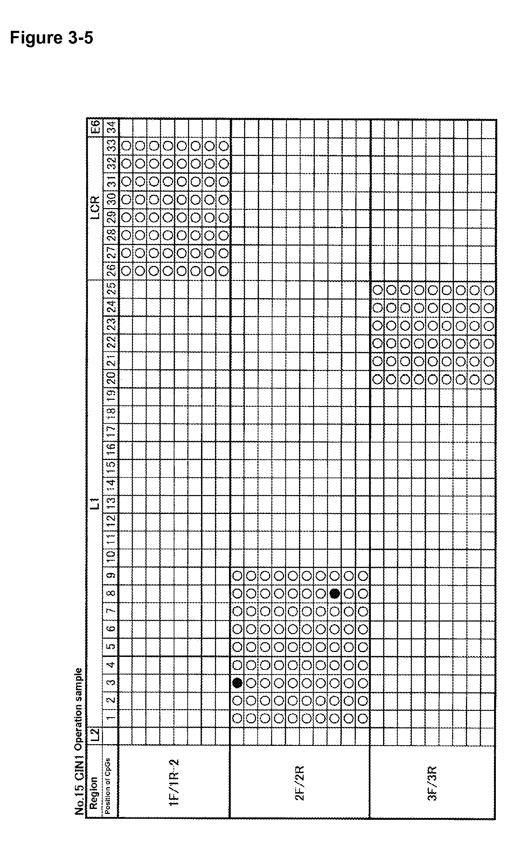

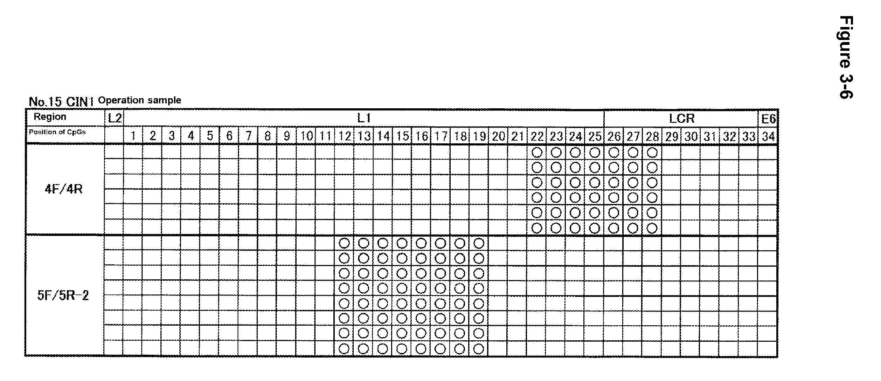

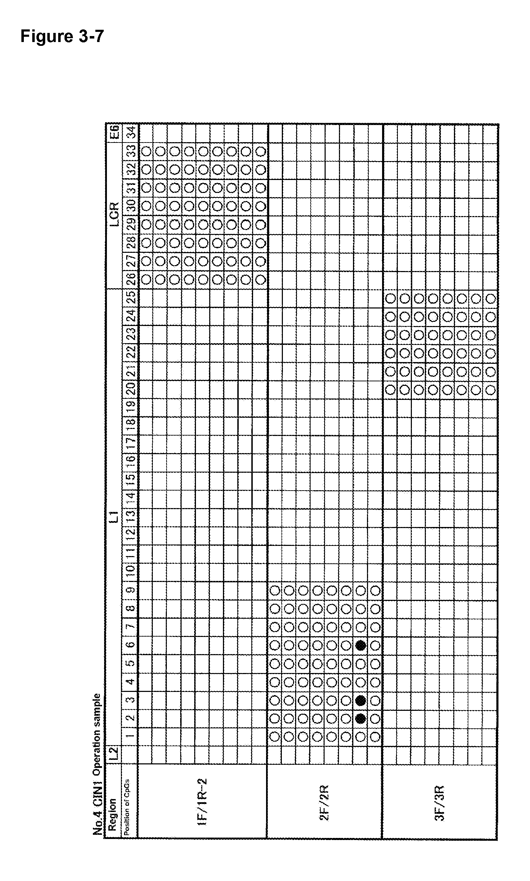

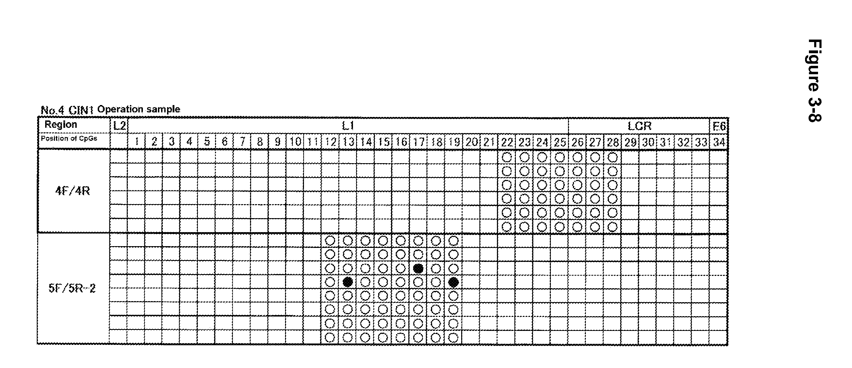

Fig. 3 shows the results of detection of methylated CpGs in L1 region or LCR of HPV-58 genomic DNA.

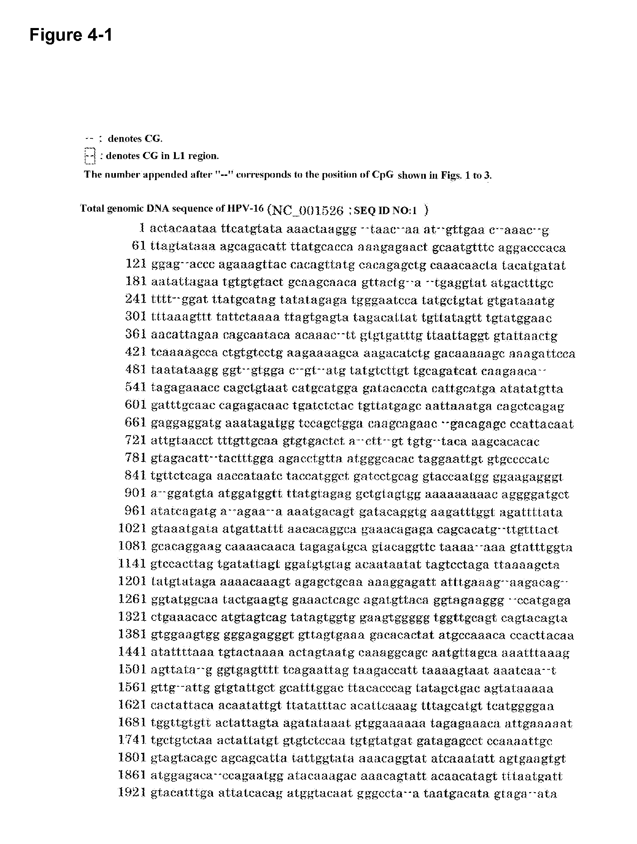

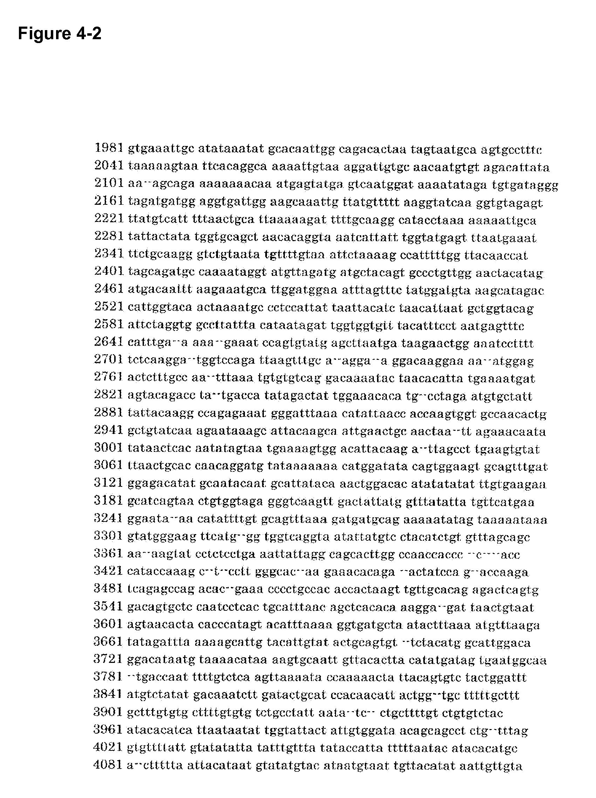

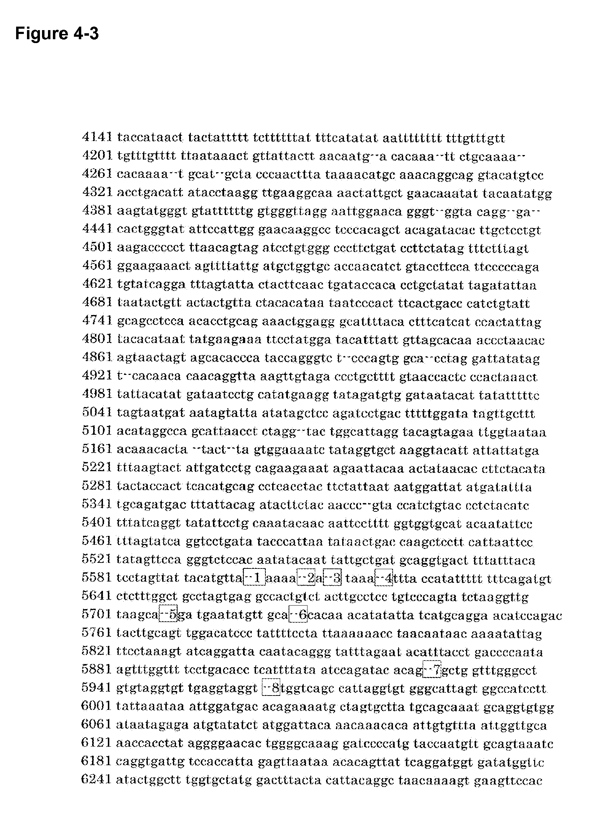

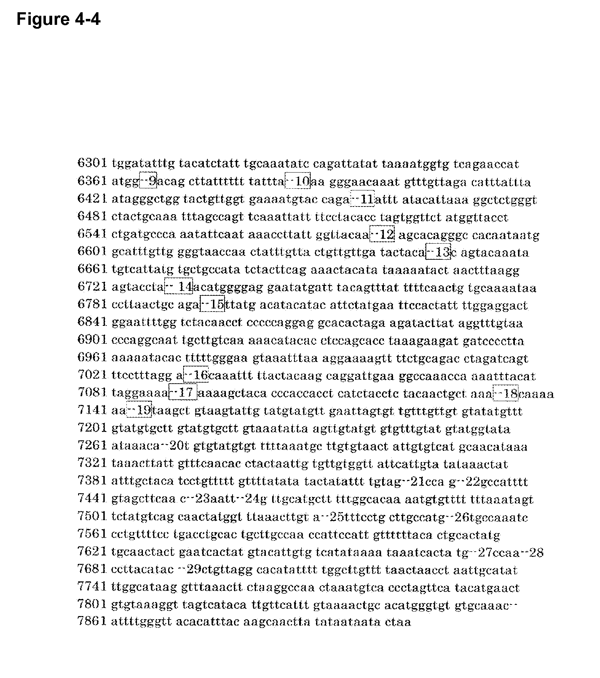

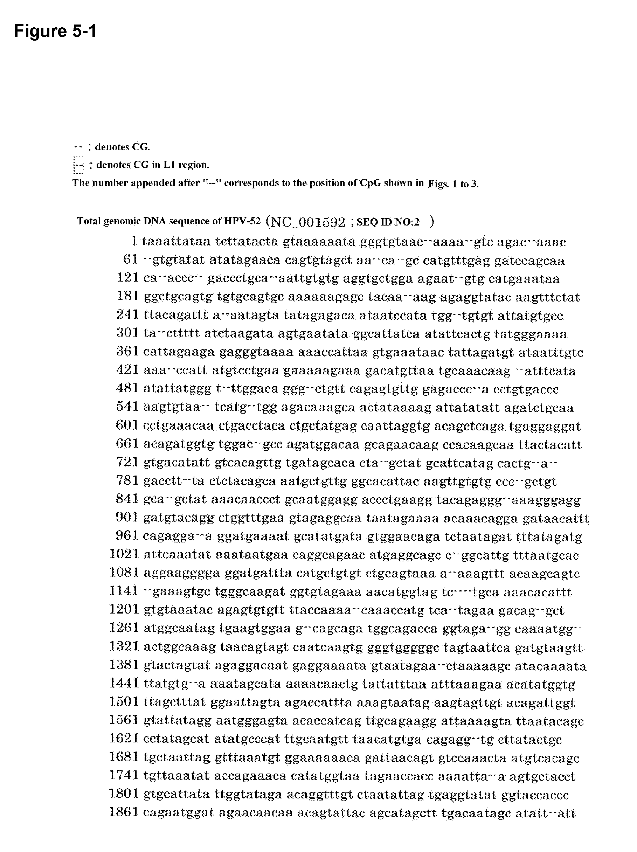

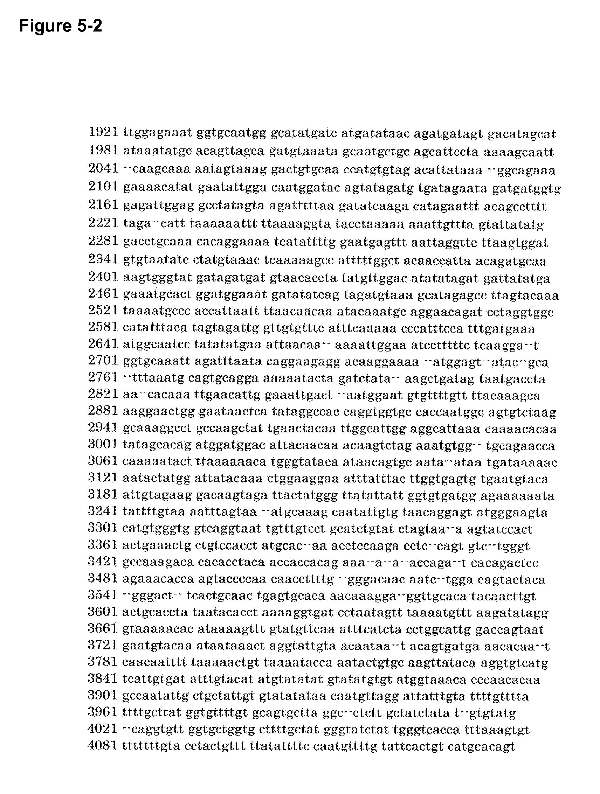

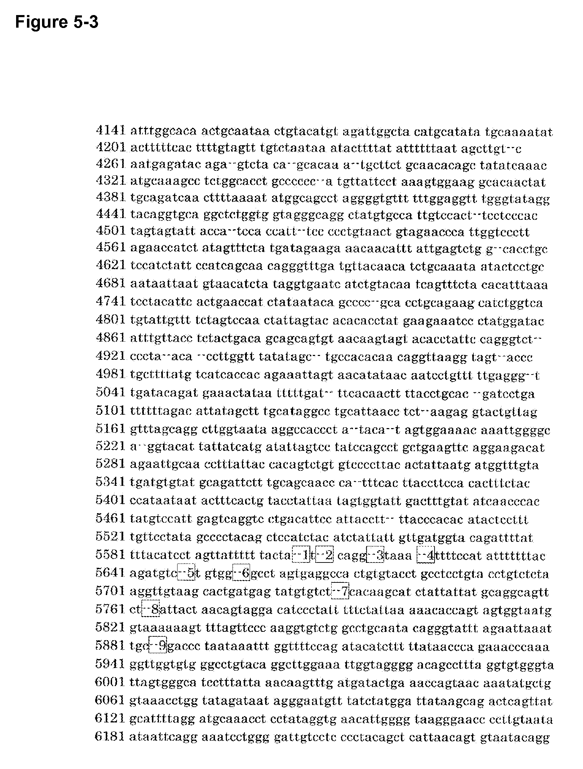

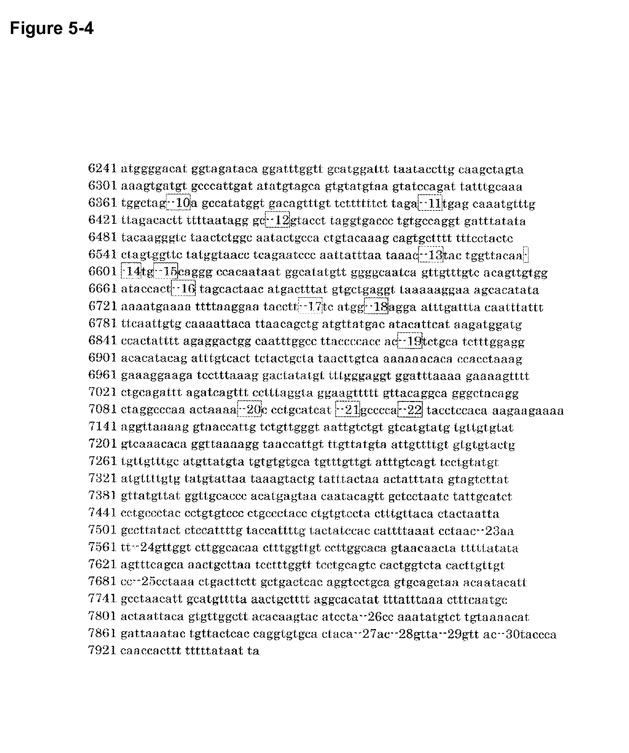

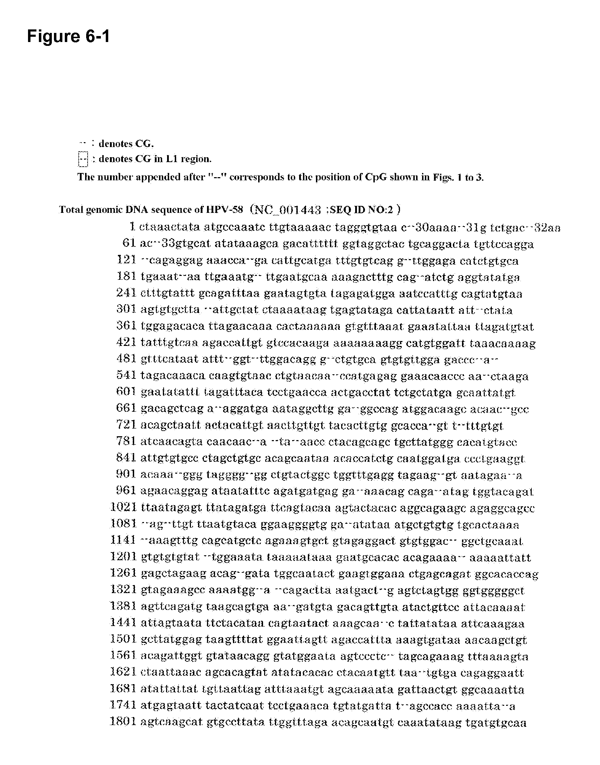

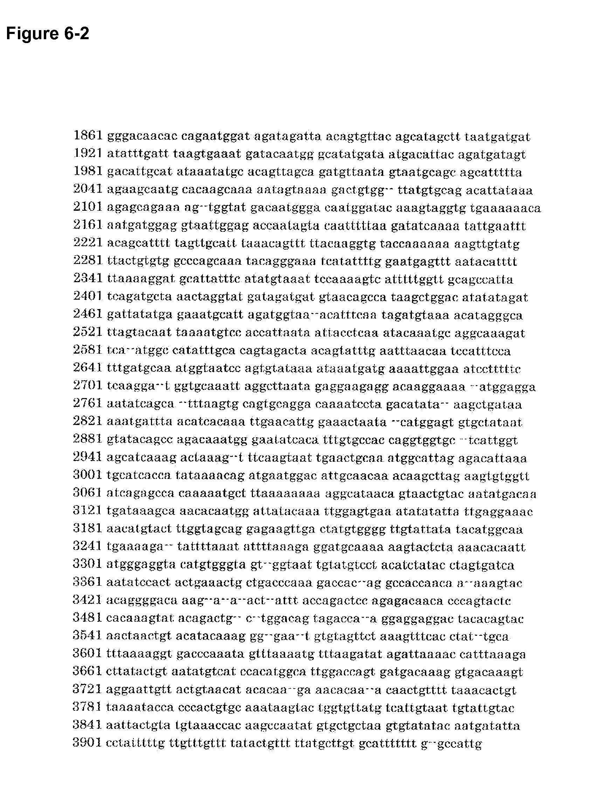

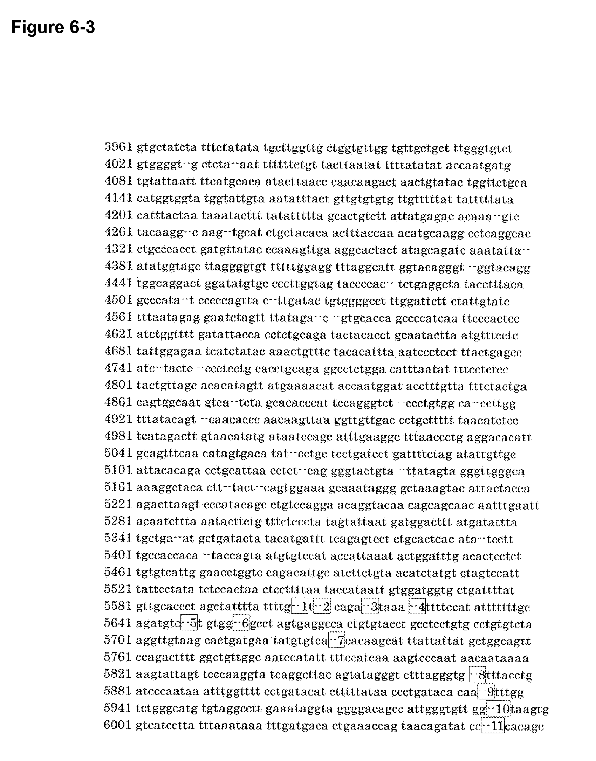

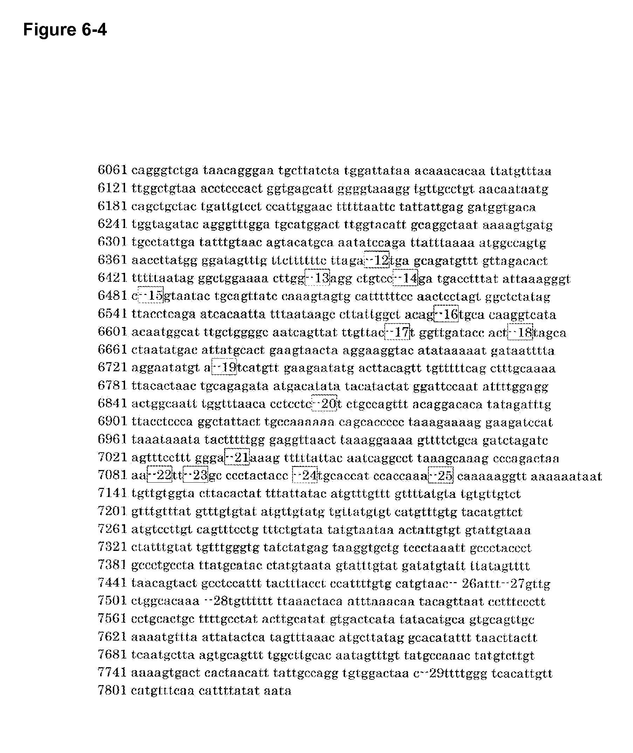

In Figs. 1 to 3, the primer sets used for PCR follwoing the bisulfite treatment are shown on the upper part of each Figure as, e.g. "6F/6R". "Position of CpGs" in "L1" or "LCR" region corresponds to CpGs shown with numbers in Figs. 4 to 6. For example, when the primer set "6F/6R" is used as shown in Fig. 1, the region comprising the 11$^{th}$ to 15$^{th}$ CpGs from 5'-terminal of L1 region of HPV-16 can be amplified and methylation of these CpGs can be measured.

In addition, Figs. 1 to 3 show the results of the sequencing of several *E. coli* clones obtained by TA cloning of the products amplified by PCR with the primer sets. For example, for the subject No. 1 shown in Fig. 1, methylation was measured for seven *E. coli* clones containing the products amplified by PCR with the primer set "6F/6R". Among CpGs in L1 region of HPV-16 measured for methylation, those which measured as "methylated" and "not methylated" are shown with • and o, respectively.

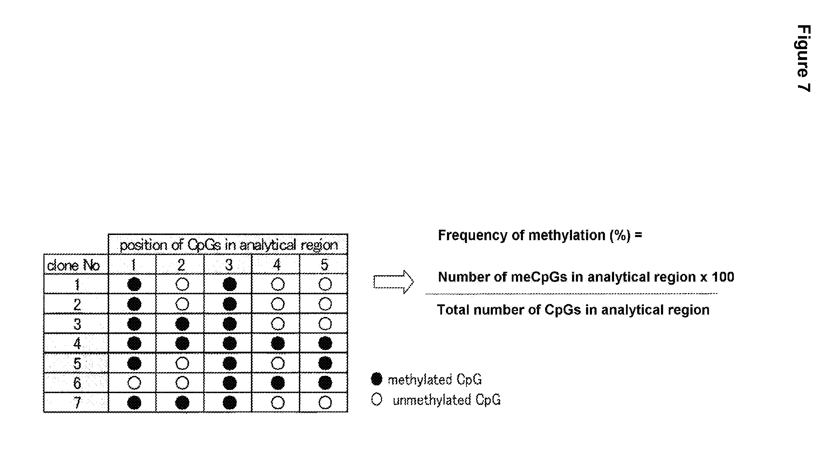

The frequency of methylation was calculated as shown in Fig. 7. Namely, the number of methylated CpG(s) among all CpGs in the analytical region was obtained from the results of all measured clones.

3-1. Results for operation samples and discussion

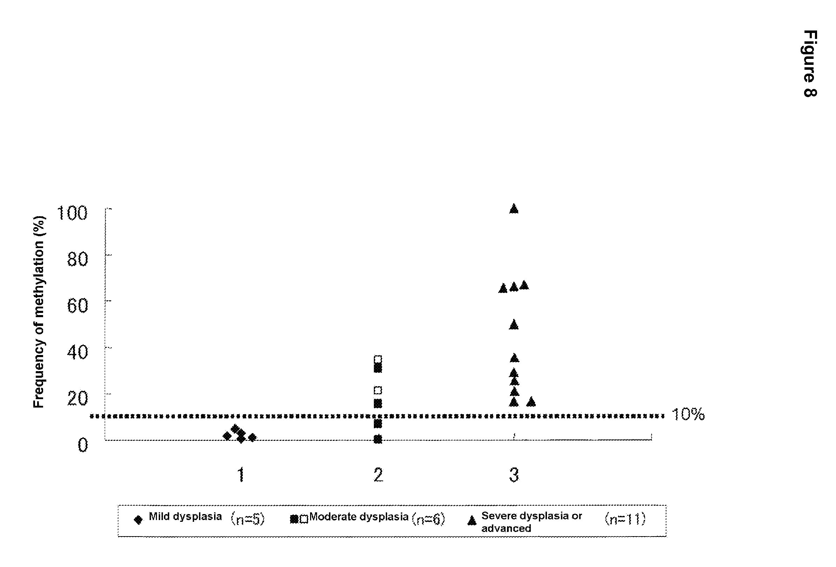

The frequencies of methylation in L1 regions of HPV-16, HPV-58 and HPV-52 obtained as above for the operation samples from total 22 subjects are shown in Fig. 8 as a scatter diagram in relation to the severity of lesion diagnosed by histological diagnosis.

Among 22 subjects diagnosed by histological diagnosis, 5 subjects were diagnosed as mild dysplasia (in the figure, shown as "1" and ), 6 were diagnosed as moderate dysplasia (in the figure, shown as "2" and ■ and □) and 11 were diagnosed as severe dysplasia or lesion in more advanced stages (in the figure, shown as "3" and ▲). Among samples from the 6 subjects diagnosed as moderate dysplasia, the samples shown as □ were suspected as "severe dysplasia" by histological diagnosis.

The results in Fig. 8 shows that when the threshold is set to be the frequency of methylation of 10%, the samples from the subjects having severe dysplasia or lesion in more advanced stages can be clearly distinguished from those from other subjects. There were four samples which were diagnosed as moderate dysplasia by histological diagnosis and had the frequency of methylation of higher than 10%. Two samples among these were the samples suspected as "severe dysplasia" by histological diagnosis.

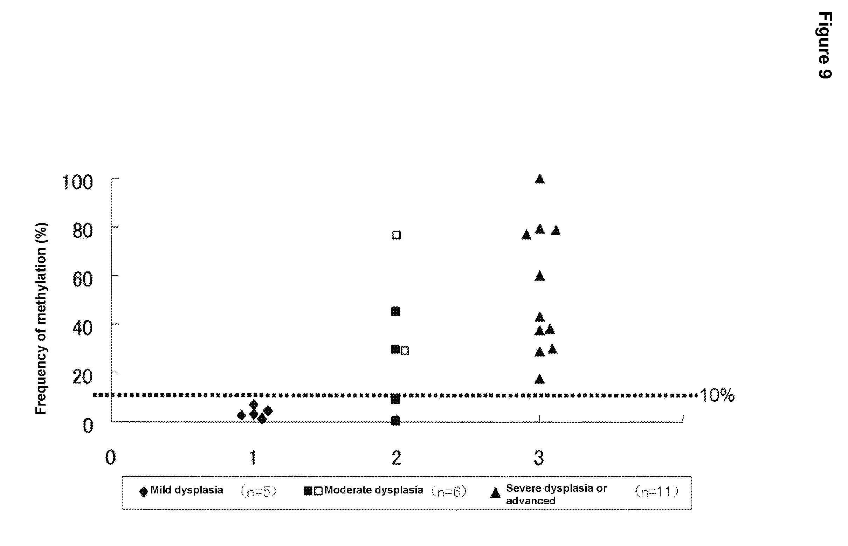

Among CpGs in L1 region, CpGs which exist within 80% from 5'-terminal were specifically analyzed and, similar to Fig. 8, a scatter diagram was obtained in Fig. 9 of the methylation frequency in relation to the severity of lesion diagnosed by histological diagnosis. CpGs which exist within 80% from 5'-terminal correspond to the 1$^{st}$ to 15$^{th}$ CpGs from 5'-terminal for HPV-16 (15/19 = 0.789). They correspond to the 1$^{st}$ to 17$^{th}$ CpGs (17/22 = 0.772) and the 1$^{st}$ to 19$^{th}$ CpGs (19/25 = 0.76) for HPV-52 and HPV-58, respectively.

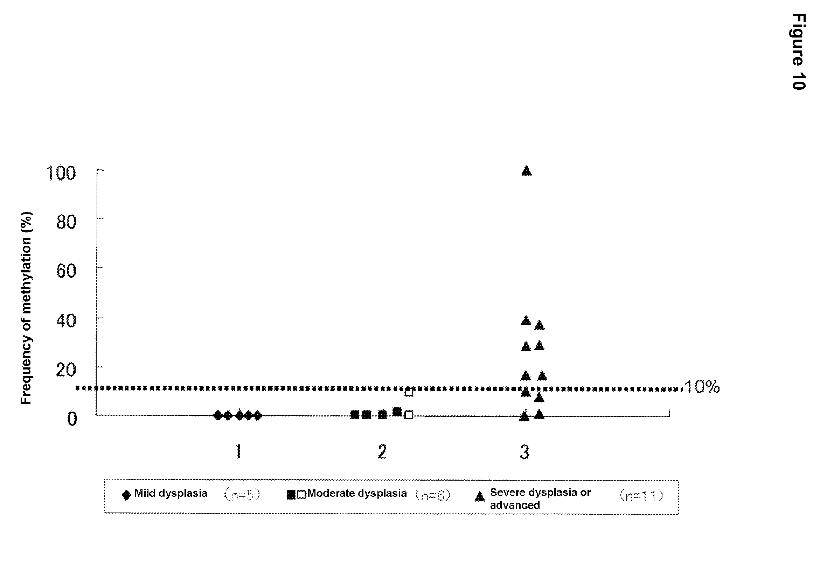

Fig. 10 shows a scatter diagram, as Fig. 8, of the frequency of methylation of the rest of CpGs, i.e. the 16$^{th}$ to 19$^{th}$, the 18$^{th}$ to 22$^{nd}$ and the 20$^{th}$ to 25$^{th}$ CpGs from 5'-terminal for HPV-16, HPV-52 and HPV-58, respectively, in relation to the severity of lesion diagnosed by histological diagnosis.

The results in Figs. 9 and 10 show that the results from the measurement of the frequency of methylation of CpGs existing within 80% from 5'-terminal among all CpGs in L1 region are in more conformity with the results of histological diagnosis than those from the measurement of the frequency of methylation of other CpGs.

3-2. Results for biopsy samples and discussion

The frequencies of methylation in L1 regions of HPV-16, HPV-58 and HPV-52 were measured according to the present method for the biopsy samples obtained from 10 subjects, and whether they have severe dysplasia or lesion in more advanced stages (•) or not (o) was detected with the threshold for the frequency of methylation of 10%.

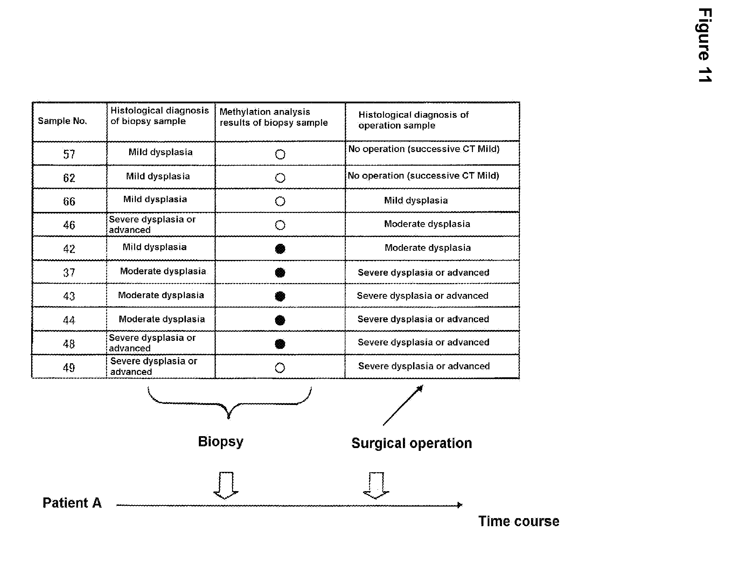

Fig. 11 shows the results of the determination according to the present method together with the results of histological diagnosis of the biopsy samples and results of histological diagnosis carried out on the operation samples taken from the same subjects.

Generally, histological diagnosis on biopsy samples is liable to give false diagnosis results due to the storage condition of the samples, difficulties in the sample preparation of tissue sections and the like. On the other hand, histological diagnosis on operation samples is likely to give more accurate diagnosis results than that on biopsy samples because the operation samples are tissue which contains higher number of cells.

The results in Fig. 11 show that the present method can give results which are rather in conformity with the results by histological diagnosis on the operation samples, even when the biopsy samples are analyzed.
Thus, it is found that the present method allows accurate determination on the presence or absence of abnormal cells originated from severe dysplasia or lesion in more advanced stages in samples, regardless of whether the samples are operation samples or biopsy samples.

Example 2

The frequency of methylation in L1 region was measured on the paraffin blocks of the biopsy samples obtained at the first visit of seven subjects whose progressions have been followed and whose uterine cervical lesions have disappeared within three years from the first visit. Among these 7 subjects, two patients were infected with HPV-16, one with HPV-52 and four with HPV-58.

Similarly, the frequency of methylation in L1 region was measured on the paraffin blocks of the biopsy samples obtained at the first visit of nine subjects whose progressions have been followed and whose uterine cervical lesions have progressed to severe dysplasia or lesion in more advanced stages within three years from the first visit. Among these 9 subjects, two patients were infected with HPV-16, one with HPV-31, three with HPV-52 and three with HPV-58.

The frequency of methylation in L1 region was measured according to Example 1, except that the primer sets used were those shown in Table 3. The PCR conditions for the HPV-31 primer sets were the same as those for HPV-16/HPV-52.

[Table 3]

| Target HPV | SEQID NO: | Name | Amplified Region | Amplified size | Sequence |
|---|---|---|---|---|---|
| HPV-16 | 24 | 5F(16) | L1 Anterior * | 439bp | TTGTTGATGTAGGTGATTTTTATTTATATTTTAGTT |
| | 25 | 5R(16) | | | CCACTAATACCCACACCTAATAACTAACC |
| | 32 | 5F-2 (16) | L1 Anterior * | 222bp | GATGTAGGTGATTTTTATTTATATTTTAGTT |
| | 33 | 5R-2 (16) | | | ATCCAACTACAAATAATCTAAATATTC |
| | 6 | 7F(16) | L1-LCR | 440bp | AGTAGGATTGAAGGTTAAATTAAAATTTAT |
| | 7 | 7R(16) | | | AACACATTTTATACCAAAAAACATACAACC |
| | 8 | 6F(16) | L1 | 424bp | AATAGGGTTGGTATTGTTGGTGAAAAT |
| | 9 | 6R(16) | | | TTCCAATCCTCCAAAATAATAAAATTCATA |
| HPV-31 | 34 | 1F(31) | L1-LCR | 339bp | GATTATGTATTTTGGGAGGTTAATTTAAAA |
| | 35 | 1R(31) | | | CATAACATACATACACACATAACATACTAT |
| | 36 | 3F(31) | L1 | 424bp | GGTGATTGTTTTTTATTAGAATTAAAAA |
| | 37 | 3R(31) | | | AATACCATTATTATATCCCTAAACAC |
| HPV-52 | 26 | 5F(52) | L1 Middle* | 408bp | TTTAGGTGATTTTGTGTTAGGT |
| | 27 | 5R(52) | | | TCCTCTAAAATAATAACATCCATCT |
| | 28 | 4F(52) | L1 Middle* | 385bp | TTGGTTGTATGGATTTTAATATTT |
| | 29 | 4R(52) | | | AACAAACAACTAATTACCCCA |
| | 12 | 2F(52) | L1 Anterior | 367bp | TTTATTTATATTTATTATTGTTGATGGTAT |
| | 13 | 2R(52) | | | TAAAAAACCAAATTTATTAAAATCC |
| | 14 | 3F(52) | L1 Posterior* | 299bp | ATTTTAGAGGATTGGTAATTTGG |
| | 15 | 3R(52) | | | AACCTTTTTCTTCTTTATAAAAATAC |

(continued)

| Target HPV | SEQID NO: | Name | Amplified Region | Amplified size | Sequence |
|---|---|---|---|---|---|
| HPV-58 | 30 | 5F(58) | L1 Middle* | 427bp | TGGTTAGTGAATTTTATGGGG |
| | 31 | 5-2R (58) | | | TTACAAAACTAAAAAACAAACTATAAATCA |
| | 18 | 2F(58) | L1 Anterior | 415bp | ATGGTGTTGATTTTATGTTGTATT |
| | 19 | 2R(58) | | | AACTATCCCCTACCTATTTCAAAAC |
| | 20 | 3F(58) | L1 Posterior* | 344bp | TTAATATTTTGGAGGATTGGTAAT |
| | 21 | 3R(58) | | | ATATAATAAAATAATATAAATACCACAACA |
| | 22 | 4F(58) | L1-LCR | 489bp | AATTAGGTTTTAAAGTAAAGTTTAGATTA |
| | 23 | 4R(58) | | | TTATTTAAATTATAATTTAAAAAAAACAC |
| "L1 Anterior" means a region proximal to 5'-terminal of L1 region, "L1 Posterior" mans a region proximal to 3'-terminal of L1 region and "L1 Middle" means a central region of L1 region. | | | | | |

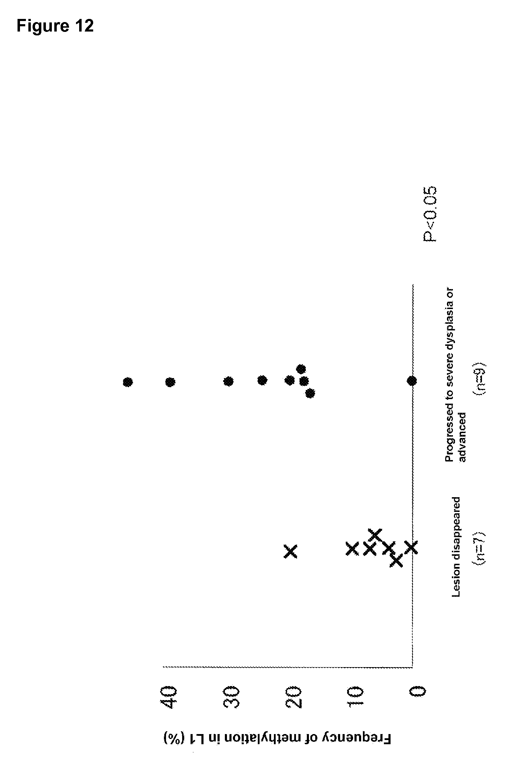

Fig. 12 shows a scatter diagram of the frequency of methylation (%) in L1 region at the first visit of the patients with different courses. According to Fig. 12, it is found that the frequencies of methylation at the first visit of the subjects whose lesion have progressed after the first visit to severe dysplasia or lesion in more advanced stages are higher than those of the subjects whose lesion have disppeared after the first visit.

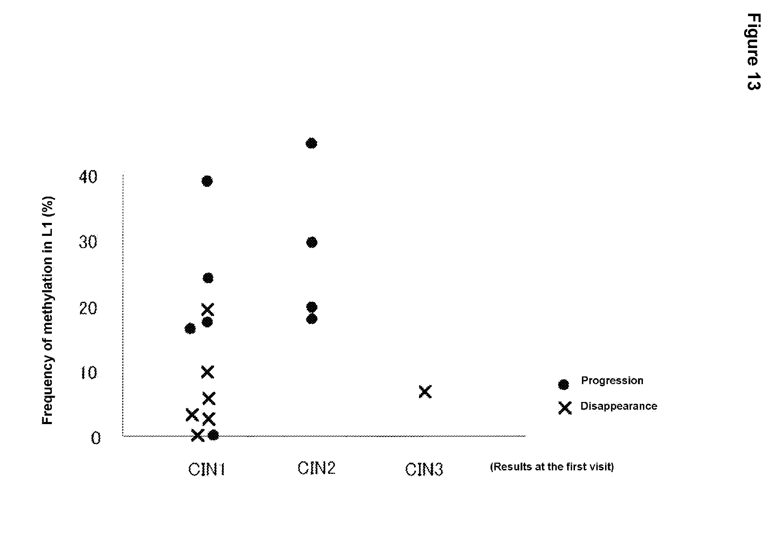

Fig. 13 shows a scatter diagram of the frequency of methylation in L1 region at the first visit of patients in relation to the severity of lesion diagnosed by histological diagnosis. In Fig. 13, "x" shows the subjects whose lesion have disappeared after the first visit and "•" shows the subjects whose lesion have progressed to severe dysplasia or lesion in more advanced stages after the first visit. According to Fig. 13, it is found that when the frequency of methylation in L1 region is high, the lesion may progress with high possibility to severe dysplasia or lesion in more advanced stages even when it was diagnosed as mild dysplasia by histological diagnosis at the first visit. On the contrary, when the frequency of methylation in L1 region is low, the lesion may disappear after the first visit even when it was diagnosed as severe dysplasia or lesion in more advanced stages by histological diagnosis at the first visit.

These results indicate that the measurement of the frequency of methylation in L1 region of HPV genomic DNA in samples allows the prediction on whether or not uterine cervical tissue of subjects progresses to severe dysplasia or lesion in more advanced stages.

The present application relates to Japanese Patent Application No. 2008-273257 filed on October 23, 2008, whose claims, specification, drawings and abstract are incorporated herein by reference.

**Claims**

1. A method of determining the presence or absence of abnormal cells originated from severe dysplasia or lesion in more advanced stages of uterine cervix in a sample obtained from uterine cervix of a subject, comprising the steps of:

   measuring a frequency of methylation of 5'-(CG)-3' (CpG) in L1 region of human papilloma virus genomic DNA in the sample; and
   determining whether or not the abnormal cells are contained in the sample based on the measured frequency of methylation.

2. The method according to claim 1, wherein, in the step of determining, the measured frequency of methylation is compared to a predetermined threshold and the sample is determined to contain the abnormal cells when the frequency of methylation is higher than the threshold.

3. A method of predicting whether or not uterine cervix tissue of a subject progresses to severe dysplasia or lesion in more advanced stages, comprising the steps of:

   measuring a frequency of methylation of 5'-(CG)-3' (CpG) in L1 region of human papilloma virus genomic DNA in a sample obtained from uterine cervix of the subject; and
   predicting whether or not the tissue progresses to severe dysplasia or lesion in more advanced stages based on the measured frequency of methylation.

4. The method according to claim 3, wherein, in the step of predicting, the measured frequency of methylation is compared to a predetermined threshold and the tissue is predicted to progress to severe dysplasia or lesion in more advanced stages when the frequency of methylation is higher than the threshold.

5. The method according to any one of claims 1 to 4, wherein the frequency of methylation is obtained by dividing the number of methylated CpG(s) present in said L1 region which is subjected to the measurement of the frequency of methylation by the number of all CpGs present in said L1 region.

**6.** The method according to any one of claims 1 to 5, wherein said L1 region which is subjected to the measurement of the frequency of methylation is a region which comprises at least one CpG existing within 80% from 5'-terminal among all CpGs in the L1 region.

**7.** The method according to any one of claims 1 to 6, wherein human papilloma virus is at least one selected from HPV-16, HPV-18, HPV31, HPV33, HPV35, HPV-52 and HPV-58.

**8.** The method according to claim 7, wherein human papilloma virus is HPV-16 and wherein said L1 region which is subjected to the measurement of the frequency of methylation is a region which comprises at least one CpG among the $1^{st}$ to $15^{th}$ CpGs from 5'-terminal of L1 region of HPV-16 and does not comprise the $16^{th}$ to $19^{th}$ CpGs.

**9.** The method according to claim 7, wherein human papilloma virus is HPV-31 and wherein said L1 region which is subjected to the measurement of the frequency of methylation is a region which comprises at least one CpG among the $1^{st}$ to $17^{th}$ CpGs from 5'-terminal of L1 region of HPV-31 and does not comprise the $18^{th}$ to $22^{nd}$ CpGs.

**10.** The method according to claim 7, wherein human papilloma virus is HPV-52 and wherein said L1 region which is subjected to the measurement of the frequency of methylation is a region which comprises at least one CpG among the $1^{st}$ to $17^{th}$ CpGs from 5'-terminal of L1 region of HPV-52 and does not comprise the $18^{th}$ to $22^{nd}$ CpGs.

**11.** The method according to claim 7, wherein human papilloma virus is HPV-58 and wherein said L1 region which is subjected to the measurement of the frequency of methylation is a region which comprises at least one CpG among the $1^{st}$ to $19^{th}$ CpGs from 5'-terminal of L1 region of HPV-58 and does not comprise the $20^{th}$ to $25^{th}$ CpGs.

**12.** The method according to any one of claims 1 to 11, wherein severe dysplasia or lesion in more advanced stages includes severe dysplasia, intraepithelial cancer, microinvasive squamous cancer and invasive squamous cancer.

**13.** A primer set for determining the presence or absence of abnormal cells originated from severe dysplasia or lesion in more advanced stages of uterine cervix, or for predicting the progress to severe dysplasia or lesion in more advanced stages, which is used in a nucleic acid amplification method for amplification of a region comprising at least one CpG existing within 80% from 5'-terminal among all CpGs in L1 region of human papilloma virus genomic DNA, said region having been treated with bisulfite.

**14.** The primer set according to claim 13, wherein HPV is at least one selected from HPV-16, HPV-18, HPV-31, HPV33, HPV35, HPV-52 and HPV-58.

**15.** The primer set according to claim 14, wherein HPV is HPV-16 and wherein the region amplified in the amplification method is a region which comprises at least one CpG among the $1^{st}$ to $15^{th}$ CpGs from 5'-terminal of L1 region of HPV-16 and does not comprise the $16^{th}$ to $19^{th}$ CpGs, said region having been treated with bisulfite.

**16.** The primer set according to claim 14, wherein HPV is HPV-18 and wherein the region amplified in the amplification method is a region which comprises at least one CpG among the $1^{st}$ to $25^{th}$ CpGs from 5'-terminal of L1 region of HPV-18 and does not comprise the $26^{th}$ to $32^{nd}$ CpGs, said region having been treated with bisulfite.

**17.** The primer set according to claim 14, wherein HPV is HPV-31 and wherein the region amplified in the amplification method is a region which comprises at least one CpG among the $1^{st}$ to $17^{th}$ CpGs from 5'-terminal of L1 region of HPV-31 and does not comprise the $18^{th}$ to $22^{nd}$ CpGs, said region having been treated with bisulfite.

**18.** The primer set according to claim 14, wherein HPV is HPV-33 and wherein the region amplified in the amplification method is a region which comprises at least one CpG among the $1^{st}$ to $16^{th}$ CpGs from 5'-terminal of L1 region of HPV-33 and does not comprise the $17^{th}$ to $21^{st}$ CpGs, said region having been treated with bisulfite.

**19.** The primer set according to claim 14, wherein HPV is HPV-35 and wherein the region amplified in the amplification method is a region which comprises at least one CpG among the $1^{st}$ to $13^{th}$ CpGs from 5'-terminal of L1 region of HPV-35 and does not comprise the $14^{th}$ to $17^{th}$ CpGs, said region having been treated with bisulfite.

**20.** The primer set according to claim 14, wherein HPV is HPV-52 and wherein the region amplified in the amplification method is a region which comprises at least one CpG among the $1^{st}$ to $17^{th}$ CpGs from 5'-terminal of L1 region of HPV-52 and does not comprise the $18^{th}$ to $22^{nd}$ CpGs, said region having been treated with bisulfite.

**21.** The primer set according to claim 14, wherein HPV is HPV-58 and wherein the region amplified in the amplification method is a region which comprises at least one CpG among the $1^{st}$ to $19^{th}$ CpGs from 5'-terminal of L1 region of HPV-58 and does not comprise the $20^{th}$ to $25^{th}$ CpGs, said region having been treated with bisulfite.

**Figure 1-1**

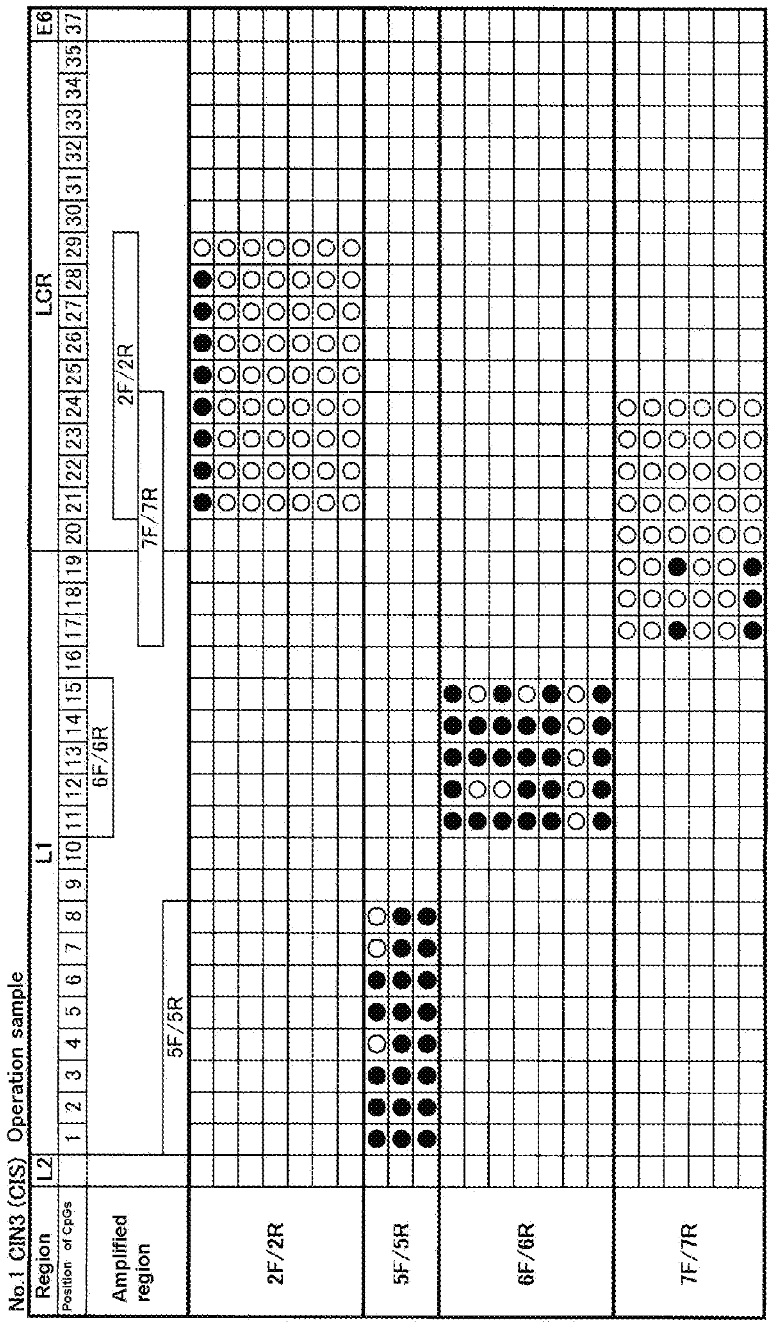
No.1 CIN3 (CIS) Operation sample
Region
L2
L1
LCR
E6
Position of CpGs
1 2 3 4 5 6 7 8 9 10 11 12 13 14 15 16 17 18 19 20 21 22 23 24 25 26 27 28 29 30 31 32 33 34 35 37
Amplified region
5F/5R
6F/6R
7F/7R
2F/2R
2F/2R
5F/5R
6F/6R
7F/7R

**Figure 1-2**

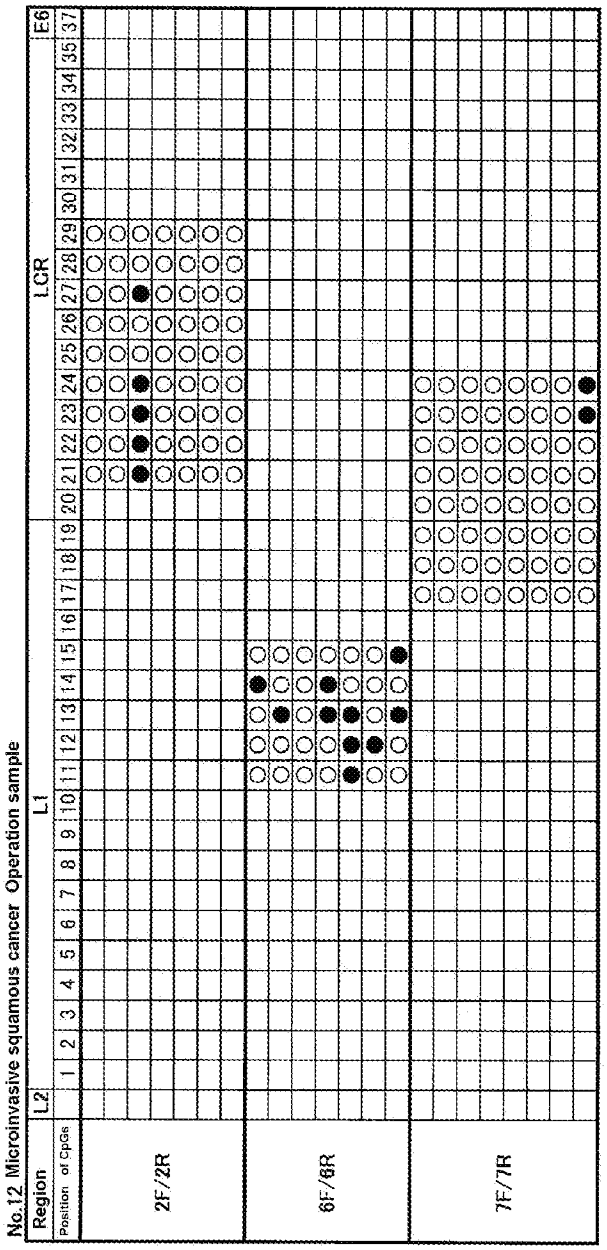
No.12 Microinvasive squamous cancer Operation sample
Region
L2
L1
LCR
E6
Position of CpGs
2F/2R
6F/6R
7F/7R

Figure 1-3

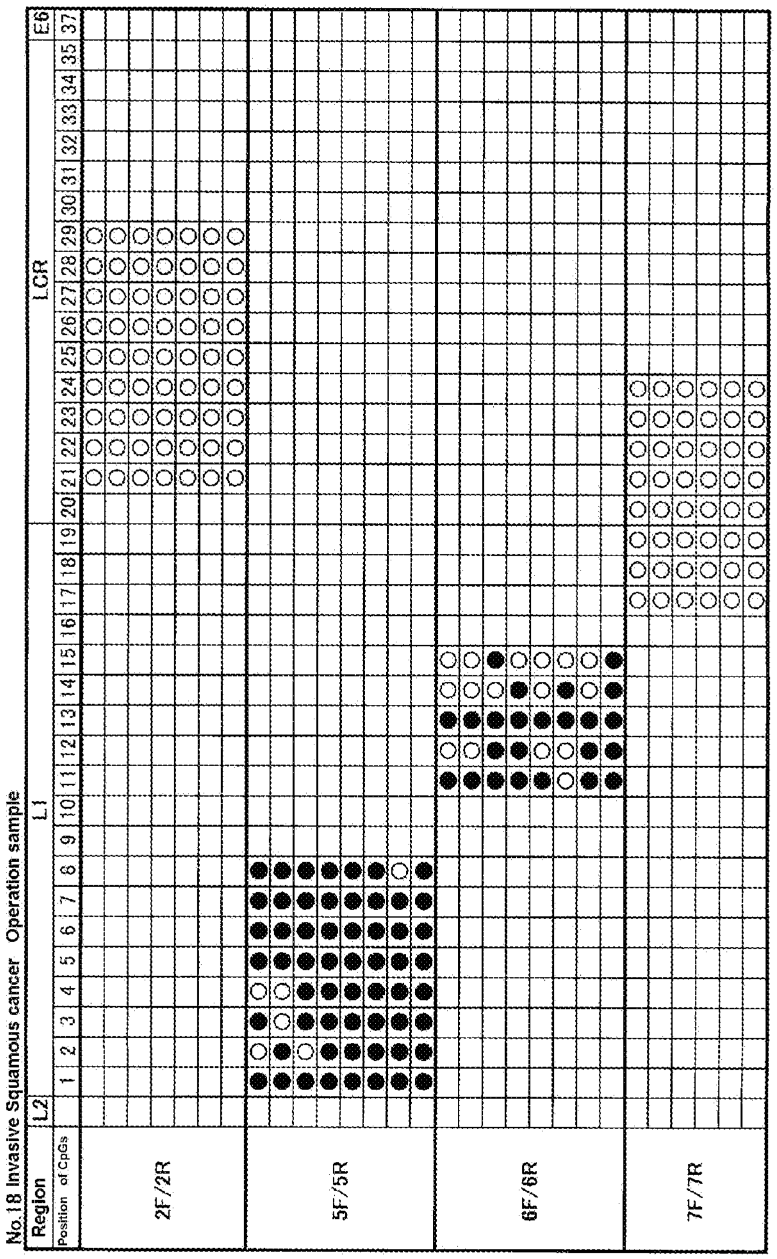
No.18 Invasive Squamous cancer Operation sample
Region
L2
L1
LCR
E6
Position of CpGs
1 2 3 4 5 6 7 8 9 10 11 12 13 14 15 16 17 18 19 20 21 22 23 24 25 26 27 28 29 30 31 32 33 34 35 37
2F/2R
5F/5R
6F/6R
7F/7R

## Figure 1-4

No.27 CIN2 (partially containing a part suspected to be CIN 3) Operation sample

| Region | L2 | L1 | | | | | | | | | | | | | | | | | | LCR | | | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| 6F/6R | | | | | | | | | | | | ● | ● | ● | ● | ○ | | | | | | | | | | | | | | | | | | | | | |
| | | | | | | | | | | | | ● | ● | ● | ● | ○ | | | | | | | | | | | | | | | | | | | | | |
| | | | | | | | | | | | | ● | ● | ● | ● | ○ | | | | | | | | | | | | | | | | | | | | | |
| | | | | | | | | | | | | ● | ● | ● | ○ | ○ | | | | | | | | | | | | | | | | | | | | | |
| | | | | | | | | | | | | ● | ● | ● | ● | ○ | | | | | | | | | | | | | | | | | | | | | |
| 7F/7R | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |

**Figure 1-5**

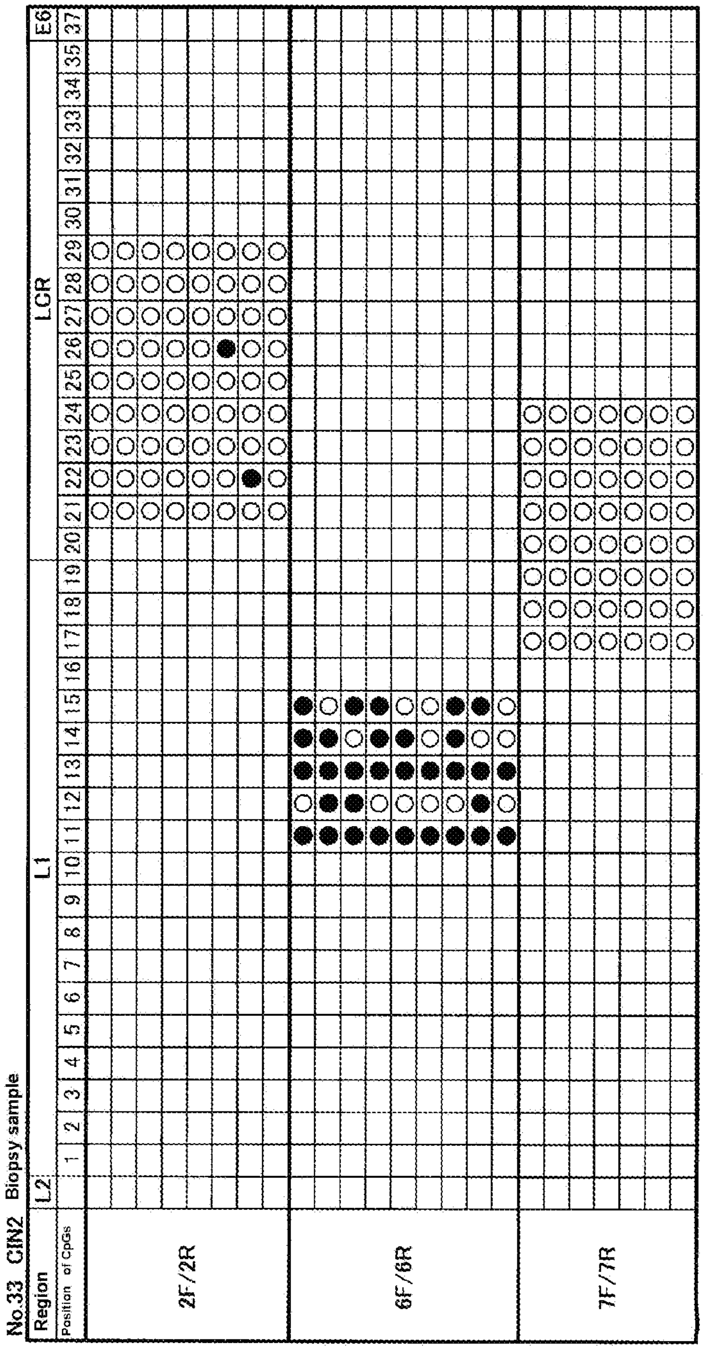
No.33 CIN2 Biopsy sample
Region
L2
L1
LCR
E6
Position of CpGs
1 2 3 4 5 6 7 8 9 10 11 12 13 14 15 16 17 18 19 20 21 22 23 24 25 26 27 28 29 30 31 32 33 34 35 37
2F/2R
6F/6R
7F/7R

## Figure 1-6

No.48 CIN3 Biopsy sample

| Region | L2 | L1 | | | | | | | | | | | | | | | | | | | LCR | | | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| 6F/6R | | | | | | | | | | | ● | ● | ● | ● | ● | | | | | | | | | | | | | | | | | | | | | | |
| | | | | | | | | | | | ○ | ● | ● | ○ | ○ | | | | | | | | | | | | | | | | | | | | | | |
| | | | | | | | | | | | ○ | ● | ○ | ● | ● | | | | | | | | | | | | | | | | | | | | | | |
| 7F/7R | | | | | | | | | | | | | | | | | ○ | ○ | ○ | ○ | ○ | ○ | ○ | ○ | | | | | | | | | | | | |
| | | | | | | | | | | | | | | | | | ● | ● | ○ | ○ | ○ | ○ | ○ | ○ | | | | | | | | | | | | |
| | | | | | | | | | | | | | | | | | ○ | ○ | ○ | ○ | ○ | ○ | ○ | ○ | | | | | | | | | | | | |
| | | | | | | | | | | | | | | | | | ○ | ○ | ○ | ○ | ○ | ○ | ○ | ○ | | | | | | | | | | | | |
| | | | | | | | | | | | | | | | | | ○ | ○ | ○ | ○ | ○ | ○ | ○ | ○ | | | | | | | | | | | | |
| | | | | | | | | | | | | | | | | | ○ | ○ | ○ | ○ | ○ | ○ | ○ | ○ | | | | | | | | | | | | |

Figure 1-7

No.4 CIN1 Operation sample

| Region | L2 | L1 | | | | | | | | | | | | | | | | | | | LCR | | | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| 5F/5R | | ○ | ○ | ○ | ○ | ○ | ○ | ○ | ● | | | | | | | | | | | | | | | | | | | | | | | | | | | | |
| | | ○ | ○ | ○ | ○ | ○ | ○ | ● | ● | | | | | | | | | | | | | | | | | | | | | | | | | | | | |
| | | ○ | ○ | ○ | ○ | ○ | ○ | ○ | ○ | | | | | | | | | | | | | | | | | | | | | | | | | | | | |
| 6F/6R | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| 7F/7R | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |

## Figure 1-8

No.24 CIN2 Operation sample

| Region | L2 | L1 |  |  |  |  |  |  |  |  |  |  |  |  |  |  |  |  |  |  | LCR |  |  |  |  |  |  |  |  |  |  |  |  |  |  |  | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs |  | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
|  |  |  |  |  |  |  |  |  |  |  |  |  |  |  |  |  |  |  |  |  |  | ○ | ○ | ○ | ○ | ○ | ○ | ○ | ○ | ○ |  |  |  |  |  |  |  |
| 5F/5R |  | ○ | ○ | ○ | ○ | ○ | ○ | ○ | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ● | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ○ | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ○ | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ○ | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ● | ○ |  |  |  |  |  |  |  |  |  |  |  |  |  |  |  |  |  |  |  |  |  |  |  |  |  |  |  |  |
|  |  | ○ | ○ | ○ | ○ | ○ | ○ | ● | ○ |  |  |  |  |  |  |  |  |  |  |  |  |  |  |  |  |  |  |  |  |  |  |  |  |  |  |  |  |
| 6F/6R |  |  |  |  |  |  |  |  |  |  |  | ○ | ○ | ○ | ○ | ○ |  |  |  |  |  |  |  |  |  |  |  |  |  |  |  |  |  |  |  |  |  |
|  |  |  |  |  |  |  |  |  |  |  |  | ○ | ○ | ○ | ○ | ○ |  |  |  |  |  |  |  |  |  |  |  |  |  |  |  |  |  |  |  |  |  |
|  |  |  |  |  |  |  |  |  |  |  |  | ○ | ○ | ○ | ○ | ○ |  |  |  |  |  |  |  |  |  |  |  |  |  |  |  |  |  |  |  |  |  |
|  |  |  |  |  |  |  |  |  |  |  |  | ○ | ○ | ○ | ○ | ○ |  |  |  |  |  |  |  |  |  |  |  |  |  |  |  |  |  |  |  |  |  |
|  |  |  |  |  |  |  |  |  |  |  |  | ○ | ○ | ○ | ○ | ○ |  |  |  |  |  |  |  |  |  |  |  |  |  |  |  |  |  |  |  |  |  |
| 7F/7R |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |
|  |  |  |  |  |  |  |  |  |  |  |  |  |  |  |  |  |  | ○ | ○ | ○ | ○ | ○ | ○ | ○ | ○ |  |  |  |  |  |  |  |  |  |  |  |  |

## Figure 1-9

No.29 CIN2 Operation sample

| Region | L2 | L1 | | | | | | | | | | | | | | | | | | | LCR | | | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ● | ● | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| 6F/6R | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| | | | | | | | | | | | | ○ | ○ | ○ | ○ | ○ | | | | | | | | | | | | | | | | | | | | | |
| 7F/7R | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |

## Figure 1-10

No.31 Normal Operation sample

| Region | L2 | L1 | | | | | | | | | | | | | | | | | | | LCR | | | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 37 |
| 2F/2R | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| | | | | | | | | | | | | | | | | | | | | | | ○ | ○ | ○ | ○ | ○ | ○ | ○ | ○ | ○ | | | | | | | |
| 7F/7R | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |
| | | | | | | | | | | | | | | | | | | ○ | ○ | ○ | ○ | ○ | ○ | ○ | ○ | | | | | | | | | | | | |

## Figure 2-1

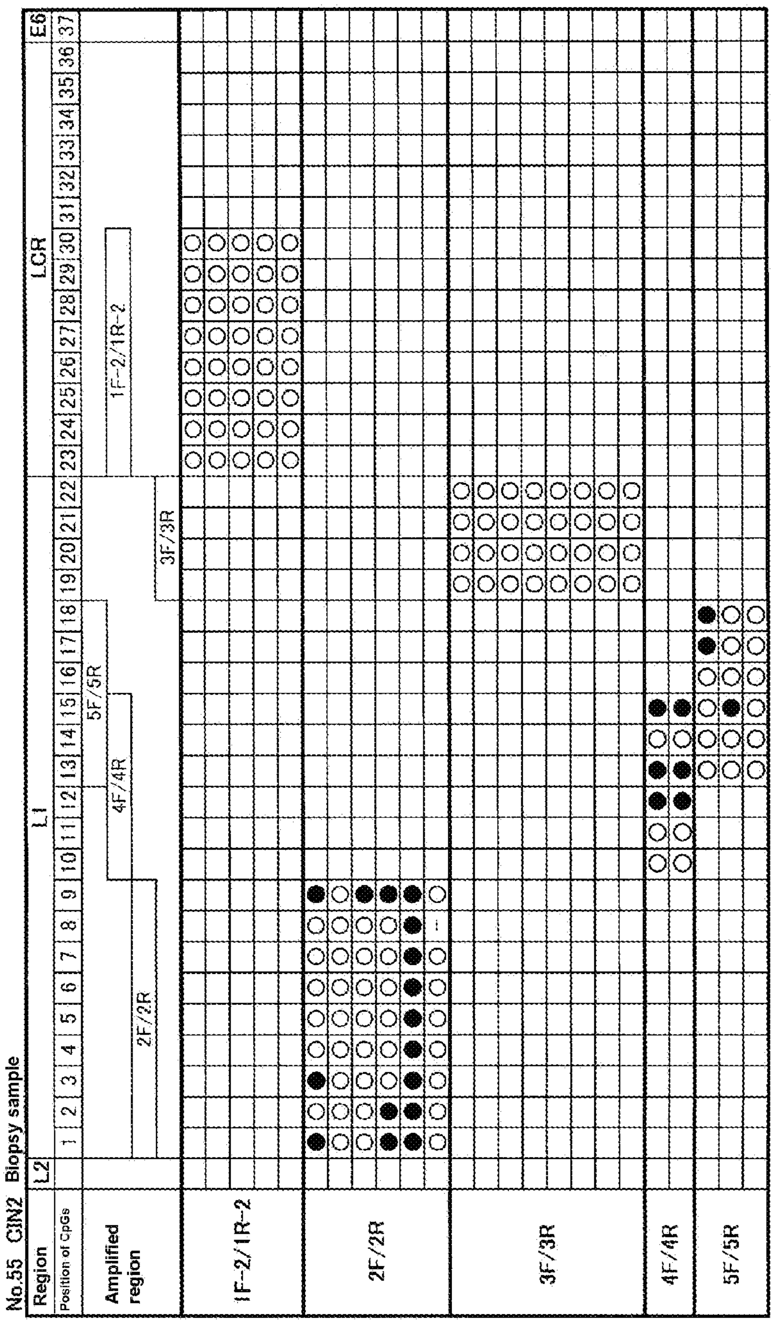
No.55 CIN2 Biopsy sample
Region
L2
L1
LCR
E6
Position of CpGs
1 2 3 4 5 6 7 8 9 10 11 12 13 14 15 16 17 18 19 20 21 22 23 24 25 26 27 28 29 30 31 32 33 34 35 36 37
Amplified region
2F/2R
4F/4R
5F/5R
3F/3R
1F-2/1R-2
1F-2/1R-2
2F/2R
3F/3R
4F/4R
5F/5R

## Figure 2-2

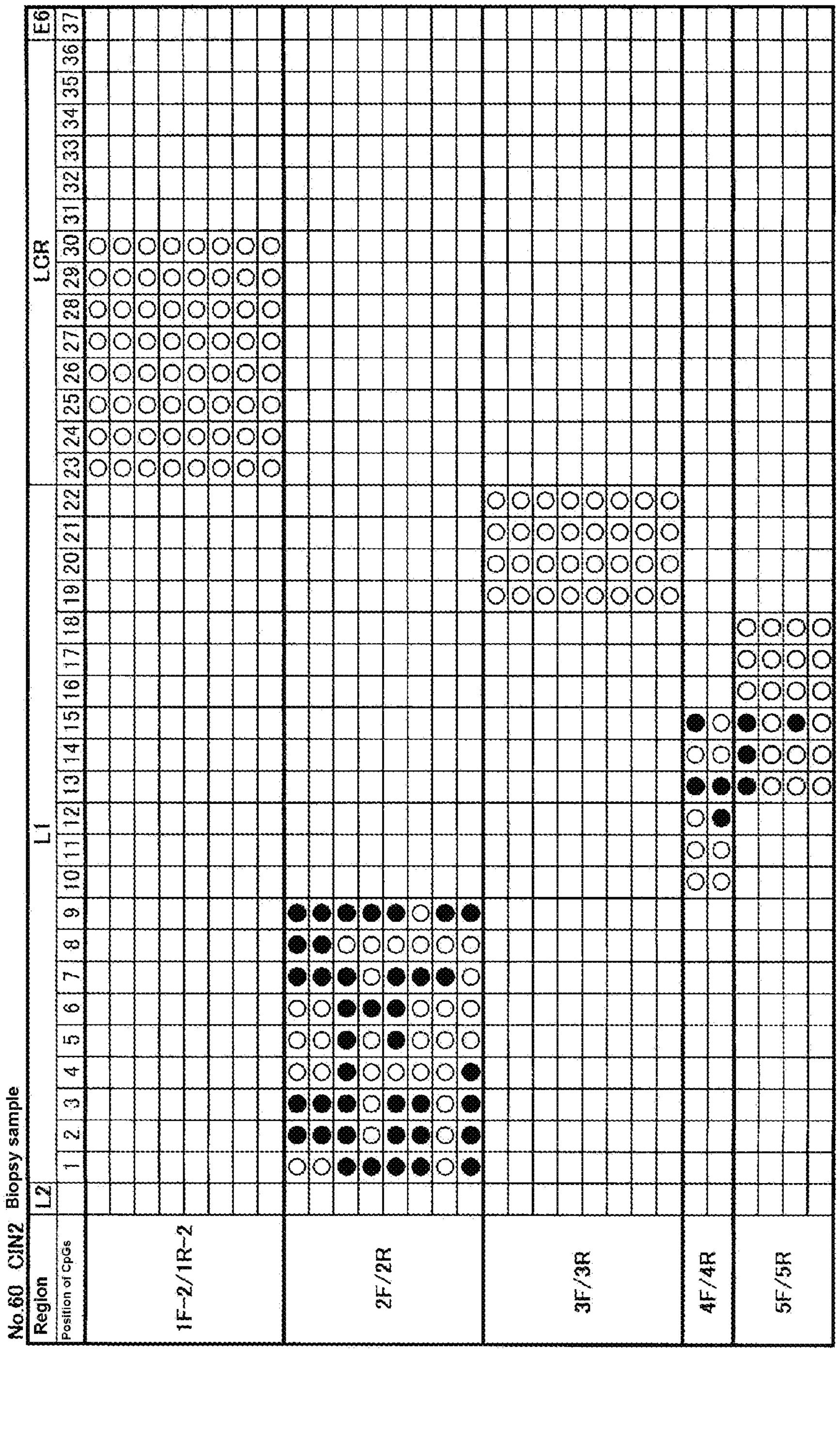
No.60 CIN2 Biopsy sample
Region
Position of CpGs
L2
L1
LCR
E6
1 2 3 4 5 6 7 8 9 10 11 12 13 14 15 16 17 18 19 20 21 22 23 24 25 26 27 28 29 30 31 32 33 34 35 36 37
1F-2/1R-2
2F/2R
3F/3R
4F/4R
5F/5R

## Figure 2-3

No.15 CIN1 Operation sample

| Region | L2 | L1 | | | | | | | | | | | | | | | | | | | | | | LCR | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | | | | | | | |
| 1F-2/1R-2 | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| | | | | | | | | | | | | | | | | | | | | | | | | ○ | ○ | ○ | ○ | ○ | ○ | ○ | ○ | | | | | | | |
| 2F/2R | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | | | | |
| 3F/3R | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |
| | | | | | | | | | | | | | | | | | | | | ○ | ○ | ○ | ○ | | | | | | | | | | | | | | | |

## Figure 2-4

No.15 CIN1 Operation sample

| Region | L2 | L1 | | | | | | | | | | | | | | | | | | | | | | LCR | | | | | | | | | | | | | | E6 |
|---|---|---|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | | | | | | | |
| 4F/4R | | | | | | | | | | | ○ | ○ | ○ | ○ | ○ | ○ | | | | | | | | | | | | | | | | | | | | | | |
| | | | | | | | | | | | ○ | ○ | ○ | ○ | ○ | ○ | | | | | | | | | | | | | | | | | | | | | | |
| | | | | | | | | | | | ○ | ○ | ○ | ○ | ○ | ○ | | | | | | | | | | | | | | | | | | | | | | |
| | | | | | | | | | | | ○ | ○ | ○ | ○ | ○ | ○ | | | | | | | | | | | | | | | | | | | | | | |
| | | | | | | | | | | | ○ | ○ | ○ | ○ | ○ | ○ | | | | | | | | | | | | | | | | | | | | | | |
| 5F/5R | | | | | | | | | | | | | | ○ | ○ | ○ | ○ | ○ | ○ | | | | | | | | | | | | | | | | | | | |
| | | | | | | | | | | | | | | ○ | ● | ○ | ○ | ○ | ○ | | | | | | | | | | | | | | | | | | | |
| | | | | | | | | | | | | | | ○ | ○ | ○ | ○ | ○ | ○ | | | | | | | | | | | | | | | | | | | |
| | | | | | | | | | | | | | | ○ | ○ | ○ | ○ | ○ | ○ | | | | | | | | | | | | | | | | | | | |
| | | | | | | | | | | | | | | ○ | ○ | ○ | ● | ○ | ○ | | | | | | | | | | | | | | | | | | | |
| | | | | | | | | | | | | | | ○ | ○ | ● | ○ | ○ | ○ | | | | | | | | | | | | | | | | | | | |

## Figure 3-1

No.28 CIN3 Operation sample

| Region | L2 | L1 | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| Amplified region | | 2F/2R | | | | | | | | | | | | | | | | | | | 3F/3R | | | | | | 1F/1R-2 | | | | | | | | |
| 1F/1R-2 | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| 2F/2R | | ○ | ○ | ● | ○ | ● | ● | ○ | ○ | ○ | | | | | | | | | | | | | | | | | | | | | | | | | |
| | | ○ | ○ | ● | ○ | ● | ● | ● | ○ | ○ | | | | | | | | | | | | | | | | | | | | | | | | | |
| | | ○ | ○ | ○ | ○ | ● | ● | ○ | ○ | ○ | | | | | | | | | | | | | | | | | | | | | | | | | |
| | | ○ | ● | ○ | ○ | ○ | ● | ● | ● | ○ | | | | | | | | | | | | | | | | | | | | | | | | | |
| | | ● | ● | ● | ○ | ● | ● | ○ | ○ | ○ | | | | | | | | | | | | | | | | | | | | | | | | | |
| | | ● | ● | ● | ○ | ○ | ● | ○ | ● | ○ | | | | | | | | | | | | | | | | | | | | | | | | | |
| 3F/3R | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |

## Figure 3-2

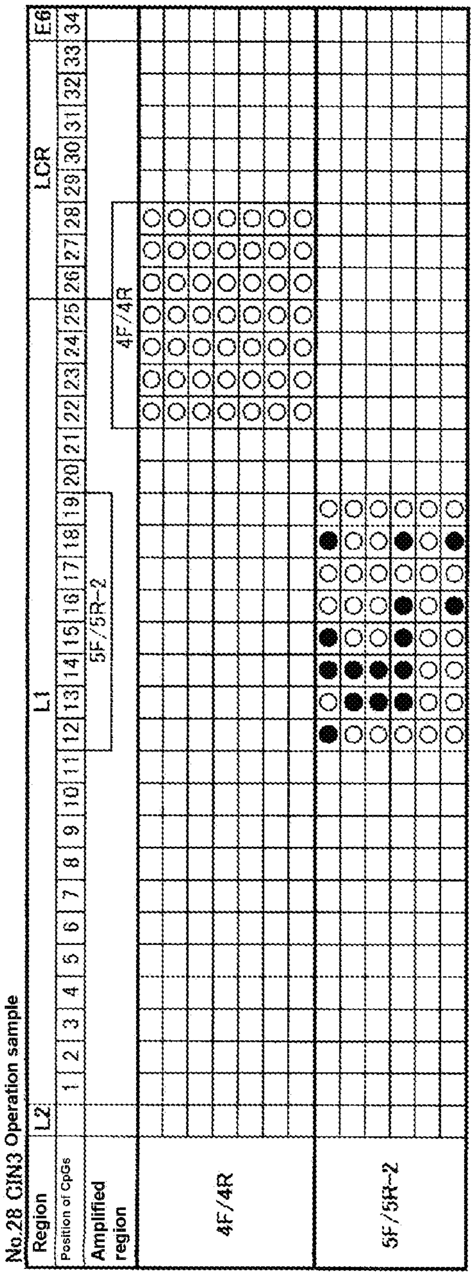
No.28 CIN3 Operation sample
Region
L2
L1
LCR
E6
Position of CpGs
1 2 3 4 5 6 7 8 9 10 11 12 13 14 15 16 17 18 19 20 21 22 23 24 25 26 27 28 29 30 31 32 33 34
Amplified region
5F/5R-2
4F/4R
4F/4R
5F/5R-2

## Figure 3-3

No.43 CIN2 Biopsy sample

| Region | L2 | L1 | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| 1F/1R-2 | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | - | ○ | ○ | ○ | ○ | ○ | |
| 2F/2R | | ● | ○ | ● | ○ | ○ | ● | ● | ○ | ○ | | | | | | | | | | | | | | | | | | | | | | | | | |
| | | ○ | ○ | ● | ○ | ○ | ● | ● | ○ | ○ | | | | | | | | | | | | | | | | | | | | | | | | | |
| | | ● | ○ | ● | ○ | ○ | ● | ● | ○ | ● | | | | | | | | | | | | | | | | | | | | | | | | | |
| | | ○ | ○ | ● | ○ | ● | ● | ● | ○ | ○ | | | | | | | | | | | | | | | | | | | | | | | | | |
| | | ● | ○ | ● | ○ | ● | ● | ● | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| 3F/3R | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| 4F/4R | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| 5F/5R-2 | | | | | | | | | | | | | ○ | ● | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ● | ○ | ○ | ● | ○ | | | | | | | | | | | | | | | |
| | | | | | | | | | | | | | ○ | ○ | ○ | ○ | ○ | ○ | ○ | ● | | | | | | | | | | | | | | | |
| | | | | | | | | | | | | | ○ | ● | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |

## Figure 3-4

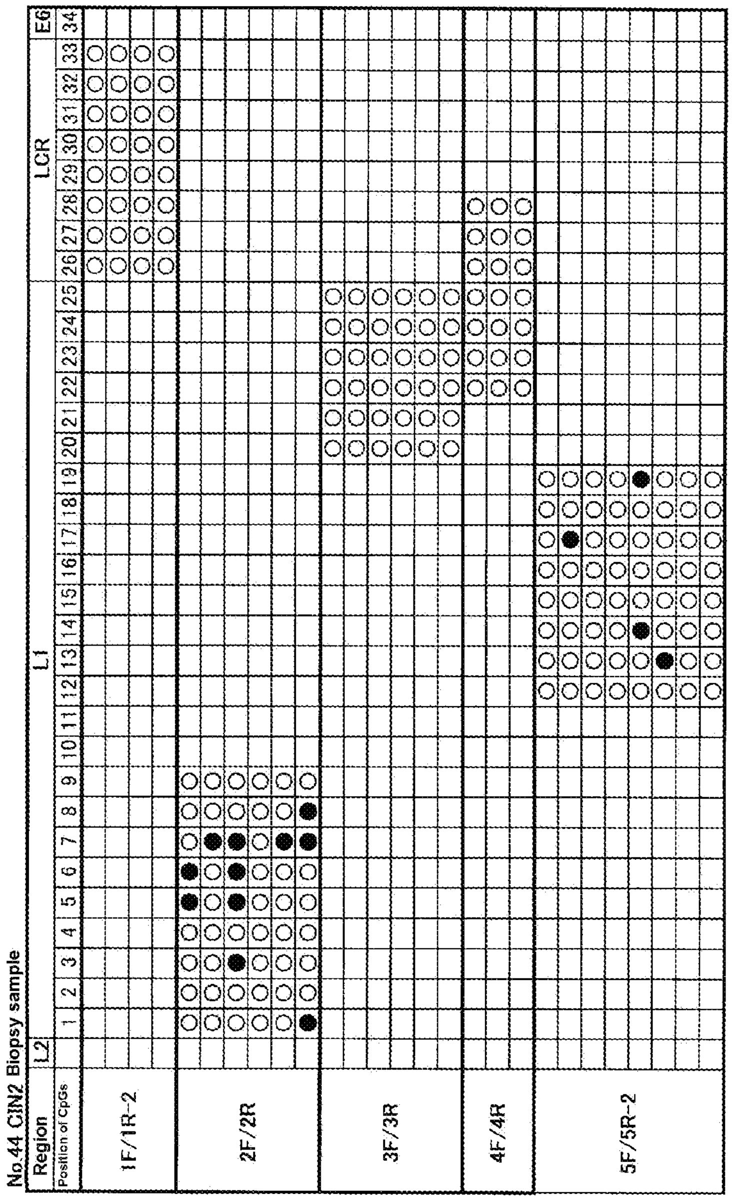
No.44 CIN2 Biopsy sample
Region
Position of CpGs
L2
L1
LCR
E6
1F/1R-2
2F/2R
3F/3R
4F/4R
5F/5R-2

## Figure 3-5

No.15 CIN1 Operation sample

| Region | L2 | L1 | | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| 1F/1R-2 | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| 2F/2R | | ○ | ○ | ● | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ● | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| 3F/3R | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |

## Figure 3-6

No.15 CIN1 Operation sample

| Region | L2 | L1 | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| 4F/4R | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| 5F/5R-2 | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |

## Figure 3-7

No.4 CIN1 Operation sample

| Region | L2 | L1 | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| 1F/1R-2 | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| | | | | | | | | | | | | | | | | | | | | | | | | | | | ○ | ○ | ○ | ○ | ○ | ○ | ○ | ○ | |
| 2F/2R | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| | | ○ | ● | ● | ○ | ○ | ● | ○ | ○ | ○ | | | | | | | | | | | | | | | | | | | | | | | | | |
| | | ○ | ○ | ○ | ○ | ○ | ○ | ○ | ○ | ○ | | | | | | | | | | | | | | | | | | | | | | | | | |
| 3F/3R | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |
| | | | | | | | | | | | | | | | | | | | | | ○ | ○ | ○ | ○ | ○ | ○ | | | | | | | | | |

## Figure 3-8

No.4 CIN1 Operation sample

| Region | L2 | L1 | | | | | | | | | | | | | | | | | | | | | | | | LCR | | | | | | | | E6 |
|---|---|---|---|---|---|---|---|---|---|---|---|---|---|---|---|---|---|---|---|---|---|---|---|---|---|---|---|---|---|---|---|---|---|---|---|
| Position of CpGs | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 |
| 4F/4R | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| | | | | | | | | | | | | | | | | | | | | | | | ○ | ○ | ○ | ○ | ○ | ○ | ○ | | | | | | |
| 5F/5R-2 | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ● | ○ | ○ | | | | | | | | | | | | | | | |
| | | | | | | | | | | | | | ○ | ● | ○ | ○ | ○ | ○ | ○ | ● | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |
| | | | | | | | | | | | | | ○ | ○ | ○ | ○ | ○ | ○ | ○ | ○ | | | | | | | | | | | | | | | |

# Figure 4-1

-- : denotes CG.

[--] : denotes CG in L1 region.

The number appended after "--" corresponds to the position of CpG shown in Figs. 1 to 3.

Total genomic DNA sequence of HPV-16 (NC_001526 ; SEQ ID NO:1 )

1 actacaataa ttcatgtata aaactaaggg --taac--aa at--gttgaa c--aaac--g
61 ttagtataaa agcagacatt ttatgcacca aaagagaact gcaatgtttc aggacccaca
121 ggag--accc agaaagttac cacagttatg cacagagctg caaacaacta tacatgatat
181 aatattagaa tgtgtgtact gcaagcaaca gttactg--a --tgaggtat atgactttgc
241 tttt--ggat ttatgcatag tatatagaga tgggaatcca tatgctgtat gtgataaatg
301 tttaaagttt tattctaaaa ttagtgagta tagacattat tgttatagtt tgtatggaac
361 aacattagaa cagcaataca acaaac--tt gtgtgatttg ttaattaggt gtattaactg
421 tcaaaagcca ctgtgtcctg aagaaaagca aagacatctg gacaaaaagc aaagattcca
481 taatataagg ggt--gtgga c--gt--atg tatgtcttgt tgcagatcat caagaaca--
541 tagagaaacc cagctgtaat catgcatgga gatacaccta cattgcatga atatatgtta
601 gatttgcaac cagagacaac tgatctctac tgttatgagc aattaaatga cagctcagag
661 gaggaggatg aaatagatgg tccagctgga caagcagaac --gacagagc ccattacaat
721 attgtaacct tttgttgcaa gtgtgactct a--ctt--gt tgtg--taca aagcacacac
781 gtagacatt--tactttgga agacctgtta atgggcacac taggaattgt gtgccccatc
841 tgttctcaga aaccataatc taccatggct gatcctgcag gtaccaatgg ggaagagggt
901 a--ggatgta atggatggtt ttatgtagag gctgtagtgg aaaaaaaac aggggatgct
961 atatcagatg a--agaa--a aaatgacagt gatacaggtg aagatttggt agattttata
1021 gtaaatgata atgattattt aacacaggca gaaacagaga cagcacatg--ttgtttact
1081 gcacaggaag caaaacaaca tagagatgca gtacaggttc taaaa--aaa gtatttggta
1141 gtccacttag tgatattagt ggatgtgtag acaataatat tagtcctaga ttaaaagcta
1201 tatgtataga aaaacaaagt agagctgcaa aaaggagatt atttgaaag--aagacag--
1261 ggtatggcaa tactgaagtg gaaactcagc agatgttaca ggtagaaggg --ccatgaga
1321 ctgaaacacc atgtagtcag tatagtggtg gaagtggggg tggttgcagt cagtacagta
1381 gtggaagtgg gggagagggt gttagtgaaa gacacactat atgccaaaca ccacttacaa
1441 atattttaaa tgtactaaaa actagtaatg caaaggcagc aatgttagca aaatttaaag
1501 agttata--g ggtgagtttt tcagaattag taagaccatt taaaagtaat aaatcaa--t
1561 gttg--attg gtgtattgct gcatttggac ttacacccag tatagctgac agtataaaaa
1621 cactattaca acaatattgt ttatatttac acattcaaag tttagcatgt tcatggggaa
1681 tggttgtgtt actattagta agatataaat gtggaaaaaa tagagaaaca attgaaaaat
1741 tgctgtctaa actattatgt gtgtctccaa tgtgtatgat gatagagcct ccaaaattgc
1801 gtagtacagc agcagcatta tattggtata aaacaggtat atcaaatatt agtgaagtgt
1861 atggagaca--ccagaatgg atacaaagac aaacagtatt acaacatagt tttaatgatt
1921 gtacatttga attatcacag atggtacaat gggccta--a taatgacata gtaga--ata

## Figure 4-2

```
1981 gtgaaattgc atataaatat gcacaattgg cagacactaa tagtaatgca agtgcctttc
2041 taaaaagtaa ttcacaggca aaaattgtaa aggattgtgc aacaatgtgt agacattata
2101 aa--agcaga aaaaaaacaa atgagtatga gtcaatggat aaaatataga tgtgataggg
2161 tagatgatgg aggtgattgg aagcaaattg ttatgttttt aaggtatcaa ggtgtagagt
2221 ttatgtcatt tttaactgca ttaaaaagat ttttgcaagg catacctaaa aaaaattgca
2281 tattactata tggtgcagct aacacaggta aatcattatt tggtatgagt ttaatgaaat
2341 ttctgcaagg gtctgtaata tgttttgtaa attctaaaag ccatttttgg ttacaaccat
2401 tagcagatgc caaaataggt atgttagatg atgctacagt gccctgttgg aactacatag
2461 atgacaattt aagaaatgca ttggatggaa atttagtttc tatggatgta aagcatagac
2521 cattggtaca actaaaatgc cctccattat taattacatc taacattaat gctggtacag
2581 attctaggtg gccttattta cataatagat tggtggtgtt tacatttcct aatgagtttc
2641 catttga--a aaa--gaaat ccagtgtatg agcttaatga taagaactgg aaatcctttt
2701 tctcaagga--tggtccaga ttaagtttgc a--agga--a ggacaaggaa aa--atggag
2761 actctttgcc aa--tttaaa tgtgtgtcag gacaaaatac taacacatta tgaaaatgat
2821 agtacagacc ta--tgacca tatagactat tggaaacaca tg--cctaga atgtgctatt
2881 tattacaagg ccagagaaat gggatttaaa catattaacc accaagtggt gccaacactg
2941 gctgtatcaa agaataaagc attacaagca attgaactgc aactaa--tt agaaacaata
3001 tataactcac aatatagtaa tgaaaagtgg acattacaag a--ttagcct tgaagtgtat
3061 ttaactgcac caacaggatg tataaaaaaa catggatata cagtggaagt gcagtttgat
3121 ggagacatat gcaatacaat gcattataca aactggacac atatatatat ttgtgaagaa
3181 gcatcagtaa ctgtggtaga gggtcaagtt gactattatg gtttatatta tgttcatgaa
3241 ggaata--aa catattttgt gcagtttaaa gatgatgcag aaaaatatag taaaaataaa
3301 gtatgggaag ttcatg--gg tggtcaggta atattatgtc ctacatctgt gtttagcagc
3361 aa--aagtat cctctcctga aattattagg cagcacttgg ccaaccaccc --c----acc
3421 cataccaaag c--t--cctt gggcac--aa gaaacacaga --actatcca g--accaaga
3481 tcagagccag acac--gaaa cccctgccac accactaagt tgttgcacag agactcagtg
3541 gacagtgctc caatcctcac tgcatttaac agctcacaca aagga--gat taactgtaat
3601 agtaacacta cacccatagt acatttaaaa ggtgatgcta atactttaaa atgtttaaga
3661 tatagattta aaaagcattg tacattgtat actgcagtgt --tctacatg gcattggaca
3721 ggacataatg taaaacataa aagtgcaatt gttacactta catatgatag tgaatggcaa
3781 --tgaccaat ttttgtctca agttaaaata ccaaaaacta ttacagtgtc tactggattt
3841 atgtctatat gacaaatctt gatactgcat ccacaacatt actgg--tgc tttttgcttt
3901 gctttgtgtg cttttgtgtg tctgcctatt aata--tc-- ctgcttttgt ctgtgtctac
3961 atacacatca ttaataatat tggtattact attgtggata acagcagcct ctg--tttag
4021 gtgttttatt gtatatatta tatttgttta tataccatta tttttaatac atacacatgc
4081 a--cttttta attacataat gtatatgtac ataatgtaat tgttacatat aattgttgta
```

## Figure 4-3

4141 taccataact tactattttt tcttttttat tttcatatat aattttttttt tttgtttgtt
4201 tgtttgtttt ttaataaact gttattactt aacaatg--a cacaaa--tt ctgcaaaa--
4261 cacaaaa--t gcat--gcta cccaacttta taaaacatgc aaacaggcag gtacatgtcc
4321 acctgacatt atacctaagg ttgaaggcaa aactattgct gaacaaatat tacaatatgg
4381 aagtatgggt gtatttttg gtgggttagg aattggaaca gggt--ggta cagg--ga--
4441 cactgggtat attccattgg gaacaaggcc tcccacagct acagatacac ttgctcctgt
4501 aagaccccct ttaacagtag atcctgtggg cccttctgat ccttctatag tttctttagt
4561 ggaagaaact agttttattg atgctggtgc accaacatct gtaccttcca ttccccccaga
4621 tgtatcagga tttagtatta ctacttcaac tgataccaca cctgctatat tagatattaa
4681 taatactgtt actactgtta ctacacataa taatcccact ttcactgacc catctgtatt
4741 gcagcctcca acacctgcag aaactggagg gcattttaca ctttcatcat ccactattag
4801 tacacataat tatgaagaaa ttcctatgga tacatttatt gttagcacaa accctaacac
4861 agtaactagt agcacaccca taccagggtc t--cccagtg gca--cctag gattatatag
4921 t--cacaaca caacaggtta aagttgtaga ccctgctttt gtaaccactc ccactaaact
4981 tattacatat gataatcctg catatgaagg tatagatgtg gataatacat tatattttc
5041 tagtaatgat aatagtatta atatagctcc agatcctgac tttttggata tagttgcttt
5101 acataggcca gcattaacct ctagg--tac tggcattagg tacagtagaa ttggtaataa
5161 acaaacacta --tact--ta gtggaaaatc tataggtgct aaggtacatt attattatga
5221 tttaagtact attgatcctg cagaagaaat agaattacaa actataacac cttctacata
5281 tactaccact tcacatgcag cctcacctac ttctattaat aatggattat atgatattta
5341 tgcagatgac tttattacag atacttctac aaccc--gta ccatctgtac cctctacatc
5401 tttatcaggt tatattcctg caaatacaac aattcctttt ggtggtgcat acaatattcc
5461 tttagtatca ggtcctgata tacccattaa tataactgac caagctcctt cattaattcc
5521 tatagttcca gggtctccac aatatacaat tattgctgat gcaggtgact tttatttaca
5581 tcctagttat tacatgtta--1 aaaa--2a--3 taaa--4ttta ccatattttt tttcagatgt
5641 ctctttggct gcctagtgag gccactgtct acttgcctcc tgtcccagta tctaaggttg
5701 taagca--5ga tgaatatgtt gca--6cacaa acatatatta tcatgcagga acatccagac
5761 tacttgcagt tggacatccc tattttccta ttaaaaaacc taacaataac aaaatattag
5821 ttcctaaagt atcaggatta caatacaggg tatttagaat acatttacct gaccccaata
5881 agtttggttt tcctgacacc tcattttata atccagatac acag--7gctg gtttgggcct
5941 gtgtaggtgt tgaggtaggt --8tggtcagc cattaggtgt gggcattagt ggccatcctt
6001 tattaaataa attggatgac acagaaaatg ctagtgctta tgcagcaaat gcaggtgtgg
6061 ataatagaga atgtatatct atggattaca aacaaacaca attgtgttta attggttgca
6121 aaccacctat aggggaacac tggggcaaag gatccccatg taccaatgtt gcagtaaatc
6181 caggtgattg tccaccatta gagttaataa acacagttat tcaggatggt gatatggttc
6241 atactggctt tggtgctatg gactttacta cattacaggc taacaaaagt gaagttccac

## Figure 4-4

6301 tggatatttg tacatctatt tgcaaatatc cagattatat taaaatggtg tcagaaccat
6361 atgg--9acag cttatttttt tattta--10aa gggaacaaat gtttgttaga catttattta
6421 atagggctgg tactgttggt gaaaatgtac caga--11attt atacattaaa ggctctgggt
6481 ctactgcaaa tttagccagt tcaaattatt ttcctacacc tagtggttct atggttacct
6541 ctgatgccca aatattcaat aaaccttatt ggttacaa--12 agcacagggc cacaataatg
6601 gcatttgttg gggtaaccaa ctatttgtta ctgttgttga tactaca--13c agtacaaata
6661 tgtcattatg tgctgccata tctacttcag aaactacata taaaaatact aactttaagg
6721 agtaccta-- 14acatggggag gaatatgatt tacagtttat ttttcaactg tgcaaaataa
6781 ccttaactgc aga--15ttatg acatacatac attctatgaa ttccactatt ttggaggact
6841 ggaattttgg tctacaacct cccccaggag gcacactaga agatacttat aggtttgtaa
6901 cccaggcaat tgcttgtcaa aaacatacac ctccagcacc taaagaagat gatcccctta
6961 aaaaatacac tttttgggaa gtaaatttaa aggaaaagtt ttctgcagac ctagatcagt
7021 ttcctttagg a--16caaattt ttactacaag caggattgaa ggccaaacca aaatttacat
7081 taggaaaa--17 aaaagctaca cccaccacct catctacctc tacaactgct aaa--18caaaa
7141 aa--19taagct gtaagtattg tatgtatgtt gaattagtgt tgtttgttgt gtatatgttt
7201 gtatgtgctt gtatgtgctt gtaaatatta agttgtatgt gtgtttgtat gtatggtata
7261 ataaaca--20t gtgtatgtgt ttttaaatgc ttgtgtaact attgtgtcat gcaacataaa
7321 taaacttatt gtttcaacac ctactaattg tgttgtggtt attcattgta tataaactat
7381 atttgctaca tcctgttttt gttttatata tactatattt tgtag--21cca g--22gccatttt
7441 gtagcttcaa c--23aatt--24g ttgcatgctt tttggcacaa aatgtgtttt tttaaatagt
7501 tctatgtcag caactatggt ttaaacttgt a--25tttcctg cttgccatg--26tgccaaatc
7561 cctgttttcc tgacctgcac tgcttgccaa ccattccatt gtttttttaca ctgcactatg
7621 tgcaactact gaatcactat gtacattgtg tcatataaaa taaatcacta tg--27ccaa--28
7681 ccttacatac --29ctgttagg cacatatttt tggcttgttt taactaacct aattgcatat
7741 ttggcataag gtttaaactt ctaaggccaa ctaaatgtca ccctagttca tacatgaact
7801 gtgtaaaggt tagtcataca ttgttcattt gtaaaactgc acatgggtgt gtgcaaac--
7861 attttgggtt acacatttac aagcaactta tataataata ctaa

# Figure 5-1

-- : denotes CG.

[--] : denotes CG in L1 region.

The number appended after "--" corresponds to the position of CpG shown in Figs. 1 to 3.

Total genomic DNA sequence of HPV-52 (NC_001592 ; SEQ ID NO:2 )

```
   1 taaattataa tcttatacta gtaaaaaata gggtgtaac--aaaa--gtc agac--aaac
  61 --gtgtatat atatagaaca cagtgtagct aa--ca--gc catgtttgag gatccagcaa
 121 ca--accc-- gaccctgca--aattgtgtg aggtgctgga agaat--gtg catgaaataa
 181 ggctgcagtg tgtgcagtgc aaaaaagagc tacaa--aag agaggtatac aagtttctat
 241 ttacagattt a--aatagta tatagagaca ataatccata tgg--tgtgt attatgtgcc
 301 ta--cttttt atctaagata agtgaatata ggcattatca atattcactg tatgggaaaa
 361 cattagaaga gagggtaaaa aaaccattaa gtgaaataac tattagatgt ataatttgtc
 421 aaa--ccatt atgtcctgaa gaaaaagaaa gacatgttaa tgcaaacaag --atttcata
 481 atattatggg t--ttggaca ggg--ctgtt cagagtgttg gagaccc--a cctgtgaccc
 541 aagtgtaa-- tcatg--tgg agacaaagca actataaaag attatatatt agatctgcaa
 601 cctgaaacaa ctgacctaca ctgctatgag caattaggtg acagctcaga tgaggaggat
 661 acagatggtg tggac--gcc agatggacaa gcagaacaag ccacaagcaa ttactacatt
 721 gtgacatatt gtcacagttg tgatagcaca cta--gctat gcattcatag cactg--a--
 781 gacctt--ta ctctacagca aatgctgttg ggcacattac aagttgtgtg ccc--gctgt
 841 gca--gctat aaacaaccct gcaatggagg accctgaagg tacagaggg--aaagggagg
 901 gatgtacagg ctggtttgaa gtagaggcaa taatagaaaa acaaacagga gataacattt
 961 cagagga--a ggatgaaaat gcatatgata gtggaacaga tctaatagat tttatagatg
1021 attcaaatat aaataatgaa caggcagaac atgaggcagc c--ggcattg tttaatgcac
1081 aggaagggga ggatgattta catgctgtgt ctgcagtaaa a--aaagttt acaagcagtc
1141 --gaaagtgc tgggcaagat ggtgtagaaa aacatggtag tc----tgca aaacacattt
1201 gtgtaaatac agagtgtgtt ttaccaaaa--caaaccatg tca--tagaa gacag--gct
1261 atggcaatag tgaagtggaa g--cagcaga tggcagacca ggtaga--gg caaaatgg--
1321 actggcaaag taacagtagt caatcaagtg gggtgggggc tagtaattca gatgtaagtt
1381 gtactagtat agaggacaat gaggaaaata gtaatagaa--ctaaaagc atacaaaata
1441 ttatgtg--a aaatagcata aaaacaactg tattatttaa atttaaagaa acatatggtg
1501 ttagctttat ggaattagta agaccattta aaagtaatag aagtagttgt acagattggt
1561 gtattatagg aatgggagta acaccatcag ttgcagaagg attaaaagta ttaatacagc
1621 cctatagcat atatgcccat ttgcaatgtt taacatgtga cagagg--tg cttatactgc
1681 tgctaattag gtttaaatgt ggaaaaaaca gattaacagt gtccaaacta atgtcacagc
1741 tgttaaatat accagaaaca catatggtaa tagaaccacc aaaatta--a agtgctacct
1801 gtgcattata ttggtataga acaggtttgt ctaatattag tgaggtatat ggtaccaccc
1861 cagaatggat agaacaacaa acagtattac agcatagctt tgacaatagc atatt--att
```

## Figure 5-2

1921 ttggagaaat ggtgcaatgg gcatatgatc atgatataac agatgatagt gacatagcat
1981 ataaatatgc acagttagca gatgtaaata gcaatgctgc agcattccta aaaagcaatt
2041 --caagcaaa aatagtaaag gactgtgcaa ccatgtgtag acattataaa --ggcagaaa
2101 gaaaacatat gaatattgga caatggatac agtatagatg tgatagaata gatgatggtg
2161 gagattggag gcctatagta agatttttaa gatatcaaga catagaattt acagcctttt
2221 taga--catt taaaaaattt ttaaaaggta tacctaaaaa aaattgttta gtattatatg
2281 gacctgcaaa cacaggaaaa tcatattttg gaatgagttt aattaggttc ttaagtggat
2341 gtgtaatatc ctatgtaaac tcaaaaagcc atttttggct acaaccatta acagatgcaa
2401 aagtgggtat gatagatgat gtaacaccta tatgttggac atatatagat gattatatga
2461 gaaatgcact ggatggaaat gatatatcag tagatgtaaa gcatagagcc ttagtacaaa
2521 taaaatgccc accattaatt ttaacaacaa atacaaatgc aggaacagat cctaggtggc
2581 catatttaca tagtagattg gttgtgtttc atttcaaaaa cccatttcca tttgatgaaa
2641 atggcaatcc tatatatgaa attaacaa-- aaaattggaa atccttttttc tcaagga--t
2701 ggtgcaaatt agatttaata caggaagagg acaaggaaaa --atggagt--atac--gca
2761 --tttaaatg cagtgcagga aaaaatacta gatctata-- aagctgatag taatgaccta
2821 aa--cacaaa ttgaacattg gaaattgact --aatggaat gtgttttgtt ttacaaagca
2881 aaggaactgg gaataactca tataggccac caggtggtgc caccaatggc agtgtctaag
2941 gcaaaggcct gccaagctat tgaactacaa ttggcattgg aggcattaaa caaaacacaa
3001 tatagcacag atggatggac attacaacaa acaagtctag aaatgtgg-- tgcagaacca
3061 caaaaatact ttaaaaaaca tgggtataca ataacagtgc aata--ataa tgataaaaac
3121 aatactatgg attatacaaa ctggaaggaa atttatttac ttggtgagtg tgaatgtaca
3181 attgtagaag gacaagtaga ttactatggg ttatattatt ggtgtgatgg agaaaaaata
3241 tattttgtaa aatttagtaa --atgcaaag caatattgtg taacaggagt atgggaagta
3301 catgtgggtg gtcaggtaat tgtttgtcct gcatctgtat ctagtaa--a agtatccact
3361 actgaaactg ctgtccacct atgcac--aa acctccaaga cctc--cagt gtc--tgggt
3421 gccaaagaca cacacctaca accaccacag aaa--a--a--accaga--t cacagactcc
3481 agaaacacca agtaccccaa caacctttg --gggacaac aatc--tgga cagtactaca
3541 --gggact-- tcactgcaac tgagtgcaca aacaaagga--ggttgcaca tacaacttgt
3601 actgcaccta taatacacct aaaaggtgat cctaatagtt taaaatgttt aagatatagg
3661 gtaaaaacac ataaaagttt gtatgttcaa atttcatcta cctggcattg gaccagtaat
3721 gaatgtacaa ataataaact aggtattgta acaataa--t acagtgatga aacacaa--t
3781 caacaatttt taaaaactgt taaaatacca aatactgtgc aagttataca aggtgtcatg
3841 tcattgtgat atttgtacat atgtatatat gtatatgtgt atggtaaaca cccaacacaa
3901 gccaatattg ctgctattgt gtatatataa caatgttagg attatttgta ttttgtttta
3961 ttttgcttat ggtgttttgt gcagtgctta ggc--ctctt gctatctata t--gtgtatg
4021 --caggtgtt ggtgctggtg cttttgctat gggtatctat tgggtcacca tttaaagtgt
4081 tttttttgta cctactgttt ttatattttc caatgttttg tattcactgt catgcacagt

## Figure 5-3

4141 atttggcaca actgcaataa ctgtacatgt agattggcta catgcatata tgcaaaatat
4201 actttttcac ttttgtagtt tgtctaataa atacttttat attttttaat agcttgt--c
4261 aatgagatac aga--gtcta ca--gcacaa a--tgcttct gcaacacagc tatatcaaac
4321 atgcaaagcc tctggcacct gcccccc--a tgttattcct aaagtggaag gcacaactat
4381 tgcagatcaa cttttaaaat atggcagcct aggggtgttt tttggaggtt tgggtatagg
4441 tacaggtgca ggctctggtg gtagggcagg ctatgtgcca ttgtccact--tcctcccac
4501 tagtagtatt acca--tcca ccatt--tcc ccctgtaact gtagaaccca ttggtccctt
4561 agaaccatct atagtttcta tgatagaaga aacaacattt attgagtctg g--cacctgc
4621 tccatctatt ccatcagcaa cagggtttga tgttacaaca tctgcaaata atactcctgc
4681 aataattaat gtaacatcta taggtgaatc atctgtacaa tcagtttcta cacatttaaa
4741 tcctacattc actgaaccat ctataataca gcccc--gca cctgcagaag catctggtca
4801 tgtattgttt tctagtccaa ctattagtac acacacctat gaagaaatcc ctatggatac
4861 atttgttacc tctactgaca gcagcagtgt aacaagtagt acacctattc cagggtct--
4921 cccta--aca --ccttggtt tatatagc-- tgccacacaa caggttaagg tagt--accc
4981 tgcttttatg tcatcaccac agaaattagt aacatataac aatcctgttt ttgaggg--t
5041 tgatacagat gaaactataa tttttgat-- ttcacaactt ttacctgcac --gatcctga
5101 tttttagac attatagctt tgcataggcc tgcattaacc tct--aagag gtactgttag
5161 gtttagcagg cttggtaata aggccaccct a--taca--t agtggaaaac aaattggggc
5221 a--ggtacat tattatcatg atattagtcc tatccagcct gctgaagttc aggaagacat
5281 agaattgcaa cctttattac cacagtctgt gtccccttac actattaatg atggtttgta
5341 tgatgtgtat gcagattctt tgcagcaacc ca--tttcac ttaccttcca cactttctac
5401 ccataataat actttcactg tacctattaa tagtggtatt gactttgtat atcaacccac
5461 tatgtccatt gagtcaggtc ctgacattcc attacctt-- ttacccacac atactccttt
5521 tgttcctata gcccctacag ctccatctac atctattatt gttgatggta cagattttat
5581 tttacatcct agttattttt tacta--1t--2 cagg--3taaa --4ttttccat attttttac
5641 agatgtc--5t gtgg--6gcct agtgaggcca ctgtgtacct gcctcctgta cctgtctcta
5701 aggttgtaag cactgatgag tatgtgtct--7cacaagcat ctattattat gcaggcagtt
5761 ct--8attact aacagtagga catccctatt tttctattaa aaacaccagt agtggtaatg
5821 gtaaaaaagt tttagttccc aaggtgtctg gcctgcaata cagggtattt agaattaaat
5881 tgc--9gaccc taataaattt ggttttccag atacatcttt ttataaccca gaaacccaaa
5941 ggttggtgtg ggcctgtaca ggcttggaaa ttggtagggg acagccttta ggtgtgggta
6001 ttagtgggca tcctttatta aacaagtttg atgatactga aaccagtaac aaatatgctg
6061 gtaaacctgg tatagataat agggaatgtt tatctatgga ttataagcag actcagttat
6121 gcattttagg atgcaaacct cctataggtg aacattgggg taagggaacc ccttgtaata
6181 ataattcagg aaatcctggg gattgtcctc ccctacagct cattaacagt gtaatacagg

## Figure 5-4

6241 atggggacat ggtagataca ggatttggtt gcatggattt taataccttg caagctagta
6301 aaagtgatgt gcccattgat atatgtagca gtgtatgtaa gtatccagat tatttgcaaa
6361 tggctag--10a gccatatggt gacagtttgt tctttttct taga--11tgag caaatgtttg
6421 ttagacactt ttttaatagg gc--12gtacct taggtgaccc tgtgccaggt gatttatata
6481 tacaagggtc taactctggc aatactgcca ctgtacaaag cagtgctttt tttcctactc
6541 ctagtggttc tatggtaacc tcagaatccc aattatttaa taaac--13tac tggttacaa-
6601 -14tg--15caggg ccacaataat ggcatatgtt ggggcaatca gttgtttgtc acagttgtgg
6661 ataccact--16 tagcactaac atgactttat gtgctgaggt taaaaaggaa agcacatata
6721 aaaatgaaaa ttttaaggaa taccttt--17tc atgg--18agga atttgattta caatttattt
6781 ttcaattgtg caaaattaca ttaacagctg atgttatgac atacattcat aagatggatg
6841 ccactatttt agaggactgg caatttggcc ttaccccacc ac--19tctgca tctttggagg
6901 acacatacag atttgtcact tctactgcta taacttgtca aaaaaacaca ccacctaaag
6961 gaaaggaaga tcctttaaag gactatatgt tttgggaggt ggatttaaaa gaaaagtttt
7021 ctgcagattt agatcagttt cctttaggta ggaagttttt gttacaggca gggctacagg
7081 ctaggcccaa actaaaa--20c cctgcatcat --21gcccca--22 tacctccaca aagaagaaaa
7141 aggttaaaag gtaaccattg tctgttgggt aattgtctgt gtcatgtatg tgttgtgtat
7201 gtcaaacaca ggttaaaagg taaccattgt ttgttatgta attgttttgt gtgtgtactg
7261 tgttgtttgc atgttatgta tgtgtgtgca tgtttgttgt atttgtcagt tcctgtatgt
7321 atgttttgtg tatgtattaa taaagtactg tatttactaa actatttata gtagtcttat
7381 gttatgttat ggttgcaccc acatgagtaa caatacagtt gctcctaatc tattgcatct
7441 cctgccctac cctgtgtccc ctgccctacc ctgtgtccta ctttgttaca ctactaatta
7501 gccttatact ctccattttg taccattttg tactatccac cattttaaat cctaac--23aa
7561 tt--24gttggt cttggcacaa ctttggttgt ccttggcaca gtaacaacta ttttatata
7621 agtttcagca aactgcttaa tcctttggtt tcctgcagtc cactggtcta cacttgttgt
7681 cc--25cctaaa ctgacttctt gctgactcac aggtcctgca gtgcagctaa acaatacatt
7741 gcctaacatt gcatgtttta aactgctttt aggcacatat tttatttaaa ctttcaatgc
7801 actaattaca gtgttggctt acacaagtac atccta--26cc aaatatgtct tgtaaaacat
7861 gattaaatac tgttactcac caggtgtgca ctaca--27ac--28gtta--29gtt ac--30taccca
7921 caaccacttt tttttataat ta

## Figure 6-1

-- : denotes CG.
[--] : denotes CG in L1 region.
The number appended after "--" corresponds to the position of CpG shown in Figs. 1 to 3.

**Total genomic DNA sequence of HPV-58 (NC_001443 :SEQ ID NO:2 )**

1 ctaaactata atgccaaatc ttgtaaaaac tagggtgtaa c--30aaaa--31g tctgac--32aa
61 ac--33gtgcat atataaagca gacatttttt ggtaggctac tgcaggacta tgttccagga
121 --cagaggag aaacca--ga cattgcatga tttgtgtcag g--ttggaga catctgtgca
181 tgaaat--aa ttgaaatg-- ttgaatgcaa aaagactttg cag--atctg aggtatatga
241 ctttgtattt gcagatttaa gaatagtgta tagagatgga aatccatttg cagtatgtaa
301 agtgtgctta --attgctat ctaaaataag tgagtataga cattataatt att--ctata
361 tggagacaca ttagaacaaa cactaaaaaa gtgtttaaat gaaatattaa ttagatgtat
421 tatttgtcaa agaccattgt gtccacaaga aaaaaaaagg catgtggatt taaacaaaag
481 gtttcataat attt--ggt--ttggacagg g--ctgtgca gtgtgttgga gaccc--a--
541 tagacaaaca caagtgtaac ctgtaacaa--ccatgagag gaaacaaccc aa--ctaaga
601 gaatatattt tagatttaca tcctgaacca actgacctat tctgctatga gcaattatgt
661 gacagctcag a--aggatga aataggcttg ga--ggccag atggacaagc acaac--gcc
721 acagctaatt actacattgt aacttgttgt tacacttgtg gcacca--gt t--tttgtgt
781 atcaacagta caacaac--a --ta--aacc ctacagcagc tgcttatggg cacatgtacc
841 attgtgtgcc ctagctgtgc acagcaataa acaccatctg caatggatga ccctgaaggt
901 acaaa--ggg taggggg--gg ctgtactggc tggtttgagg tagaag--gt aatagaa--a
961 agaacaggag ataatatttc agatgatgag ga--aaacag caga--atag tggtacagat
1021 ttaatagagt ttatagatga ttcagtacaa agtactacac aggcagaagc agaggcagcc
1081 --ag--ttgt ttaatgtaca ggaaggggtg ga--atataa atgctgtgtg tgcactaaaa
1141 --aaagtttg cagcatgctc agaaagtgct gtagaggact gtgtggac-- ggctgcaaat
1201 gtgtgtgtat --tggaaata taaaaataaa gaatgcacac acagaaaa-- aaaaattatt
1261 gagctagaag acag--gata tggcaatact gaagtggaaa ctgagcagat ggcacaccag
1321 gtagaaagcc aaaatgg--a --cagactta aatgact--g agtctagtgg ggtgggggct
1381 agttcagatg taagcagtga aa--gatgta gacagttgta atactgttcc attacaaaat
1441 attagtaata ttctacataa cagtaatact aaagcaa--c tattatataa attcaaagaa
1501 gcttatggag taagttttat ggaattagtt agaccattta aaagtgataa aacaagctgt
1561 acagattggt gtataacagg gtatggaata agtccctc-- tagcagaaag tttaaaagta
1621 ctaattaaac agcacagtat atatacacac ctacaatgtt taa--tgtga cagaggaatt
1681 atattattat tgttaattag atttaaatgt agcaaaaata gattaactgt ggcaaaatta
1741 atgagtaatt tactatcaat tcctgaaaca tgtatgatta t--agccacc aaaatta--a
1801 agtcaagcat gtgccttata ttggtttaga acagcaatgt caaatataag tgatgtgcaa

## Figure 6-2

1861 gggacaacac cagaatggat agatagatta acagtgttac agcatagctt taatgatgat
1921 atatttgatt taagtgaaat gatacaatgg gcatatgata atgacattac agatgatagt
1981 gacattgcat ataaatatgc acagttagca gatgttaata gtaatgcagc agcattttta
2041 agaagcaatg cacaagcaaa aatagtaaaa gactgtgg-- ttatgtgcag acattataaa
2101 agagcagaaa ag--tggtat gacaatggga caatggatac aaagtaggtg tgaaaaaaca
2161 aatgatggag gtaattggag accaatagta caatttttaa gatatcaaaa tattgaattt
2221 acagcatttt tagttgcatt taaacagttt ttacaaggtg taccaaaaaa aagttgtatg
2281 ttactgtgtg gcccagcaaa tacagggaaa tcatattttg gaatgagttt aatacatttt
2341 ttaaaaggat gcattatttc atatgtaaat tccaaaagtc atttttggtt gcagccatta
2401 tcagatgcta aactaggtat gatagatgat gtaacagcca taagctggac atatatagat
2461 gattatatga gaaatgcatt agatggtaa--acatttcaa tagatgtaaa acatagggca
2521 ttagtacaat taaaatgtcc accattaata attacctcaa atacaaatgc aggcaaagat
2581 tca--atggc catatttgca cagtagacta acagtatttg aatttaacaa tccatttcca
2641 tttgatgcaa atggtaatcc agtgtataaa ataaatgatg aaaattggaa atcctttttc
2701 tcaagga--t ggtgcaaatt aggcttaata gaggaagagg acaaggaaaa --atggagga
2761 aatatcagca --tttaagtg cagtgcagga caaaatccta gacatata-- aagctgataa
2821 aaatgattta acatcacaaa ttgaacattg gaaactaata --catggagt gtgctataat
2881 gtatacagcc agacaaatgg gaatatcaca tttgtgccac caggtggtgc --tcattggt
2941 agcatcaaag actaaag--t ttcaagtaat tgaactgcaa atggcattag agacattaaa
3001 tgcatcacca tataaaacag atgaatggac attgcaacaa acaagcttag aagtgtggtt
3061 atcagagcca caaaaatgct ttaaaaaaaa aggcataaca gtaactgtac aatatgacaa
3121 tgataaagca aacacaatgg attatacaaa ttggagtgaa atatatatta ttgaggaaac
3181 aacatgtact ttggtagcag gagaagttga ctatgtgggg ttgtattata tacatggcaa
3241 tgaaaaga-- tattttaaat attttaaaga ggatgcaaaa aagtactcta aaacacaatt
3301 atgggaggta catgtgggta gt--ggtaat tgtatgtcct acatctatac ctagtgatca
3361 aatatccact actgaaactg ctgacccaaa gaccac--ag gccaccaaca a--aaagtac
3421 acaggggaca aag--a--a--act--attt accagactcc agagacaaca cccagtactc
3481 cacaaagtat acagactg-- c--tggacag tagacca--a ggaggaggac tacacagtac
3541 aactaactgt acatacaaag gg--gaa--t gtgtagttct aaagtttcac ctat--tgca
3601 tttaaaaggt gacccaaata gtttaaaatg tttaagatat agattaaaac catttaaaga
3661 cttatactgt aatatgtcat ccacatggca ttggaccagt gatgacaaag gtgacaaagt
3721 aggaattgtt actgtaacat acacaa--ga aacacaa--a caactgtttt taaacactgt
3781 taaaatacca cccactgtgc aaataagtac tggtgttatg tcattgtaat tgtattgtac
3841 aattactgta tgtaaaccac aagccaatat gtgctgctaa gtgtatatac aatgatatta
3901 cctattttg ttgtttgttt tatactgttt ttatgcttgt gcattttttt g--gccattg

## Figure 6-3

3961 gtgctatcta tttctatata tgcttggttg ctggtgttgg tgttgctgct ttgggtgtct
4021 gtggggt--g ctcta--aat ttttttctgt tacttaatat ttttatatat accaatgatg
4081 tgtattaatt ttcatgcaca atacttaacc caacaagact aactgtatac tggttctgca
4141 catggtggta tggtattgta aatatttact gttgtgtgtg ttgtttttat tatttttata
4201 catttactaa taaatacttt tatattttta gcactgtctt attatgagac acaaa--gtc
4261 tacaagg--c aag--tgcat ctgctacaca actttaccaa acatgcaagg cctcaggcac
4321 ctgcccacct gatgttatac ccaaagttga aggcactact atagcagatc aaatatta--
4381 atatggtagc ttaggggtgt tttttggagg tttaggcatt ggtacagggt --ggtacagg
4441 tggcaggact ggatatgtgc cccttggtag taccccac-- tctgaggcta tacctttaca
4501 gcccata--t cccccagtta c--ttgatac tgtggggcct ttggattctt ctattgtatc
4561 tttaatagag gaatctagtt ttataga--c --gtgcacca gccccatcaa ttcccactcc
4621 atctggtttt gatattacca cctctgcaga tactacacct gcaatactta atgtttcctc
4681 tattggagaa tcatctatac aaactgtttc tacacattta aatccctcct ttactgagcc
4741 atc--tactc --ccctcctg cacctgcaga ggcctctgga catttaatat tttcctctcc
4801 tactgttagc acacatagtt atgaaaacat accaatggat acctttgtta tttctactga
4861 cagtggcaat gtca--tcta gcacacccat tccagggtct --ccctgtgg ca--ccttgg
4921 tttatacagt --caacaccc aacaagttaa ggttgttgac cctgcttttt taacatctcc
4981 tcatagactt gtaacatatg ataatccagc atttgaaggc tttaaccctg aggacacatt
5041 gcagtttcaa catagtgaca tat--cctgc tcctgatcct gattttctag atattgttgc
5101 attacacaga cctgcattaa cctct--cag gggtactgta --ttatagta gggttgggca
5161 aaaggctaca ctt--tact--cagtggaaa gcaaataggg gctaaagtac attactacca
5221 agacttaagt cccatacagc ctgtccagga acaggtacaa cagcagcaac aatttgaatt
5281 acaatcttta aatacttctg tttctcccta tagtattaat gatggacttt atgatattta
5341 tgctga--at gctgatacta tacatgattt tcagagtcct ctgcactcac ata--tcctt
5401 tgccaccaca --taccagta atgtgtccat accattaaat actggatttg acactcctct
5461 tgtgtcattg gaacctggtc cagacattgc atcttctgta acatctatgt ctagtccatt
5521 tattcctata tctccactaa ctcctttttaa taccataatt gtggatggtg ctgattttat
5581 gttgcaccct agctatttta ttttg--1t--2 caga--3taaa --4ttttccat atttttttgc
5641 agatgtc--5t gtgg--6gcct agtgaggcca ctgtgtacct gcctcctgtg cctgtgtcta
5701 aggttgtaag cactgatgaa tatgtgtca--7cacaagcat ttattattat gctggcagtt
5761 ccagactttt ggctgttggc aatccatatt tttccatcaa aagtcccaat aacaataaaa
5821 aagtattagt tcccaaggta tcaggcttac agtatagggt ctttagggtg --8tttacctg
5881 atcccaataa atttggtttt cctgatacat ctttttataa ccctgataca caa--9tttgg
5941 tctgggcatg tgtaggcctt gaaataggta ggggacagcc attgggtgtt gg--10taagtg
6001 gtcatcctta tttaaataaa tttgatgaca ctgaaaccag taacagatat cc--11cacagc

## Figure 6-4

6061 cagggtctga taacagggaa tgcttatcta tggattataa acaaacacaa ttatgtttaa
6121 ttggctgtaa acctcccact ggtgagcatt ggggtaaagg tgttgcctgt aacaataatg
6181 cagctgctac tgattgtcct ccattggaac tttttaattc tattattgag gatggtgaca
6241 tggtagatac agggtttgga tgcatggact ttggtacatt gcaggctaat aaaagtgatg
6301 tgcctattga tatttgtaac agtacatgca aatatccaga ttatttaaaa atggccagtg
6361 aaccttatgg ggatagtttg ttcttttttc ttaga--12tga gcagatgttt gttagacact
6421 tttttaatag ggctggaaaa cttgg--13agg ctgtcc--14ga tgacctttat attaaagggt
6481 c--15gtaatac tgcagttatc caaagtagtg catttttttcc aactcctagt ggctctatag
6541 ttacctcaga atcacaatta tttaataagc cttattggct acag--16tgca caaggtcata
6601 acaatggcat ttgctggggc aatcagttat ttgttac--17t ggttgatacc act--18tagca
6661 ctaatatgac attatgcact gaagtaacta aggaaggtac atataaaaat gataatttta
6721 aggaatatgt a--19tcatgtt gaagaatatg acttacagtt tgtttttcag ctttgcaaaa
6781 ttacactaac tgcagagata atgacatata tacatactat ggattccaat attttggagg
6841 actggcaatt tggtttaaca cctcctc--20t ctgccagttt acaggacaca tatagatttg
6901 ttacctccca ggctattact tgccaaaaaa cagcaccccc taaagaaaag gaagatccat
6961 taaataaata tactttttgg gaggttaact taaaggaaaa gttttctgca gatctagatc
7021 agtttccttt ggga--21aaag tttttattac aatcaggcct taaagcaaag cccagactaa
7081 aa--22tt--23gc ccctactacc --24tgcaccat ccaccaaa--25 caaaaaggtt aaaaaataat
7141 tgttgtggta cttacactat tttattatac atgtttgttt gttttatgta tgtgttgtct
7201 gtttgtttat gtttgtgtat atgttgtatg tgttatgtgt catgtttgtg tacatgttct
7261 atgtccttgt cagtttcctg tttctgtata tatgtaataa actattgtgt gtattgtaaa
7321 ctatttgtat tgtttgggtg tatctatgag taaggtgctg tccctaaatt gccctaccct
7381 gccctgccta ttatgcatac ctatgtaata gtatttgtat gatatgtatt ttatagtttt
7441 taacagtact gcctccattt tactttacct ccattttgtg catgtaac-- 26attt--27gttg
7501 ctggcacaaa --28tgtttttt ttaaactaca atttaaacaa tacagttaat cctttccctt
7561 cctgcactgc ttttgcctat acttgcatat gtgactcata tatacatgca gtgcagttgc
7621 aaaatgttta attatactca tagtttaaac atgcttatag gcacatattt taacttactt
7681 tcaatgctta agtgcagttt tggcttgcac aatagtttgt tatgccaaac tatgtcttgt
7741 aaaagtgact cactaacatt tattgccagg tgtggactaa c--29ttttggg tcacattgtt
7801 catgtttcaa cattttatat aata

Figure 7

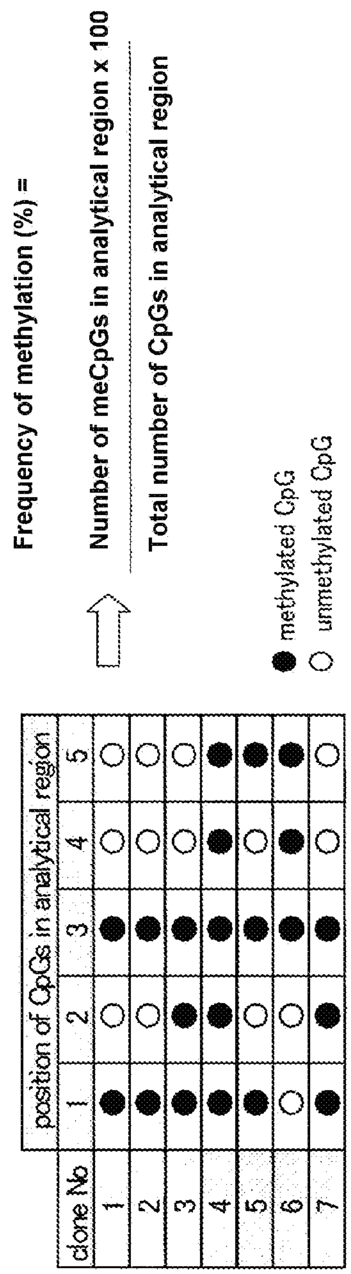
clone No
position of CpGs in analytical region
1
2
3
4
5
6
7
Frequency of methylation (%) =
Number of meCpGs in analytical region x 100
Total number of CpGs in analytical region
methylated CpG
unmethylated CpG

Figure 8

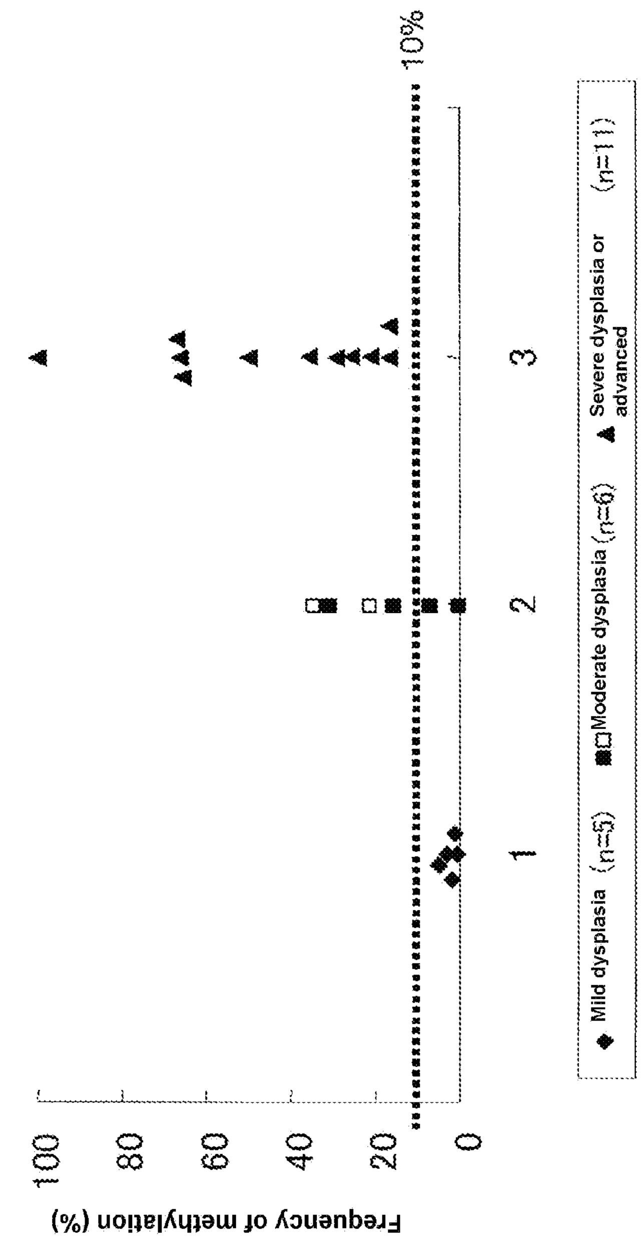
Frequency of methylation (%)
100
80
60
40
20
0
10%
1
2
3
Mild dysplasia (n=5)
Moderate dysplasia (n=6)
Severe dysplasia or advanced (n=11)

**Figure 9**

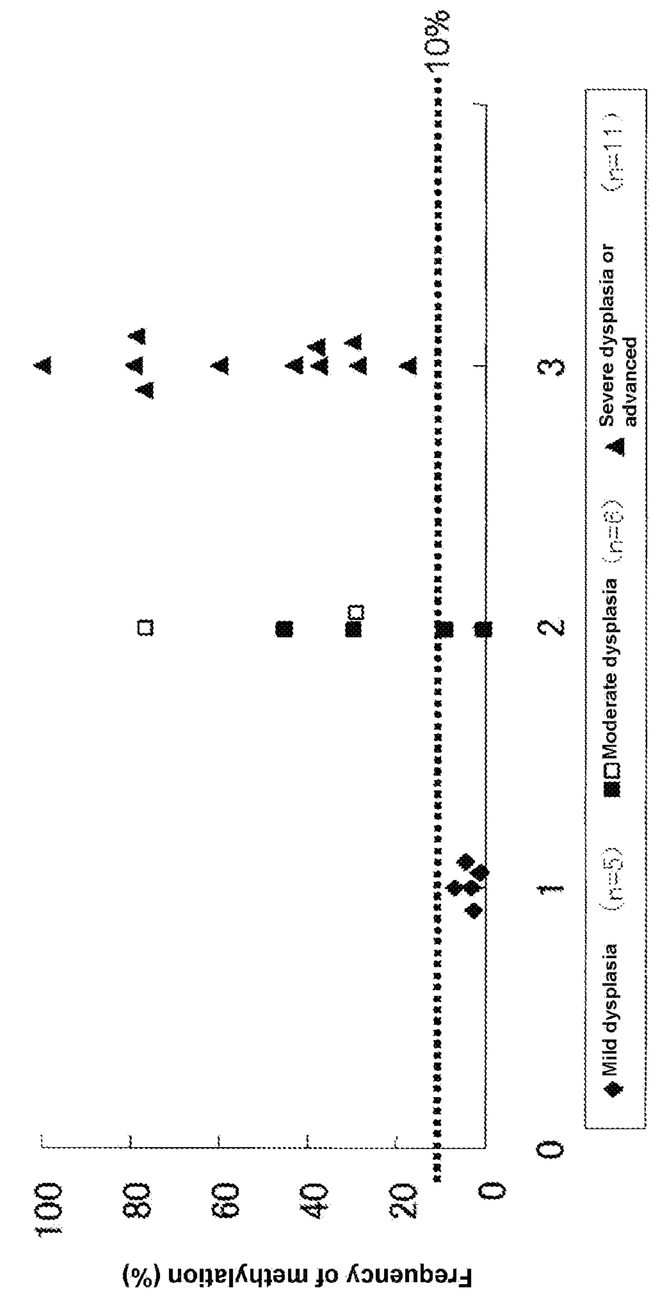
Frequency of methylation (%)
100
80
60
40
20
0
0
1
2
3
10%
Mild dysplasia (n=5)
Moderate dysplasia (n=6)
Severe dysplasia or advanced (n=11)

**Figure 10**

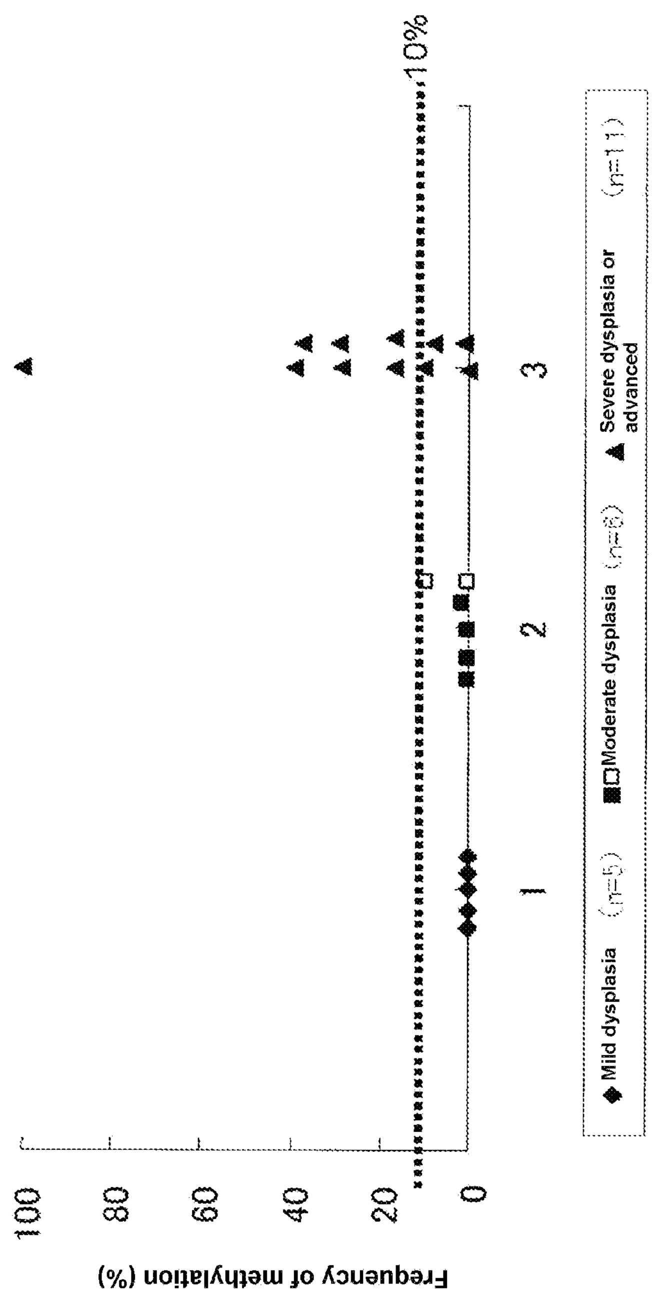
Frequency of methylation (%)
100
80
60
40
20
0
10%
1
2
3
Mild dysplasia (n=5)
Moderate dysplasia (n=8)
Severe dysplasia or advanced (n=11)

## Figure 11

| Sample No. | Histological diagnosis of biopsy sample | Methylation analysis results of biopsy sample | Histological diagnosis of operation sample |
|---|---|---|---|
| 57 | Mild dysplasia | ○ | No operation (successive CT Mild) |
| 62 | Mild dysplasia | ○ | No operation (successive CT Mild) |
| 66 | Mild dysplasia | ○ | Mild dysplasia |
| 46 | Severe dysplasia or advanced | ○ | Moderate dysplasia |
| 42 | Mild dysplasia | ● | Moderate dysplasia |
| 37 | Moderate dysplasia | ● | Severe dysplasia or advanced |
| 43 | Moderate dysplasia | ● | Severe dysplasia or advanced |
| 44 | Moderate dysplasia | ● | Severe dysplasia or advanced |
| 48 | Severe dysplasia or advanced | ● | Severe dysplasia or advanced |
| 49 | Severe dysplasia or advanced | ○ | Severe dysplasia or advanced |

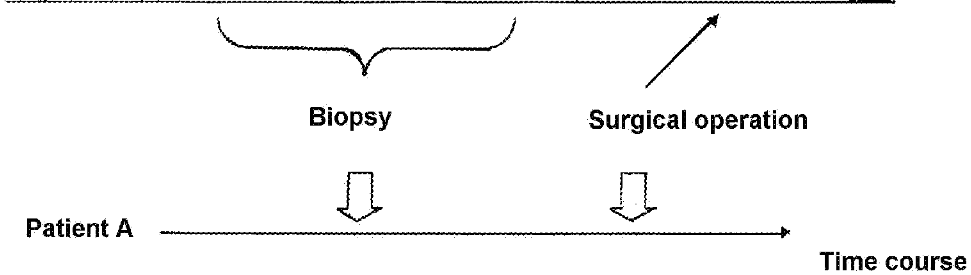

Figure 12

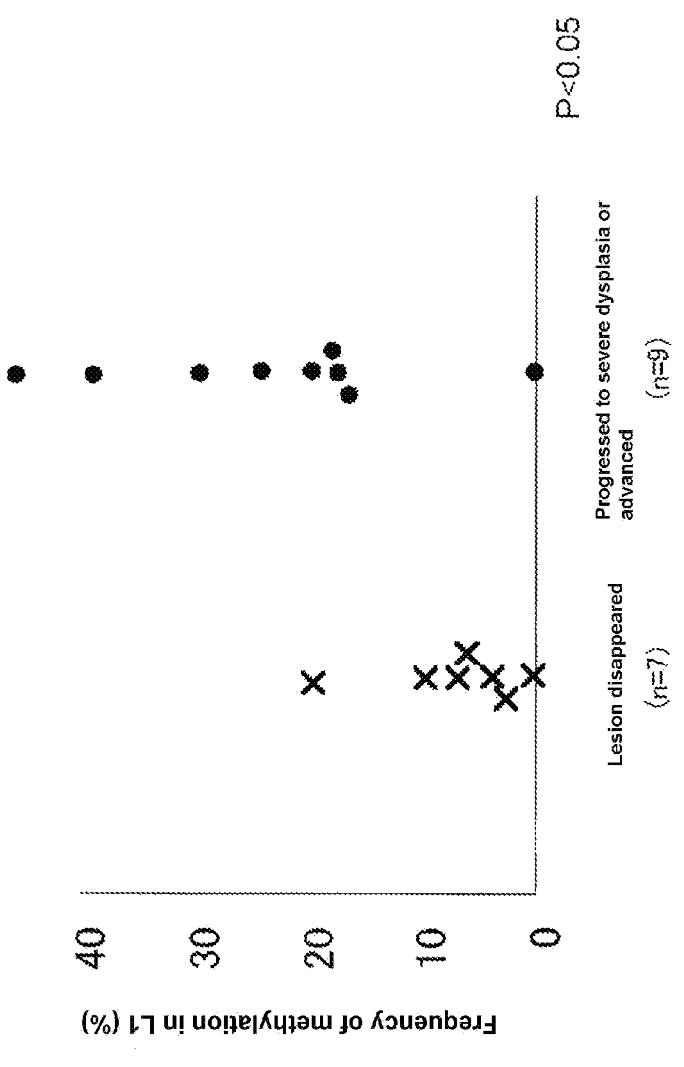
Frequency of methylation in L1 (%)
0
10
20
30
40
Lesion disappeared
(n=7)
Progressed to severe dysplasia or advanced
(n=9)
P<0.05

Figure 13

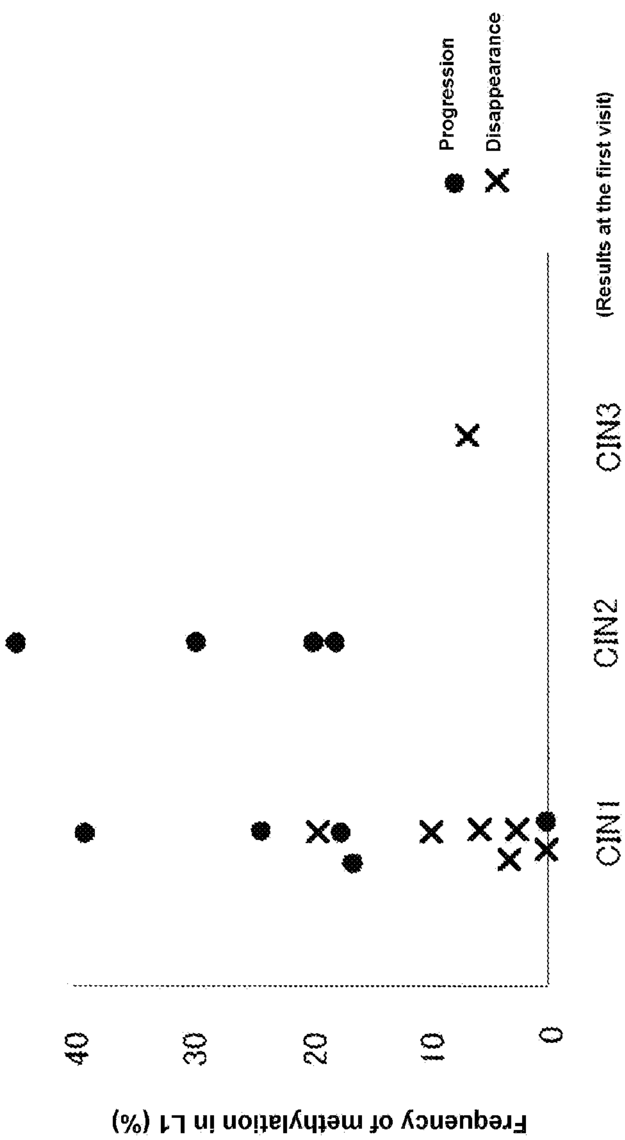
Frequency of methylation in L1 (%)
40
30
20
10
0
CIN1
CIN2
CIN3
(Results at the first visit)
Progression
Disappearance

**INTERNATIONAL SEARCH REPORT**

International application No.
PCT/JP2009/066963

A. CLASSIFICATION OF SUBJECT MATTER
C12Q1/68(2006.01)i, C12N15/09(2006.01)n

According to International Patent Classification (IPC) or to both national classification and IPC

B. FIELDS SEARCHED

Minimum documentation searched (classification system followed by classification symbols)
C12Q1/68, C12N15/09

Documentation searched other than minimum documentation to the extent that such documents are included in the fields searched

Electronic data base consulted during the international search (name of data base and, where practicable, search terms used)
JSTPlus/JMEDPlus/JST7580(JDreamII), PubMed

C. DOCUMENTS CONSIDERED TO BE RELEVANT

| Category* | Citation of document, with indication, where appropriate, of the relevant passages | Relevant to claim No. |
|---|---|---|
| X/Y | OKA N. ET AL, Clinical significance of HPV genome methylation in cervical cancer., Nihon Gan Gakkai Gakujutsu Sokai Kiji, 2008.09.30, Vol.67th, p.395(P-8009), entire text | 1-6,12,13/ 7-12,14-21 |
| Y | MUNOZ N. ET AL, Epidemiologic classification of human papillomavirus types associated with cervical cancer., N. Engl. J. Med., 2003, Vol.348, No.6, p.518-527, p.518, CONCLUTION | 7-12,14-21 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 October, 2009 (27.10.09) | 02 November, 2009 (02.11.09) |
| Name and mailing address of the ISA/ Japanese Patent Office | Authorized officer |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008071998 A
- WO 2006132022 A
- JP 2008273257 A

### Non-patent literature cited in the description


- **T. Turan et al.** Methylation of human papillomavirus-18 L1 gene: A biomarker of neoplastic progression?. *Virology,* 2006, vol. 349, 175-183
- **James G.HERMAN et al.** Methylation-specific PCR:A novel PCR assay for methylation status of CpG islands. *Proc. Natl. Acad. Sci. USA,* September 1996, vol. 93, 9821-9826